# EUROPEAN PATENT SPECIFICATION

(11) **EP 1 176 956 B1**
(45) Date of publication and mention of the grant of the patent: **09.01.2008**
(21) Application number: 00937527.0
(22) Date of filing: 05.05.2000
(51) Int. Cl.: A61K 31/215, A61K 31/335, A61K 31/38, A61K 31/4025, A61K 31/44, A61K 31/4427, A61K 31/445, A61K 31/4523, A61K 31/47, A61K 31/506, C07C 69/66, C07D 207/04, C07D 207/18, C07D 211/68, C07D 211/72, C07D 213/02, C07D 215/00

(54) **CARBOXYLIC ACID DERIVATIVES THAT INHIBIT THE BINDING OF INTEGRINS TO THEIR RECEPTORS**
CARBONSÄUREDERIVATE DIE DIE BINDUNG VON INTEGRINEN AN IHRE REZEPTOREN HEMMEN
DERIVES D'ACIDE CARBOXYLIQUE INHIBANT LA LIAISON ENTRE DES INTEGRINES ET LEURS RECEPTEURS

(30) Priority: 07.05.1999 US 132971 P
(43) Date of publication of application: 06.02.2002
(73) Proprietor: ENCYSIVE PHARMACEUTICALS, INC, Houston, TX 77081 (US)
(72) Inventor: BIEDIGER, Ronald, J., Houston, TX 77095 (US); CHEN, Qi, Pearland, TX 77584 (US); HOLLAND, George, W., North Caldwell, NJ 07006 (US); KASSIR, Jamal, M., Houston, TX 77054 (US); LI, Wen, Houston, TX 77057 (US); MARKET, Robert, V., Pearland, TX 77581 (US); SCOTT, Ian, L., Delanson, NY 12053 (US); WU, Chengde, Pearland, TX 77584 (US)
(74) Representative: Weber-Quitzau, Martin
(86) International application number: PCT/US2000/012303
(87) International publication number: WO 2000/067746

(56) References cited:
- EP-A- 0 508 798
- WO-A-00/17197
- WO-A-97/43650
- WO-A-98/11896
- WO-A-99/64397
- DE-A- 2 614 189
- US-A- 5 484 946
- US-A- 5 721 366
- US-A- 5 770 573
- PATENT ABSTRACTS OF JAPAN vol. 1996, no. 03, 29 March 1996 (1996-03-29) -& JP 07 304755 A (YAMANOUCHI PHARMACEUT CO LTD), 21 November 1995 (1995-11-21)
- DATABASE CROSSFIRE BEILSTEIN [Online] Beilstein Institut zur Förderung der Chemischen Wissenschaften, Frankfurt am Main, DE; Database accession no. 3412719 XP002196547 & BIRKOFER ET AL.: JUSTUS LIEBIGS ANN. CHEM., vol. 628, 1959, pages 162-170,
- DATABASE CROSSFIRE BEILSTEIN [Online] Beilstein Institut zur Förderung der Chemischen Wissenschaften, Frankfurt am Main, DE; Database accession no. 352769,338274 XP002196548 & RODIONOW ET AL.: J. OBSC. CHIM. , vol. 3, 1933, pages 628-632,
- DATABASE CROSSFIRE BEILSTEIN [Online] Beilstein Institut zur Förderung der Chemischen Wissenschaften, Frankfurt am Main, DE; Database accession no. 3460771 XP002196549 & JOHNSON ET AL.: J. AMER.CHEM. SOC., vol. 58, 1936, pages 299-301,
- DATABASE CROSSFIRE BEILSTEIN [Online] Beilstein Institut zur Förderung der Chemischen Wissenschaften, Frankfurt am Main, DE; Database accession no. 3384432 XP002196550 & MORSCH: MONATSH. CHEM., vol. 64, 1934, pages 333-338,
- DATABASE CROSSFIRE BEILSTEIN [Online] Beilstein Institut zur Förderung der Chemischen Wissenschaften, Frankfurt am Main, DE; Database accession no. 8351021 XP002196551 & SHANTARE ET AL.: CHEM. HETERCYCL. COMPD. (ENGL. TRANSL.), vol. 34, no. 3, 1998, pages 351-358,
- DATABASE CROSSFIRE BEILSTEIN [Online] Beilstein Institut zur Förderung der Chemischen Wissenschaften, Frankfurt am Main, DE; Database accession no. 5430991 XP002196552 & FLOUZAT ET AL.: TETRAHEDRON LETT., vol. 33, no. 32, 1992, pages 4571-4574,
- DATABASE CROSSFIRE BEILSTEIN [Online] Beilstein Institut zur Förderung der Chemischen Wissenschaften, Frankfurt am Main, DE; Database accession no. 6523266 XP002196553 & CRIDER ET AL.: J. PHARM. SCI., vol. 70, no. 2, 1981, pages 192-195,
- WALTERS ET AL.: 'Genetically evolved receptor models: A computational approach to construction of receptor models' J. MED. CHEM., vol. 37, 1994, pages 2527 - 2536, XP002931057

## Description

### Field of the Invention

This invention is directed generally to the inhibition of the binding of α₄β₁ integrin to its receptors, for example VCAM-1 (vascular cell adhesion molecule-1) and fibronectin. The invention also relates to compounds that inhibit this binding; to pharmaceutically active compositions comprising such compounds; and to such compounds either as above, or in formulations for use in the control or prevention of disease states in which α₄β₁ is involved.

### Background of the Invention

When a tissue has been invaded by a microorganism or has been damaged, white blood cells, also called leukocytes, play a major role in the inflammatory response. One of the most important aspects of the inflammatory response involves the cell adhesion event. Generally, white blood cells are found circulating through the bloodstream. However, when a tissue is infected or becomes damaged, the white blood cells recognize the invaded or damaged tissue, bind to the wall of the capillary and migrate through the capillary into the affected tissue. These events are mediated by a family of proteins called cell adhesion molecules.

There are three main types of white blood cells: granulocytes, monocytes and lymphocytes. The integrin α₄β₁ (also called VLA-4 for very late antigen-4) is a heterodimeric protein expressed on the surface of monocytes, lymphocytes and two subclasses of granulocytes: eosinophils and basophils. This protein plays a key role in cell adhesion through its ability to recognize and bind VCAM-1 and fibronectin, proteins associated with the endothelial cells that line the interior wall of capillaries.

Following infection or damage of tissue surrounding a capillary, endothelial cells express a series of adhesion molecules, including VCAM-1, that are critical for binding the white blood cells that are necessary for fighting infection. Prior to binding to VCAM-1 or fibronectin, the white blood cells initially bind to certain adhesion molecules to slow their flow and allow the cells to "roll" along the activated endothelium. Monocytes, lymphocytes, basophils and eosinophils are then able to firmly bind to VCAM-1 or fibronectin on the blood vessel wall *via* the α₄β₁ integrin. There is evidence that such interactions are also involved in transmigration of these white blood cells into the damaged tissue as well as the initial rolling event itself.

Although white blood cell migration to the site of injury helps fight infection and destroy foreign material, in many instances this migration can become uncontrolled, with white blood cells flooding to the scene, causing widespread tissue damage. Compounds capable of blocking this process, therefore, may be beneficial as therapeutic agents. Thus, it would be useful to develop inhibitors that would prevent the binding of white blood cells to VCAM-1 and fibronectin.

Some of the diseases that might be treated by the inhibition of α₄β₁ binding include, but are not limited to, atherosclerosis, rheumatoid arthritis, asthma, allergy, multiple sclerosis, lupus, inflammatory bowel disease, graft rejection, contact hypersensitivity, and type I diabetes. In addition to being found on some white blood cells, α₄β₁ is also found on various cancer cells, including leukemia, melanoma, lymphoma and sarcoma cells. It has been suggested that cell adhesion involving α₄β₁ may be involved in the metastasis of certain cancers. Inhibitors of α₄β₁ binding may, therefore, also be useful in the treatment of some forms of cancer.

The isolation and purification of a peptide which inhibits the binding of α₄β₁ to a protein is disclosed in U.S. Patent No. 5,510,332. Peptides which inhibit binding are disclosed in WO 95/15973, EP 0 341 915, EP 0 422 938 A1, U.S. Patent No. 5,192,746 and WO 96/06108. Novel compounds which are useful for inhibition and prevention of cell adhesion and cell adhesion-mediated pathologies are disclosed in WO 96/22966, WO 98/04247 and WO 98/04913. The intermediate document WO00/17197 discloses quinolizinones useful as integrin inhibitors.

It is therefore an object of the invention to provide novel compounds which are inhibitors of α₄β₁ binding, and pharmaceutical compositions including such novel compounds.

### Brief Summary of the Invention

The present invention is directed to compounds of Formula III 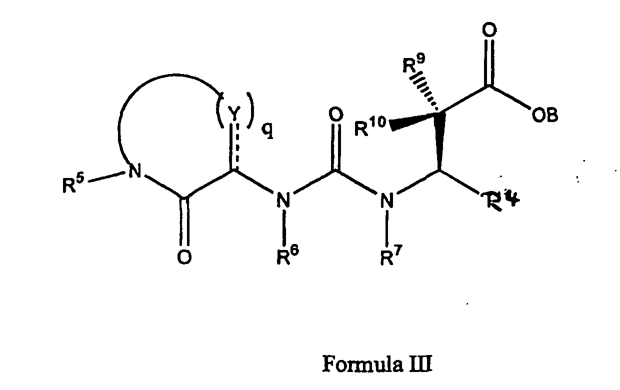 or a pharmaceutically acceptable salt thereof,
wherein Y at each occurrence, is independently selected from the group
consisting of C (0), N, CR¹, C (R²) (R³), and CH; and the ring containing Y may optionally be a bicyclic ring;
q is an integer of from 2 to 5;
B and R⁶ are independently selected from the group consisting of hydrogen and alkyl;
R¹ at each occurrence is independently selected from the group consisting of hydrogen, halogen, hydroxyl, alkyl, alkenyl, alkynyl, alkoxy, alkenoxy, alkynoxy, thioalkoxy, hydroxyalkyl, alkyl-C(O)-, alkenyl-C(O)-, alkynyl-C(O)-, -CF₃, nitro, amino, cyano, carboxy, -N(C₁-C₃ alkyl)-C(O)(C₁-C₃ alkyl), -NHC(O)N(C₁-C₃ alkyl)C(O)NH(C₁-C₃ alkyl), -NHC(O)NH(C₁-C₆ alkyl), alkylamino, alkenylamino, di(C₁-C₃)amino, -C(O)O-(C₁-C₃) alkyl, -C(O)NH-(C₁-C₃) alkyl, -C(O)N(C₁-C₃ alkyl)₂, -CH=NOH, -PO₃H₂, -OPO₃H₂, haloalkyl, alkoxyalkoxy, carboxaldehyde, carboxamide, cycloalkyl, cycloalkenyl, cycloalkylalkyl, aryl, aroyl, aryloxy, arylamino, biaryl, thioaryl, diarylamino, heterocyclyl, alkylaryl, aralkenyl, aralkyl, alkylheterocyclyl, heterocyclylalkyl, sulfonyl, -SO₂-(C₁-C₃ alkyl), -SO₃-(C₁-C₃alkyl), sulfonamido, carbamate, aryloxyalkyl, carboxyl and -C(O)NH(benzyl) groups; wherein each R¹ may be unsubstituted or substituted with one or more substituents selected from the group consisting of aryl, C₁-C₆ alkoxy, alkoxyalkoxy and alkyl;
and wherein when at least one Y is CR¹, R¹ and R⁶ taken
together may form a ring;
R² and R³ when present are hydrogen;
R⁴ is selected from the group consisting of alkyl and aryl;
R⁵ is selected from the group consisting of aralkyl, aryloxyalkyl and cycloalkylalkyl;
R⁷ is hydrogen;
R⁹ and R¹⁰ are selected from the group consisting of hydrogen and halogen; and wherein, unless otherwise indicated,
alkyl, alone or in combination, refers to C₁-C₁₂ straight or branched saturated chain radicals derived from saturated hydrocarbons by the removal of one hydrogen atom; alkenyl, alone or in combination, refers to a straight-chain or branched-chain alkenyl radical containing from 2 to 10 carbon atoms;
alkynyl, alone or in combination, refers to a straight branched chain alkynyl radical containing from 2 to 10 carbon atoms;
cycloalkyl refers to an aliphatic ring system having 3 to 10 carbon atoms and 1 to 3 rings which can be optionally substituted with one, two or three substituents independently selected from C₁-C₆ alkyl, haloalkyl, alkoxy, thioalkoxy, amino, alkylamino, dialkylamino, hydroxy, halo, mercapto, nitro, carboxaldehyde, carboxy, alkoxycarbonyl and carboxamide;
cycloalkenyl, alone or in combination, refers to a cyclic carbocycle containing from 4 to 8 carbon atoms and one or more double bonds;
cycloalkylalkyl refers to a cycloalkyl group appended to a C₁-C₆ alkyl radical; haloalkyl refers to a C₁-C₆ alkyl radical, to which is appended at least one halogen substituent;
alkoxy, alone or in combination, refers to an alkyl ether radical, wherein alkyl is as defined above;
alkenoxy refers to a radical of formula alkenyl-O, provided that the radical is not an enol ether, wherein alkenyl is as defined above;
alkynoxy refers to a radical of formula alkynyl-O, provided that the radical is not an -ynol ether,
thioalkoxy refers to a thioether radical of formula alkyl-S-, wherein alkyl is as defined above;
alkoxyalkoxy refers to R_{g}O-RₕO-wherein R_{g} is a C₁-C₆ alkyl and Rₕ is -(CH₂)_{n'}- wherein n' is an integer from 1 to 6;
alkylamino refers to RᵢNH- wherein Rᵢ is a C₁-C₆ alkyl group, provided that the radical is not an enamine;
alkenylamino refers to a radical of formula alkenyl-NH- or (alkenyl)₂N-, wherein alkenyl is as defined above, provided that the radical is not an enamine;
dialkylamino refers to RⱼRₖN- wherein Rⱼ and Rₖ are independently selected from C₁-C₆ alkyl;
aryl, alone or in combination, refers to a carbocyclic aromatic group having 6 to 12 carbon atoms or a heterocyclic aromatic group containing at least one endocyclic N, 0 or S atom, said aryl being optionally substituted with at least one group selected from the group consisting of alcohols, ethers, esters, amides, sulfones, sulfides, hydroxyl, nitro, cyano, carboxy, amines, heteroatoms, C₁-C₆ alkyl, C₁-C₆ alkoxy, C₁-C₆ alkoxy-C(O)-, alkoxyalkoxy, acyloxy, halogens, trifluoromethoxy, trifluoromethyl, alkyl, aralkyl, alkenyl, alkynyl, aryl, cyano, carboxy, carboalkoxy, carboxyalkyl, cycloalkyl, cycloalkylalkyl, heterocyclyl, alkylheterocyclyl, heterocyclylalkyl, oxo, arylsulfonyl and aralkylaminocarbonyl wherein said substituents are optionally attached by means of a linker selected from a chain of 1-3 atoms containing any combination of -C-, -C(O)-, -NH-, -S-, -S(O)-, -O-, -C(O)O- or -S(O)O-;
thioaryl refers to a radical of formula aryl-S-, wherein aryl is as defined above;
heterocyclyl, alone or in combination, refers to a non-aromatic 3 to 10-membered ring containing at least one endocyclic N, O, or S atom, said heterocyclyl being optionally aryl-fused and optionally substituted with at least one substituent independently selected from the group consisting of hydrogen, halogen, hydroxyl, amino, nitro, trifluoromethyl, trifluoromethoxy, alkyl, aralkyl, alkenyl, alkynyl, aryl, cyano, carboxy, carboalkoxy, carboxyalkyl, oxo, arylsulfonyl and aralkylaminocarbonyl.

In Formula III, the portion of the molecule 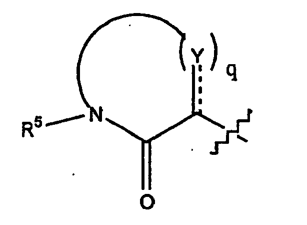 can be 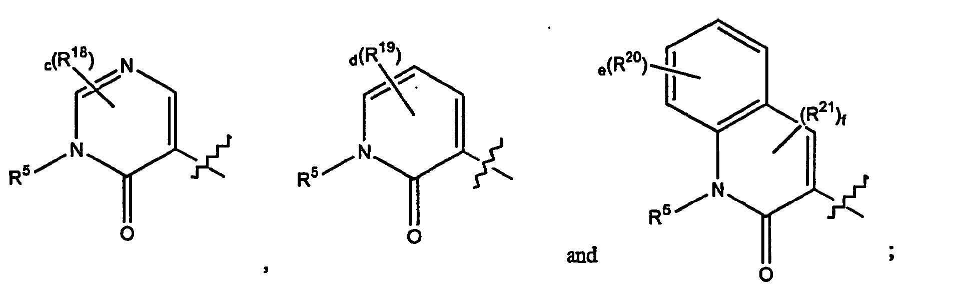 wherein R¹⁸, R¹⁹, R²⁰ and R²¹ at each occurrence are independently selected from the group consisting of hydrogen, halogen, hydroxyl, alkyl, alkenyl, alkynyl, alkoxy, alkenoxy, alkynoxy, thioalkoxy, hydroxyalkyl, aliphatic acyl, -CF₃, nitro, amino, cyano, carboxy, -N(C₁-C₃ alkyl)-C(O)(C₁-C₃ alkyl), -NHC(O)N(C₁-C₃ alkyl)C(O)NH(C₁-C₃alkyl), -NHC(O)NH(C₁-C₆ alkyl), alkylamino, alkenylamino, di(C₁-C₃)amino, -C(O)O-(C₁-C₃)alkyl, -C(O)NH-(C₁-C₃)alkyl, -C(O)N(C₁-C₃ alkyl)₂, -CH=NOH, -PO₃H₂, -OPO₃H₂, haloalkyl, alkoxyalkoxy, carboxaldehyde, carboxamide, cycloalkyl, cycloalkenyl, cycloalkynyl, cycloalkylalkyl, aryl, aroyl, aryloxy, arylamino, biaryl, thioaryl, diarylamino, heterocyclyl, alkylaryl, aralkenyl, aralkyl, alkylheterocyclyl, heterocyclylalkyl, sulfonyl, -SO₂-(C₁-C₃ alkyl), -SO₃-(C₁-C₃ alkyl), sulfonamido, carbamate, aryloxyalkyl, carboxyl and -C(O)NH(benzyl) groups;
c is an integer of zero to two;
d is an integer of zero to three;
e is an integer of zero to four; and
f is an integer of zero or one.

In one embodiment, R⁵ is alkylaryl; R⁴ is aryl; and, B and R⁶, are each independently hydrogen. In another embodiment, the compound of Formula III or pharmaceutically acceptable salt thereof is a compound wherein two independent R¹, R², R³ or R⁵ groups taken together may be linked to form a ring.
Presently preferred compounds include:
(3S)-3-[({[2-methyl-4-(2-methylpropyl)-6-oxo-1-(phenylmethyl)-1,6-dihydro-5-pyrimidinyl]amino}carbonyl)amino]-3-(4-methylphenyl)propanoic acid,
(3S)-3-(1,3-benzodioxol-5-yl)-3-[({[2-oxo-1-(phenylmethyl)-4-propyl-1,2-dihydro-3-pyridinyl]amino}carbonyl)amino]propanoic acid,
(3S)-3-{[({1-[(2-chlorophenylmethyl]-4-ethyl-2-oxo-1,2-dihydro-3-pyridinyl}amino)carbonyl]amino}-3-(4-methylphenyl)propanoic acid,
(3S)-3-{[({1-[(2-chlorophenyl)methyl]-2-oxo-4-propyl-1,2-dihydro-3-pyridinyl}amino)carbonyl]amino}-3-(4-methylphenyl)propanoic acid,
(3S)-3-{[({1-[(2-chlorophenyl)methyl]-4-methyl-2-oxo-1,2-dihydro-3-pyridinyl}amino)carbonyl]amino}-3-(4-methylphenyl)propanoic acid,
(3S)-3-{[({6-methyl-2-oxo-1-(phenylmethyl)-4-[(phenylmethyl)oxy]-1,2-dihydro-3-pyridinyl} amino)carbonyl]amino}-3-(4-methylphenyl)propanoic acid,
(3S)-3-{[({1-[(2-chlorophenyl)methyl]-2,4-dimethyl-6-oxo-1,6-dihydro-5-primidinyl}amino)carbonyl]amino}-3-(4-methylphenyl)propanoic acid,
(3S)-3-{[({4-amino-1-[(2-chlorophenyl)methyl]-6-methyl-2-oxo-1,2-dihydro-3-pyridinyl}amino)carbonyl]amino}-3-(4-methylphenyl)propanoic acid,
(3S)-3-{[({1-[(2-chlorophenyl)methyl]-4-methyl-2-oxo-1,2-dihydro-3-pyridinyl}amino)carbonyl]amino}-3-[4-(methyloxy)phenyl]propanoic acid,
(3S)-3-{[({1-[(2-chlorophenyl)methyl]-4-methyl-2-oxo-1,2-dihydro-3-pyridinyl}amino)carbonyl]amino}-3-(3,4-dimethylphenyl)propanoic acid,
(3S)-3- {[({4-amino-1-[(2-chlorophenyl)methyl]-2-oxo-1,2-dihydro-3-pyridinyl}amino)carbonyl]amino}-3-(4-methylphenyl)propanoic acid,
(3S)-3- {[({1-[(2-chlorophenyl)methyl]-4-hydroxy-2-oxo-1,2-dihydro-3-pyridinyl}amino)carbonyl]amino}-3-(4-methylphenyl)propanoic acid,
(3S)-3-[({[1-[(2-chlorophenyl)methyl]-4-(1,4-oxazinan-4-yl)-2-oxo-1,2-dihydro-3-pyridinyl]amino}carbonyl)amino]-3-(4-methylphenyl)propanoic acid,
(3S)-3-[({[1-[(2-chlorophenyl)methyl]-2-oxo-4-(propylamino)-1 ,2-dihydro-3-pyridinyl]amino}carbonyl)amino]-3-(4-methylphenyl)propanoic acid,
(3S)-3- {[({1-[(2-bromophenyl)methyl]-4-methyl-2-oxo-1,2-dihydro-3-pyridinyl}amino)carbonyl]amino}-3-(4-methylphenyl)propanoic acid,
(3S)-3-{[({1-[(2-chlorophenyl)methyl]-4-hydroxy-2-oxo-1,2-dihydro-3-pyridinyl}amino)carbonyl]amino}-3-[3-methyl-4-(methyloxy)phenyl]propanoic acid,
(3S)-3-{[({1-[(2-chlorophenyl)methyl]-2-oxo-4-phenyl-1,2-dihydro-3-pyridinyl} amino)carbonyl]amino}-3-(4-methylphenyl)propanoic acid,
(3S)-3-{[({1-[(2-chlorophenyl)methyl]-4-[(2-{[2-(methyloxy)ethyl]oxy}ethyl)oxy]-2-oxo-1,2-dihydro-3-pyridinyl}amino)carbonyl]amino}-3-(4-methylphenyl)propanoic acid,
(3S)-3- {[({1-[(2-chlorophenyl)methyl]-4-hydroxy-6-methyl-2-oxo-1, 2-dihydro-3-pyridinyl} amino)carbonyl]amino}-3-(4-methylphenyl)propanoic acid,
(3S)-3- {[({1-[(2-chlorophenyl)methyl]-4-[(1,1-dimethylethyl)amino]-2-oxo-1,2-dihydro-3-pyridinyl} amino)carbonyl]amino} -3-(4-methylphenyl)propanoic acid,
(3S)-3-{[({1-[(2-chlorophenyl)methyl]-4-hydroxy-2-oxo-1,2-dihydro-3-pyridinyl}amino)carbonyl]amino}-3-phenylpropanoic acid,
(3S)-3- {[({1-[(2-chlorophenyl)methyl]-4-[4-methyltetrahydro-1(2H)-pyrazinyl]-2-oxo-1,2-dihydro-3-pyridinyl}amino)carbonyl]amino}-3-(4-methylphenyl)propanoic acid,
(3S)-3-{[({1-[(2-chlorophenyl)methyl]-4-hydroxy-2-oxo-1,2-dihydro-3-pyridinyl}amino)carbonyl]amino}-3-[4-(methyloxy)phenyl]propanoicacid,
(3S)-3-{[({1-[(2-chlorophenyl)methyl]-4-hydroxy-2-oxo-1,2-dihydro-3-pyridinyl}amino)carbonyl]amino}-3-(3,5-dimethylphenyl)propanoicacid,
(3S)-3-{[({1-[(2-chlorophenyl)methyl]-4-hydroxy-2-oxo-1,2-dihydro-3-pyridinyl}amino)carbonyl]amino}-3-(3-methylphenyl)propanoic acid,
(3S)-3- {[({1-[(2-chlorophenyl)methyl]-4-hydroxy-2-oxo-1,2-dihydro-3-pyridinyl}amino)carbonyl]amino}-3-[3-(methyloxy)phenyl]propanoic acid,
(3S)-3-[3,5-bis(methyloxy)phenyl]-3- {[({1-[(2-chlorophenyl)methyl]-4-hydroxy-2-oxo-1,2-dihydro-3-pyridinyl} amino)carbonyl]amino}propanoic acid,
(3S)-3- {[({1-[(2-chlorophenyl)methyl]-4-hydroxy-2-oxo- 1,2-dihydro-3-quinolinyl}amino)carbonyl]amino}-3-(4-methylphenyl)propanoic acid,
(3S)-3-{[({1-[(2-chlorophenyl)methyl]-4-hydroxy-2-oxo-1,2-dihydro-3-pyridinyl}amino)carbonyl]amino}-3-[3-(trifluoromethyl)phenyl]propanoic acid,
(3S)-3- {[({1-[(2-chlorophenyl)methyl]-4-[({ethyl[(ethylamino)carbonyl]amino}carbonyl) amino]-2-oxo-1,2-dihydro-3-pyridinyl}amino)carbonyl]amino}-3-(4-methylphenyl)propanoic acid,
(3S)-3-{[({4-(1-azetanyl)-1-[(2-chlorophenyl)methyl]-2-oxo-1,2-dihydro-3-pyridinyl}amino)carbonyl]amino}-3-(4-methylphenyl)propanoic acid,
(3S)-3-[({[1-[(2-chlorophenyl)methyl]-4-({2-[(2-{(2-(methyloxy)ethyl]oxy}ethyl)oxy]ethyl} oxy)2-oxo-1,2-dihydro-3-pyridinyl]amino}carbonyl)amino]-3-(4-methylphenyl)propanoic acid,
(3S)-3-{[({1-[(2-fluorophenyl)methyl]-4-hydroxy-2-oxo-1,2-dihydro-3-pyridinyl}amino)carbonyl]amino}-3-(4-methylphenyl)propanoic acid,
(3S)-3-{[({1-[(2-chloro-6-fluorophenyl)methyl]-4-hydroxy-2-oxo-1,2-dihydro-3-pyridinyl}amino)carbonyl]amino}-3-(4-methylphenyl)propanoic acid,
(3S)-3- {[({1-[(2-chlorophenyl)methyl]-5-methyl-2-oxo-1,2-dihydro-3-pyridinyl}amino)carbonyl]amino}-3-(4-methylphenyl)propanoic acid,
(3S)-3-(1,3-benzodioxol-5-yl)-3-((((2-oxo-l-((4-(trifluoromethyl)phenyl)methyl)-1,2 dihydro-3-pyridinyl)amino)carbonyl)amino)propanoic acid, (3S)-3-((((1-((2-chlorophenyl)methyl)-2-oxo-1,2-dihydro-3-pyridinyl)amino)carbonyl)amino)-3-(4-methylphenyl)propanoic acid,
(3S)-3-((((1-((2-fluorophenyl)methyl)-2-oxo-1,2-dihydro-3-pyridinyl)amino)carbonyl)amino)-3-(4-methylphenyl)propanoic acid, (3S)-3-((((1-((2-bromophenyl)methyl)-2-oxo-1,2-dihydro-3-pyridinyl)amino)carbonyl)amino)-3-(4-methylphenyl)propanoic acid,
(3S)3-((((1-((2,4-dichlorophenyl)methyl)-2-oxo-1,2-dihydro-3-pyridinyl)amino)carbonyl) amino)-3-(4-methylphenyl)propanoic acid, (3S)-3-((((1-((2-chloro-6-fluorophenyl)methyl)-2-oxo-1,2-dihydro-3-pyridinyl)amino)carbonyl)amino)-3-(4-methylphenyl)propanoic acid,
(3S)-3-((((1-((2-chlorophenyl)methyl)-4-hydroxy-2-oxo-1,2-dihydro-3-pyridinyl)amino)carbonyl)amino)-3-(4-trifluorormethyl)oxy)phenyl)propanoic acid and pharmaceutically acceptable salts thereof.

Derivatives such as esters, carbamates, aminals, amides, optical isomers and pro-drugs are also contemplated.

The present invention also relates to pharmaceutical compositions comprising a physiologically acceptable diluent and at least one compound of the present invention.

Specifically the invention relates to a pharmaceutical composition comprising a compound of Formula II or a pharmaceutically acceptable salt thereof in a pharmaceutically acceptable carrier.

The present invention further relates to the use of a compound of Formula III or a pharmaceutically acceptable salt thereof for the manufacture of a medicament for selectively inhibiting the binding of α₄β₁ integrin in a mammal by administering to said mammal a therapeutic amount thereof. The α₄β₁ may be on a white blood cell such as a monocyte, lymphocyte, granulocyte; a stem cell; or any other cell that naturally expresses α₄β₁.

The invention further relates to a compound of Formula III or a pharmaceutically acceptable salt thereof for use as a medicament.

### Detailed Description of the Invention

### Definitions of Terms

The term "alkyl" as used herein, alone or in combination, refers to C₁-C₁₂ straight or branched, substituted or unsubstituted saturated chain radicals derived from saturated hydrocarbons by the removal of one hydrogen atom, unless the term alkyl is preceded by a Cₓ-.

C_{y} designation. Representative examples of alkyl groups include methyl, ethyl, n-propyl, iso-propyl, n-butyl, sec-butyl, iso-butyl, and tert-butyl among others.

The term "alkenyl" as used herein, alone or in combination, refers to a substituted or unsubstituted straight-chain or substituted or unsubstituted branched-chain alkenyl radical containing from 2 to 10 carbon atoms. Examples of such radicals include, but are not limited to, ethenyl, E- and Z-pentenyl, decenyl and the like.

The term "alkynyl" as used herein, alone or in combination, refers to a substituted or unsubstituted straight or substituted or unsubstituted branched chain alkynyl radical containing from 2 to 10 carbon atoms. Examples of such radicals include, but are not limited to ethynyl, propynyl, propargyl, butynyl, hexynyl, decynyl and the like.

The term "lower" modifying "alkyl", "alkenyl", "alkynyl" or "alkoxy" refers to a C₁-C₆ unit for a particular functionality. For example lower alkyl means C₁-C₆ alkyl.

The term "aliphatic acyl" as used herein, alone or in combination, refers to radicals of formula alkyl-C(O)-, alkenyl-C(O)- and alkynyl-C(O)- derived from an alkane-, alkene- or alkyncarboxylic acid, wherein the terms "alkyl", "alkenyl" and "alkynyl" are as defined above. Examples of such aliphatic acyl radicals include, but are not limited to, acetyl, propionyl, butyryl, valeryl, 4-methylvaleryl, acryloyl, crotyl, propiolyl and methylpropiolyl, among others.

The term "cycloalkyl" as used herein refers to an aliphatic ring system having 3 to 10 carbon atoms and 1 to 3 rings, including, but not limited to cyclopropyl, cyclopentyl, cyclohexyl, norbornyl, and adamantyl among others. Cycloalkyl groups can be unsubstituted or substituted with one, two or three substituents independently selected from lower alkyl, haloalkyl, alkoxy, thioalkoxy, amino, alkylamino, dialkylamino, hydroxy, halo, mercapto, nitro, carboxaldehyde, carboxy, alkoxycarbonyl and carboxamide.

"Cycloalkyl" includes cis or trans forms. Furthermore, the substituents may either be in endo or exo positions in the bridged bicyclic systems.

The term "cycloalkenyl" as used herein alone or in combination refers to a cyclic carbocycle containing from 4 to 8 carbon atoms and one or more double bonds. Examples of such cycloalkenyl radicals include, but are not limited to, cyclopentenyl, cyclohexenyl, cyclopentadienyl and the like.

The term "cycloalkylalkyl" as used herein refers to a cycloalkyl group appended to a lower alkyl radical, including, but not limited to cyclohexylmethyl.

The term "halo" or "halogen" as used herein refers to I, Br, Cl or F.

The term "haloalkyl" as used herein refers to a lower alkyl radical, to which is appended at least one halogen substituent, for example chloromethyl, fluoroethyl, trifluoromethyl and pentafluoroethyl among others.

The term "alkoxy" as used herein, alone or in combination, refers to an alkyl ether radical, wherein the term "alkyl" is as defined above. Examples of suitable alkyl ether radicals include, but are not limited to, methoxy, ethoxy, n-propoxy, iso-propoxy, n-butoxy, iso-butoxy, sec-butoxy, tert-butoxy and the like.

The term "alkenoxy" as used herein, alone or in combination, refers to a radical of formula alkenyl-O, provided that the radical is not an enol ether, wherein the term "alkenyl" is as defined above. Examples of suitable alkenoxy radicals include, but are not limited to, allyloxy, E- and Z- 3-methyl-2-propenoxy and the like.

The term "alkynoxy" as used herein, alone or in combination, refers to a radical of formula alkynyl-O, provided that the radical is not an -ynol ether. Examples of suitable alkynoxy radicals include, but are not limited to, propargyloxy, 2-butynyloxy and the like.

The term "carboxyl" as used herein refers to a carboxylic acid radical, -C(O)OH.

The term "carboxy" as used herein refers to -C(O)O-.

The term "thioalkoxy" refers to a thioether radical of formula alkyl-S-, wherein "alkyl" is as defined above.

The term "carboxaldehyde" as used herein refers to -C(O)R wherein R is hydrogen.

The terms "carboxamide" or "amide" as used herein refer to -C(O)NRₐR_{b}, wherein Rₐ and R_{b} are each independently hydrogen, alkyl or any other suitable substituent.

The term "alkoxyalkoxy" as used herein refers to R_{c}O-R_{d}O- wherein R_{c} is lower alkyl as defined above and R_{d} is alkylene wherein alkylene is -(CH₂)_{n'}- wherein n' is an integer from 1 to 6. Representative examples of alkoxyalkoxy groups include methoxymethoxy, ethoxymethoxy, t-butoxymethoxy among others.

The term "alkylamino" as used herein refers to R_{c}NH- wherein Rₑ is a lower alkyl group, for example, ethylamino, butylamino, among others.

The term "alkenylamino" as used herein, alone or in combination, refers to a radical of formula alkenyl-NH-or (alkenyl)₂N-, wherein the term "alkenyl" is as defined above, provided that the radical is not an enamine. An example of such alkenylamino radical is the allylamino radical.

The term "alkynylamino" as used herein, alone or in combination, refers to a radical of formula alkynyl-NH- or (alkynyl)₂N- wherein the term "alkynyl" is as defined above, provided that the radical is not an amine. An example of such alkynylamino radicals is the propargyl amino radical.

The term "dialkylamino" as used herein refers to R_{f}R_{g}N- wherein R_{f} and R_{g} are independently selected from lower alkyl, for example diethylamino, and methyl propylamino, among others.

The term "amino" as used herein refers to H₂N-.

The term "alkoxycarbonyl" as used herein refers to an alkoxyl group as previously defined appended to the parent molecular moiety through a carbonyl group. Examples of alkoxycarbonyl include methoxycarbonyl, ethoxycarbonyl, and isopropoxycarbonyl among others.

The term "aryl" or "aromatic" as used herein alone or in combination refers to a substituted or unsubstituted carbocyclic aromatic group having about 6 to 12 carbon atoms such as phenyl, naphthyl, indenyl, indanyl, azulenyl, fluorenyl and anthracenyl; or a heterocyclic aromatic group containing at least one endocyclic N, O or S atom such as furyl, thienyl, pyridyl, pyrrolyl, oxazolyl, thiazolyl, imidazolyl, pyrazolyl, 2-pyrazolinyl, pyrazolidinyl, isoxazolyl, isothiazolyl, 1,2,3-oxadiazolyl, 1,2,3-triazolyl, 1,3,4-thiadiazolyl, pyridazinyl, pyrimidinyl, pyrazinyl, 1,3,5-triazinyl, 1,3,5-trithianyl, indolizinyl, indolyl, isoindolyl, 3H-indolyl, indolinyl, benzo[b]furanyl, 2,3-dihydrobenzofuranyl, benzo[b]thiophenyl, 1H-indazolyl, benzimidazolyl, benzthiazolyl, purinyl, 4H-quinolizinyl, isoquinolinyl, cinnolinyl, phthalazinyl, quinazolinyl, quinoxalinyl, 1,8-naphthridinyl, pteridinyl, carbazolyl, acridinyl, phenazinyl, phenothiazinyl, phenoxyazinyl, pyrazolo[1,5-c]triazinyl and the like. "Aralkyl" and "alkylaryl" employ the term "alkyl" as defined above. Rings may be multiply substituted.

The term "aralkyl" as used herein, alone or in combination, refers to an aryl substituted alkyl radical, wherein the terms "alkyl" and "aryl" are as defined above. Examples of suitable aralkyl radicals include, but are not limited to, phenylmethyl, phenethyl, phenylhexyl, diphenylmethyl, pyridylmethyl, tetrazolyl methyl, furylmethyl, imidazolyl methyl, indolylmethyl, thienylpropyl and the like.

The term "aralkenyl" as used herein, alone or in combination, refers to an aryl substituted alkenyl radical, wherein the terms "aryl" and "alkenyl" are as defined above.

The term "arylamino" as used herein, alone or in combination, refers to a radical of formula aryl-NH-, wherein "aryl" is as defined above. Examples of arylamino radicals include, but are not limited to, phenylamino(anilido), naphthlamino, 2-, 3-, and 4- pyridylamino and the like.

The term "biaryl" as used herein, alone or in combination, refers to a radical of formula aryl-aryl, wherein the term "aryl" is as defined above.

The term "thioaryl" as used herein, alone or in combination, refers to a radical of formula aryl-S-, wherein the term "aryl" is as defined above. An example of a thioaryl radical is the thiophenyl radical.

The term "aroyl" as used herein, alone or in combination, refers to a radical of formula aryl-CO-, wherein the term "aryl" is as defined above. Examples of suitable aromatic acyl radicals include, but are not limited to, benzoyl, 4-halobenzoyl, 4-carboxybenzoyl, naphthoyl, pyridylcarbonyl and the like.

The term "heterocyclyl" as used herein, alone or in combination, refers to a non-aromatic 3- to 10- membered ring containing at least one endocyclic N, O, or S atom. The heterocycle may be optionally aryl-fused. The heterocycle may also optionally be substituted with at least one substituent which is independently selected from the group consisting of hydrogen, halogen, hydroxyl, amino, nitro, trifluoromethyl, trifluoromethoxy, alkyl, aralkyl, alkenyl, alkynyl, aryl, cyano, carboxy, carboalkoxy, carboxyalkyl, oxo, arylsulfonyl and aralkylaminocarbonyl among others.

The term "alkylheterocyclyl" as used herein refers to an alkyl group as previously defined appended to the parent molecular moiety through a heterocyclyl group, including but not limited to 2-methyl-5-thiazolyl, 2-methyl-1-pyrrolyl and 5-ethyl-2-thienyl.

The term "heterocyclylalkyl" as used herein refers to a heterocyclyl group as previously defined appended to the parent molecular moiety through an alkyl group, including but not limited to 2-thienylmethyl, 2-pyridinylmethyl and 2-(1-piperidinyl) ethyl.

The term "aminal" as used herein refers to a hemi-acetal of the structure RₕC(NRᵢRⱼ)(NRₖRₗ)- wherein Rₕ, Rᵢ, Rⱼ, Fₖ and Rₗ are each independently hydrogen, alkyl or any other suitable substituent.

The term "ester" as used herein refers to -C(O)Rₘ, wherein Rₘ is hydrogen, alkyl or any other suitable substituent.

The term "carbamate" as used herein refers to compounds based on carbamic acid NH₂C(O)OH.

Use of the above terms is meant to encompass substituted and unsubstituted moieties. Substitution may be by one or more groups such as alcohols, ethers, esters, amides, sulfones, sulfides, hydroxyl, nitro, cyano, carboxy, amines, heteroatoms, lower alkyl, lower alkoxy, lower alkoxycarbonyl, alkoxyalkoxy, acyloxy, halogens, trifluoromethoxy, trifluoromethyl, alkyl, aralkyl, alkenyl, alkynyl, aryl, cyano, carboxy, carboalkoxy, carboxyalkyl, cycloalkyl, cycloalkylalkyl, heterocyclyl, alkylheterocyclyl, heterocyclylalkyl, oxo, arylsulfonyl and aralkylaminocarbonyl or any of the substituents of the preceding paragraphs or any of those substituents either attached directly or by suitable linkers. The linkers are typically short chains of 1-3 atoms containing any combination of-C-, -C(O)-, -NH-, -S-, -S(O)-, -O-, -C(O)O- or -S(O)O-. Rings may be substituted multiple times.

The terms "electron-withdrawing" or "electron-donating" refer to the ability of a substituent to withdraw or donate electrons relative to that of hydrogen if hydrogen occupied the same position in the molecule. These terms are well-understood by one skilled in the art and are discussed in Advanced Organic Chemistry by J. March, 1985, pp. 16-18, incorporated herein by reference. Electron withdrawing groups include halo, nitro, carboxyl, lower alkenyl, lower alkynyl, carboxaldehyde, carboxyamido, aryl, quaternary ammonium, trifluoromethyl, and aryl lower alkanoyl among others. Electron donating groups include such groups as hydroxy, lower alkyl, amino, lower alkylamino, di(lower alkyl)amino, aryloxy, mercapto, lower alkylthio, lower alkylmercapto, and disulfide among others. One skilled in the art will appreciate that the aforesaid substituents may have electron donating or electron withdrawing properties under different chemical conditions. Moreover, the present invention contemplates any combination of substituents selected from the above-identified groups.

The most preferred electron donating or electron withdrawing substituents are halo, nitro, alkanoyl, carboxaldehyde, arylalkanoyl, aryloxy, carboxyl, carboxamide, cyano, sulfonyl, sulfoxide, heterocyclyl, guanidine, quaternary ammonium, lower alkenyl, lower alkynyl, sulfonium salts, hydroxy, lower alkoxy, lower alkyl, amino, lower alkylamino, di(lower alkyl)amino, amine lower alkyl mercapto, mercaptoalkyl, alkylthio and alkyldithio.

As used herein, the term "composition" is intended to encompass a product comprising the specified ingredients in the specified amounts, as well as any product which results, directly or indirectly, from a combination of the specified ingredients in the specified amounts.

The ring defined by Y in Formula III can be a mono-cyclic heterocycle or aromatic ring, or can be a bicyclic ring.

The dotted lines used in Formula III indicate that the bond between the atoms Yand W for example can be a single or double bond if Y is a substitutent such as N, C or CH. Therefore, the ring defined by Y in the Formulae can be either saturated or unsaturated, depending upon which Y is selected.

Two independent R¹, R², R³ or R⁵ groups taken together may be linked to form a ring.

### Abbreviations

Abbreviations which have been used in the schemes and the examples which follow are: BOC for t-butyloxycarbonyl; DMF for dimethylformamide; THF for tetrahydrofuran; DME for dimethoxyethane; DMSO for dimethylsulfoxide; NMM for N-methyl morpholine; DIPEA for diisopropylethylamine; CDI for 1,1'-carbonyldiimidazole; TBS for TRIS-buffered saline; Ms for methanesulfonyl, TMEDA for N,N,N',N'-tetramethylethylenediamine, DCE for 1,2-dichloroethane, NCS for N-chlorosuccinimide, NBS for N-bromosuccinimide, DPPA for diphenylphosphorylazide, DEAD for diethyl azodicarboxylate, TFAA for trifluoroacetic anhydride, DCM for dichloromethane, LHMDS for lithium bis(trimethylsilyl)amide and Cbz for benzyloxycarbonyl. Amino acids are abbreviated as follows: C for L-cysteine; D for L-aspartic acid; E for L-glutamic acid; G for glycine; H for L-histidine; I for L-isoleucine; L for L-leucine; N for L-asparagine; P for L-proline; Q for L-glutamine; S for L-serine; T for L-threonine; V for L-valine and W for L-tryptophan.

Examples of the procedures that may be used to synthesize compounds of the Formulae described above are shown in the Schemes which follow. A detailed description of the representative compounds of the present invention is set forth in the Examples below.

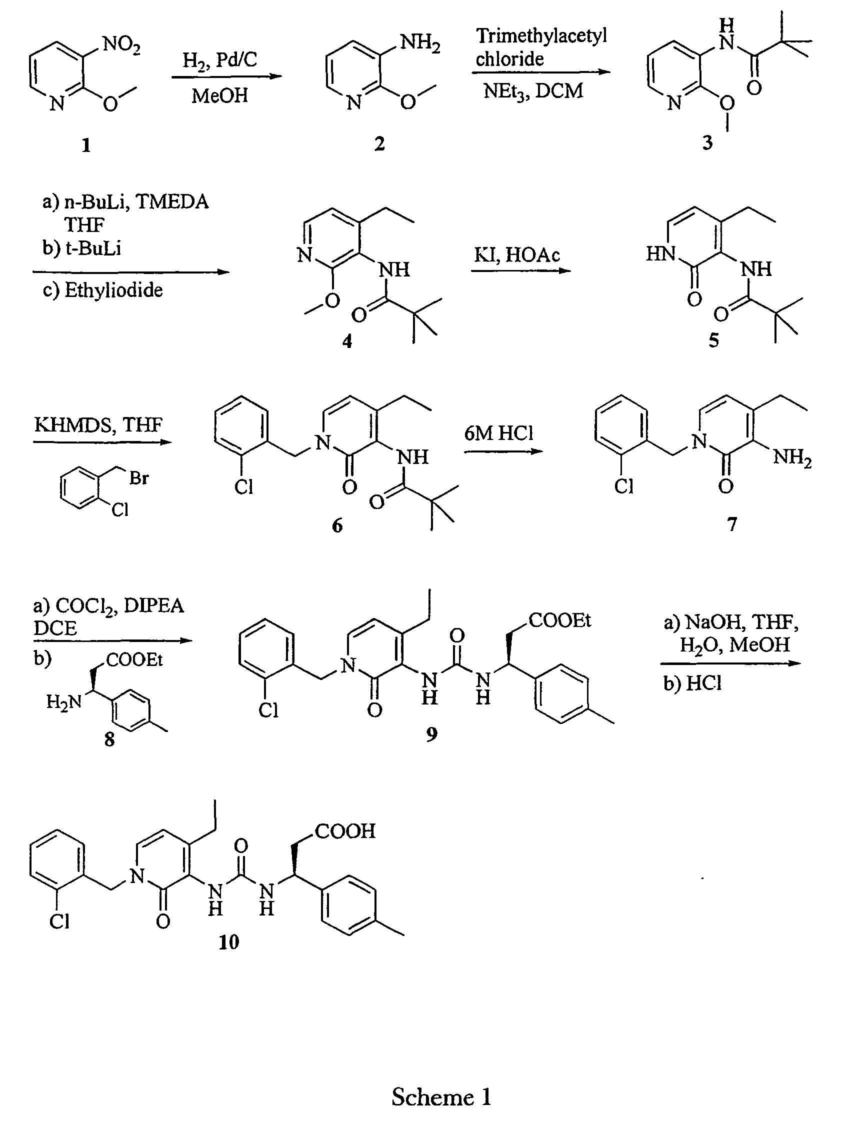

Scheme 1 above illustrates the procedure described in Example 1.

Scheme 2, illustrating the procedure of Example 2, is shown below.

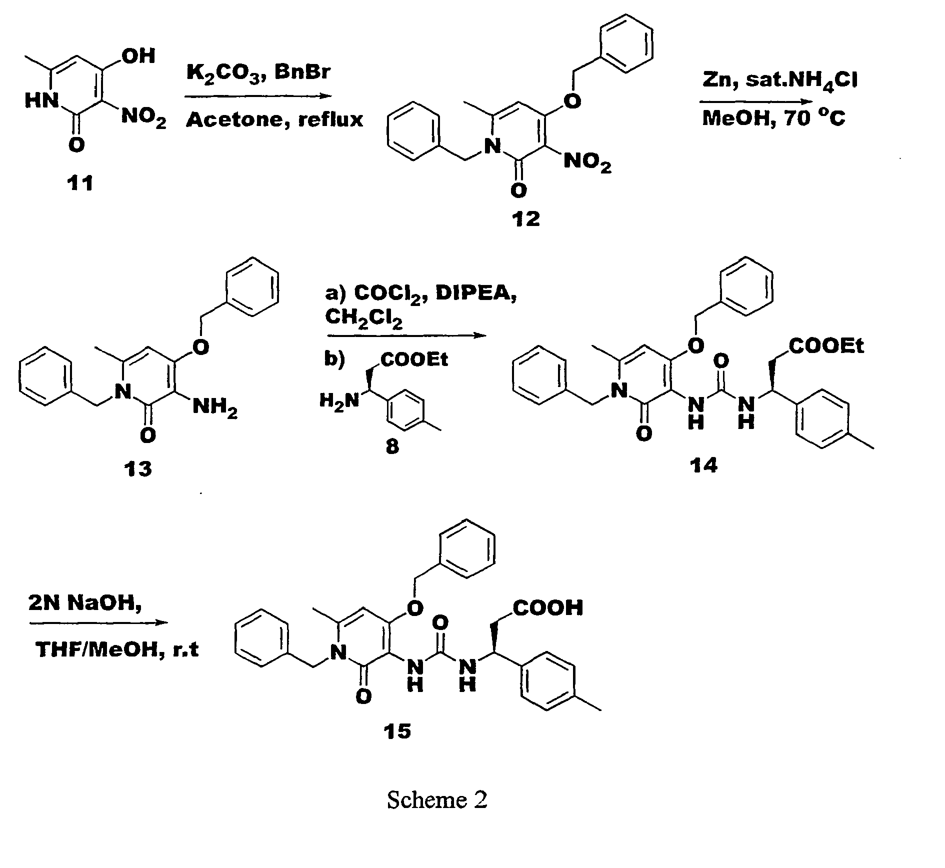

Scheme 3, illustrating the procedure of Example 3, is shown below.

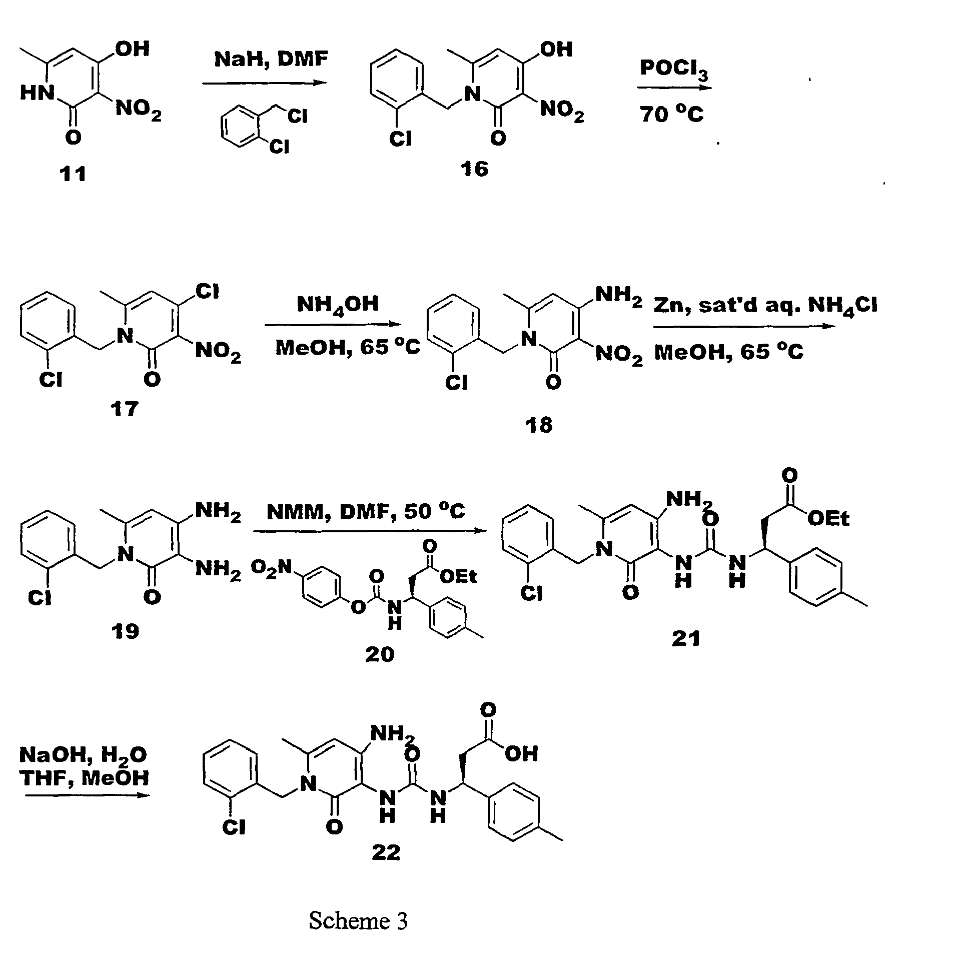

Scheme 4, illustrating the procedure of Example 4, is shown below.

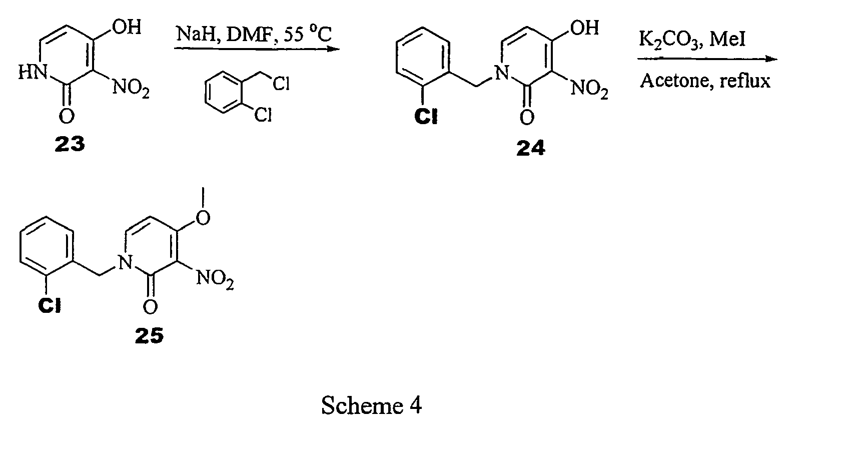

Scheme 5, illustrating the procedure of Example 5, is shown below.

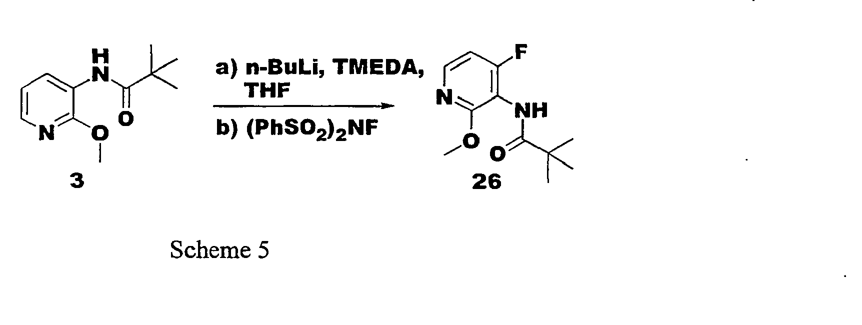

Scheme 6, illustrating the procedure of Example 6, is shown below.

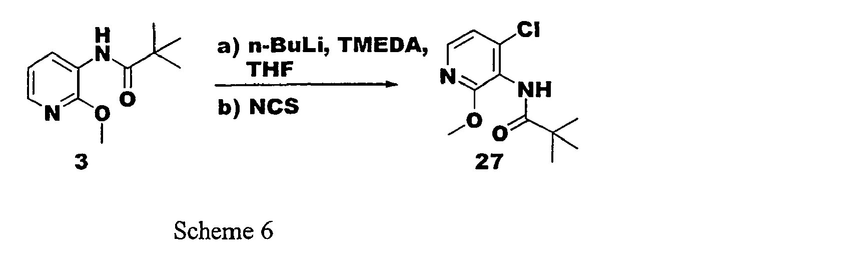

Scheme 7, illustrating the procedure of Example 7, is shown below.

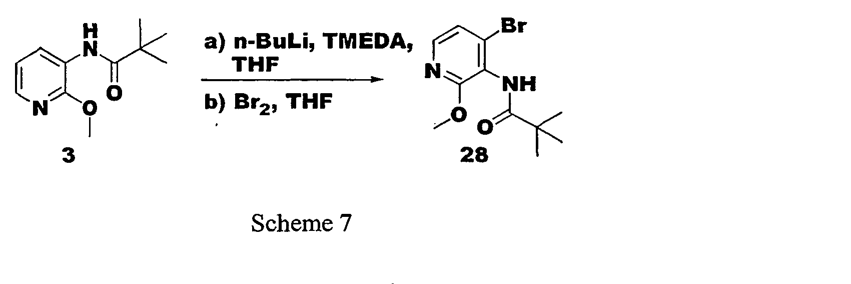

Scheme 8, illustrating the procedure of Example 8, is shown below.

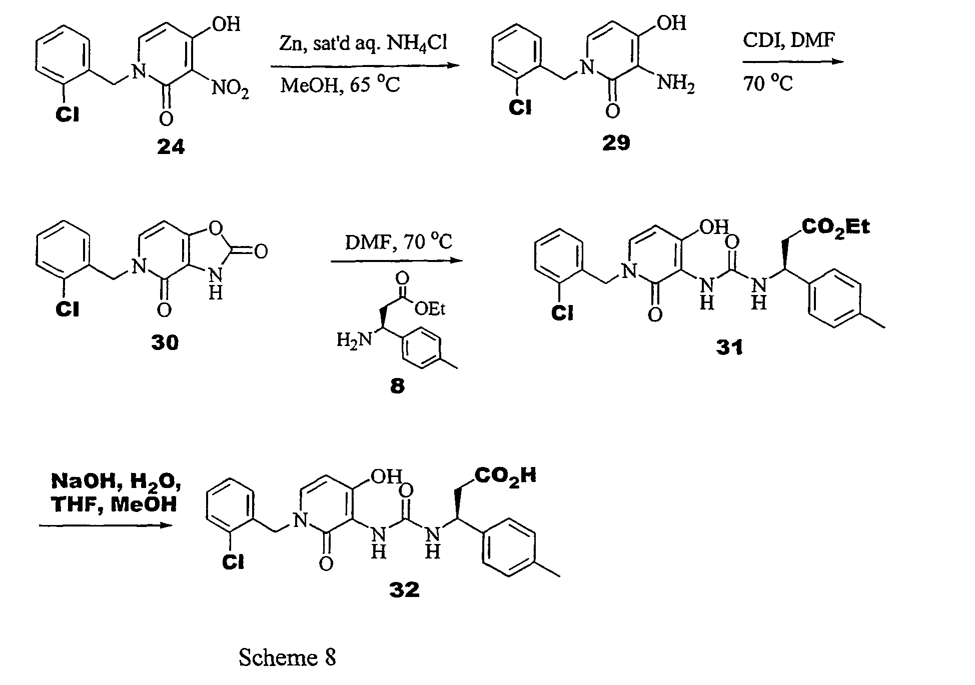

Scheme 9, illustrating the procedure of Example 9, is shown below.

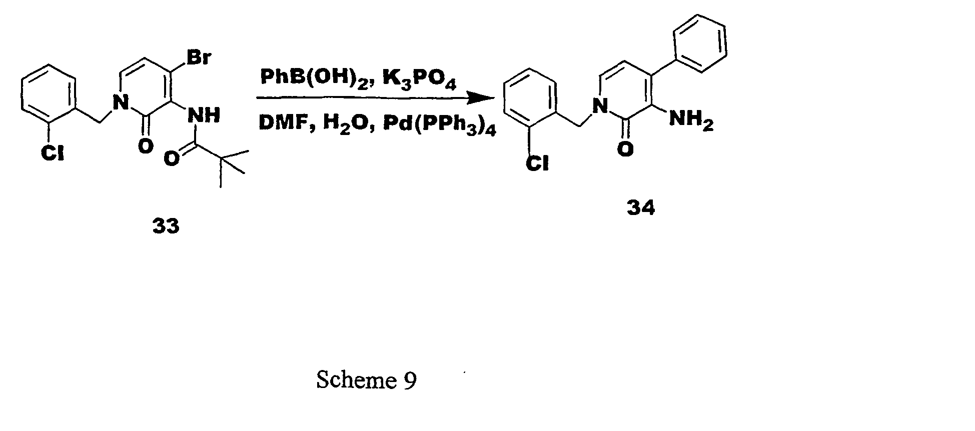

Scheme 10, illustrating the procedure of Example 10, is shown below.

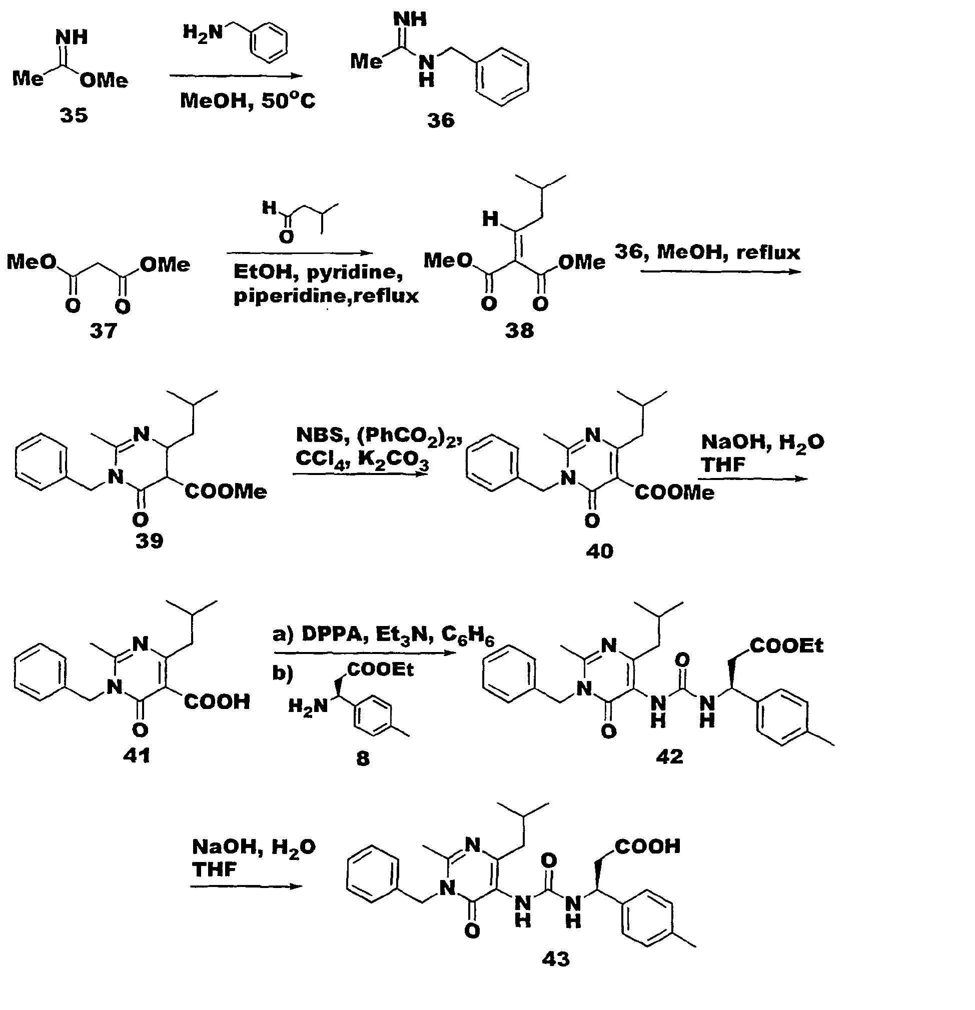

Scheme 11, illustrating the procedure of Example 11, is shown below.

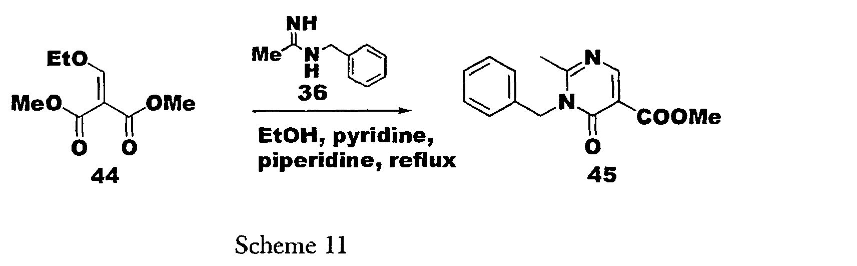

Scheme 12, illustrating the procedure of Example 12, is shown below.

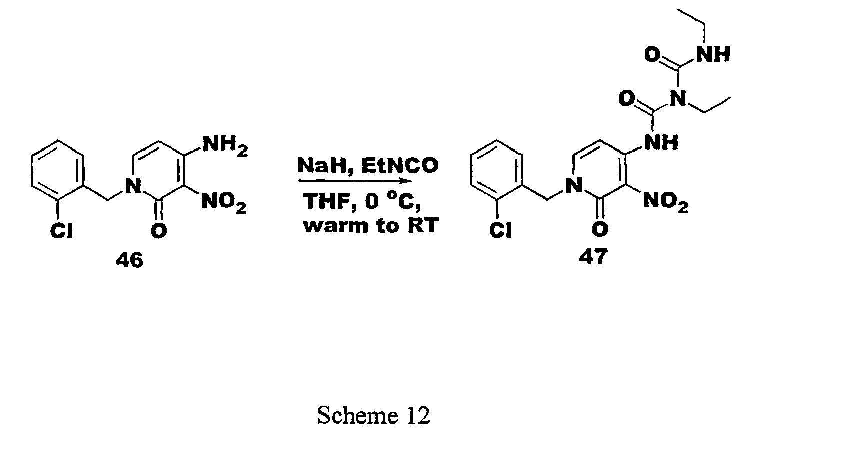

Scheme 13, illustrating the procedure of Example 13, is shown below.

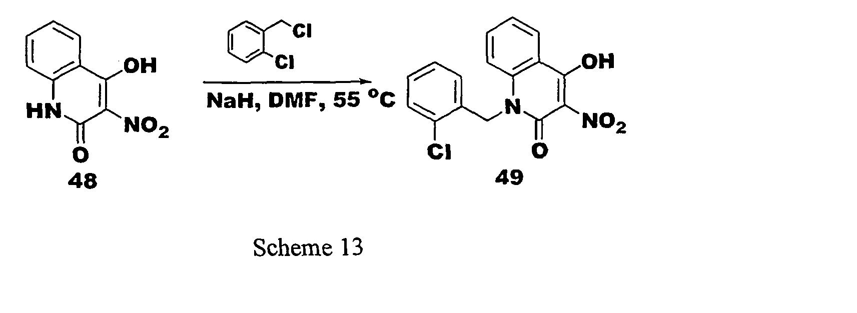

Scheme 14, illustrating the procedure of Example 14, is shown below.

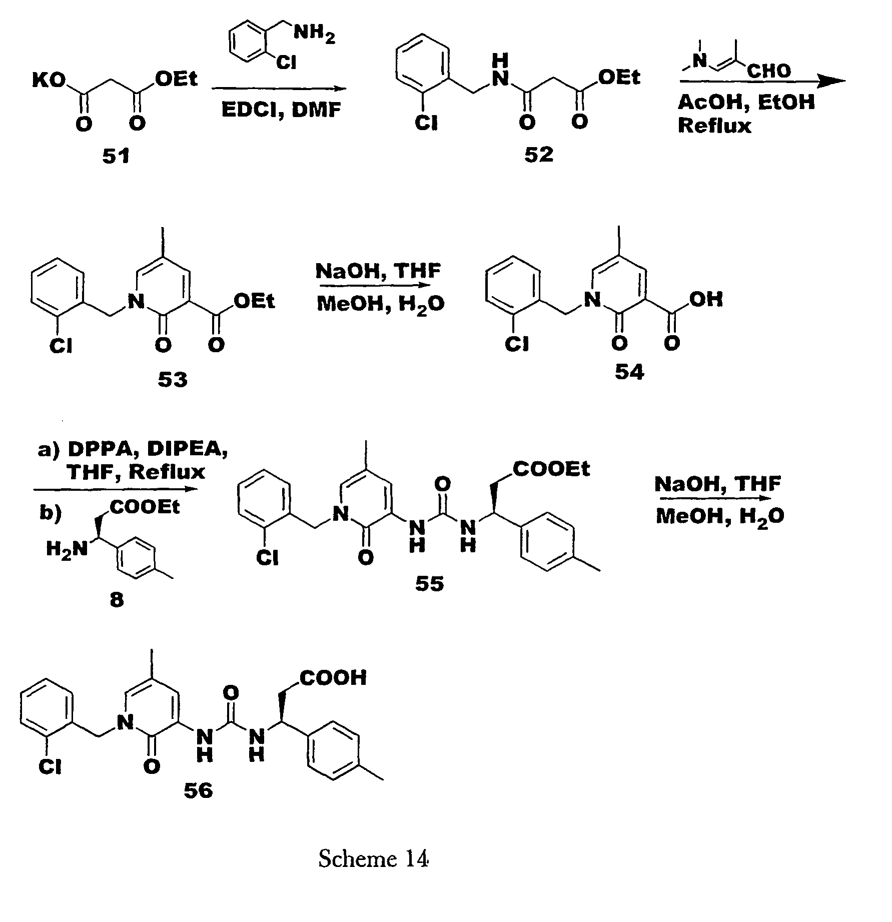

Scheme 15, illustrating the procedure of Example 15, is shown below.

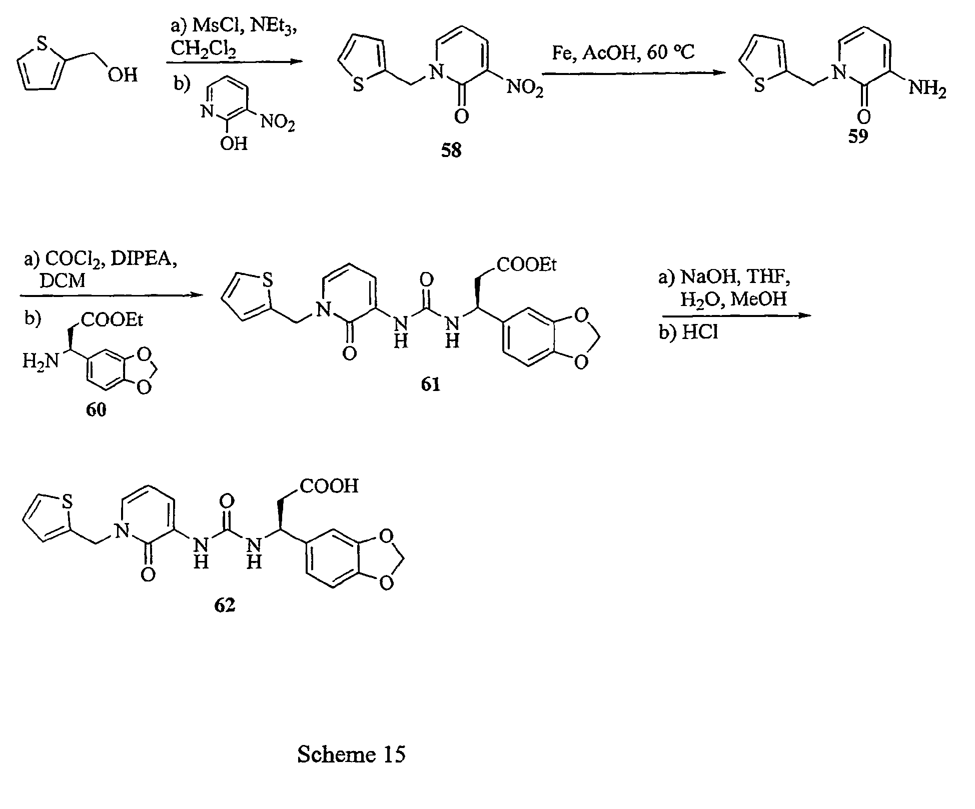

Scheme 16, illustrating the procedure of Example 16, is shown below.

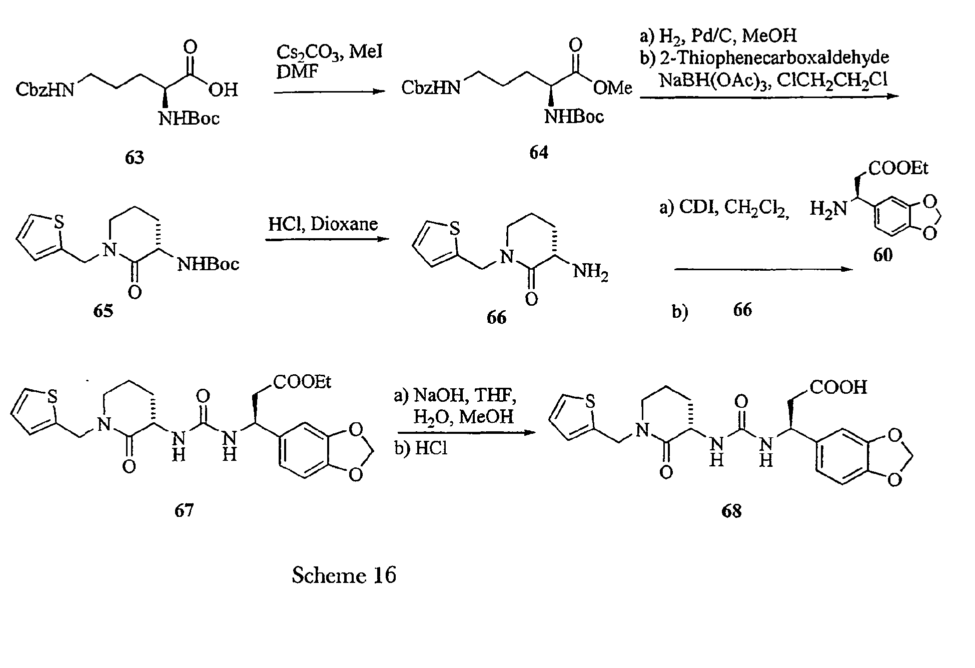

### Scheme 17, illustrating the procedure of Example 17, is shown below

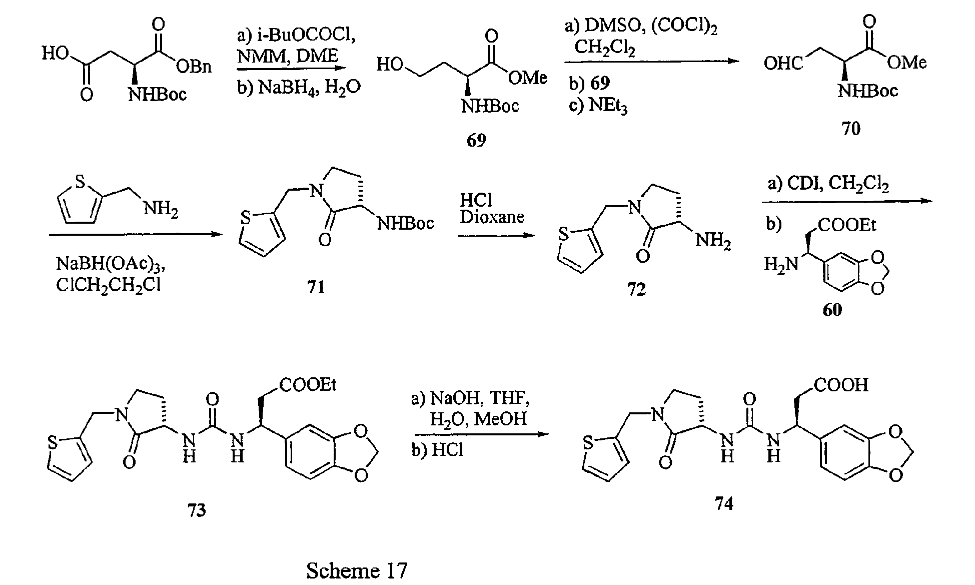

Scheme 18, illustrating the procedure of Example 18, is shown below.

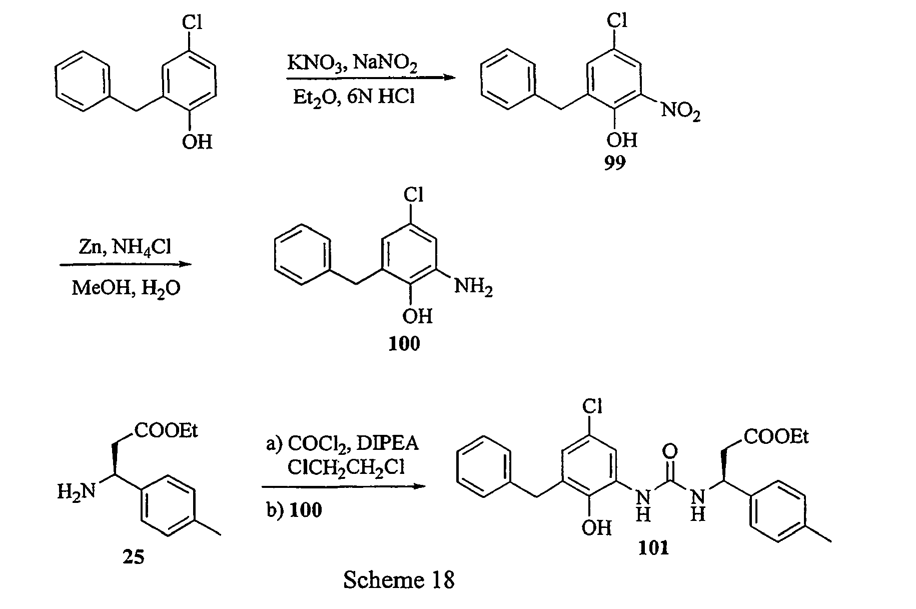

Scheme 19, illustrating the procedure of Example 19, is shown below.

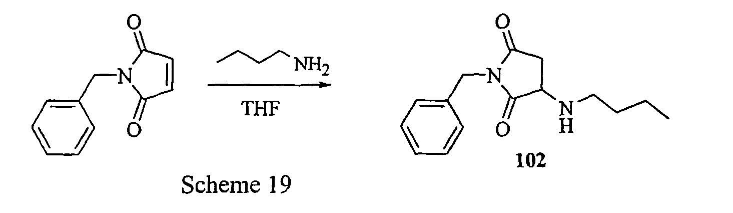

Scheme 20, illustrating the procedure of Example 20, is shown below.

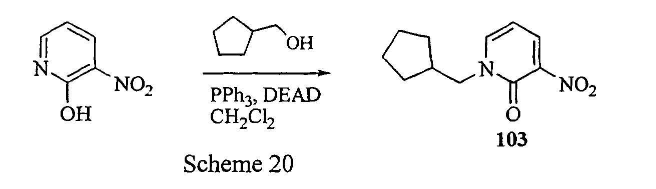

Scheme 21, illustrating the procedure of Example 21, is shown below.

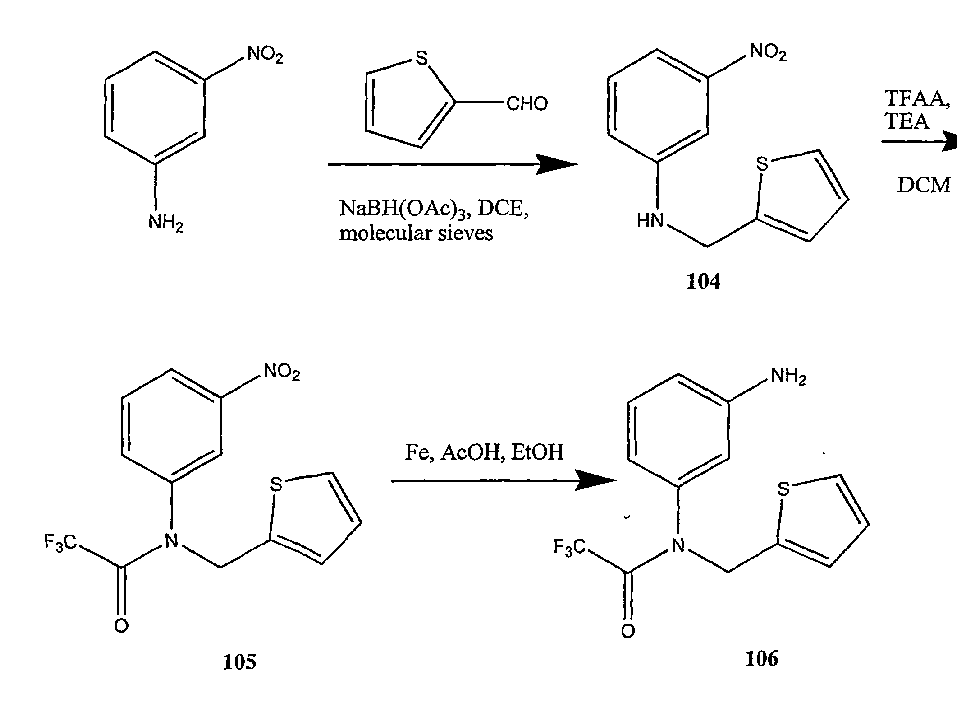

Scheme 22, illustrating the procedure of Example 22, is shown below.

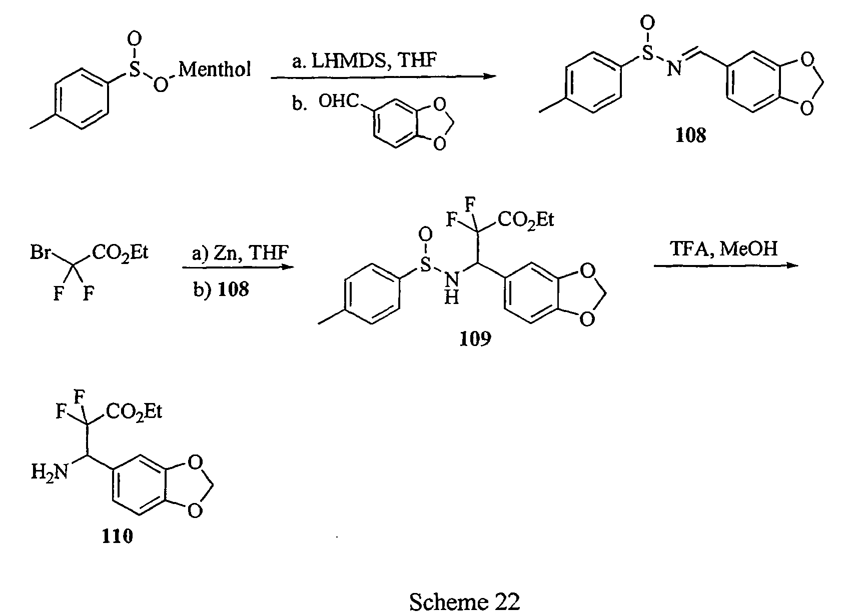

Scheme 23, illustrating the procedure of Example 23, is shown below.

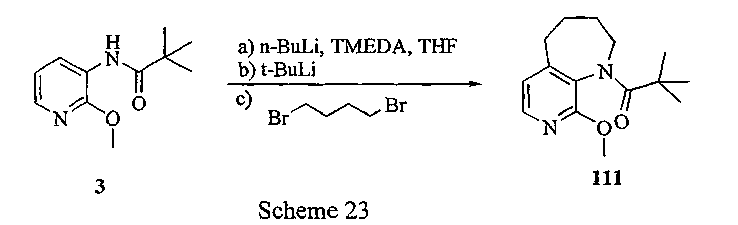

Scheme 24, illustrating the procedure of Example 24, is shown below.

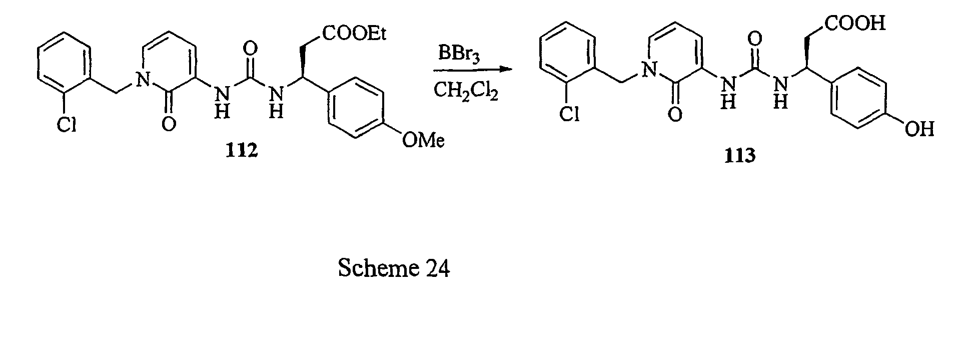

The compounds of the present invention can be used in the form of pharmaceutically acceptable salts derived from inorganic or organic acids. The phrase "pharmaceutically acceptable salt" means those salts which are, within the scope of sound medical judgement, suitable for use in contact with the tissues of humans and lower animals without undue toxicity, irritation, allergic response and the like and are commensurate with a reasonable benefit/risk ratio. Pharmaceutically acceptable salts are well-known in the art. For example, S. M. Berge et al. describe pharmaceutically acceptable salts in detail in J. Pharmaceutical Sciences, 1977, 66: 1 *et seq.* The salts can be prepared *in situ* during the final isolation and purification of the compounds of the invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, fumarate, hydrochloride, hydrobromide, hydroiodide, 2-hydroxyethansulfonate (isothionate), lactate, maleate, methanesulfonate, nicotinate, 2-naphthalenesulfonate, oxalate, palmitoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quaternized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which can be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

Basic addition salts can be prepared *in situ* during the final isolation and purification of compounds of this invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylammonium, dimethylammonium, trimethylammonium, triethylammonium, diethylammonium, and ethylammonium among others. Other representative organic amines useful for the formation of base addition salts include ethylenediamine, ethanolamine, diethanolamine, piperidine, piperazine and the like.

Dosage forms for topical administration of a compound of this invention include powders, sprays, ointments and inhalants. The active compound is mixed under sterile conditions with a pharmaceutically acceptable carrier and any needed preservatives, buffers or propellants which can be required. Opthalmic formulations, eye ointments, powders and solutions are also contemplated as being within the scope of this invention.

Actual dosage levels of active ingredients in the pharmaceutical compositions of this invention can be varied so as to obtain an amount of the active compound(s) which is effective to achieve the desired therapeutic response for a particular patient, compositions and mode of administration. The selected dosage level will depend upon the activity of the particular compound, the route of administration, the severity of the condition being treated and the condition and prior medical history of the patient being treated. However, it is within the skill of the art to start doses of the compound at levels lower than required to achieve the desired therapeutic effect and to gradually increase the dosage until the desired effect is achieved.

When used in the above or other treatments, a therapeutically effective amount of one of the compounds of the present invention can be employed in pure form or, where such forms exist, in pharmaceutically acceptable salt, ester or prodrug form. Alternatively, the compound can be administered as a pharmaceutical composition containing the compound of interest in combination with one or more pharmaceutically acceptable excipients. The phrase "therapeutically effective amount" of the compound of the invention means a sufficient amount of the compound to treat disorders, at a reasonable benefit/risk ratio applicable to any medical treatment. It will be understood, however, that the total daily usage of the compounds and compositions of the present invention will be decided by the attending physician within the scope of sound medical judgement. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than required to achieve the desired therapeutic effect and to gradually increase the dosage until the desired effect is achieved.

The total daily dose of the compounds of this invention administered to a human or lower animal may range from about 0.0001 to about 1000 mg/kg/day. For purposes of oral administration, more preferable doses can be in the range of from about 0.001 to about 5 mg/kg/day. If desired, the effective daily dose can be divided into multiple doses for purposes of administration; consequently, single dose compositions may contain such amounts or submultiples thereof to make up the daily dose.

The present invention also provides pharmaceutical compositions that comprise compounds of the present invention formulated together with one or more non-toxic pharmaceutically acceptable carriers. The pharmaceutical compositions can be specially formulated for oral administration in solid or liquid form, for parenteral injection or for rectal administration.

The pharmaceutical compositions of this invention can be administered to humans and other mammals orally, rectally, parenterally , intracisternally, intravaginally, intraperitoneally, topically (as by powders, ointments or drops), bucally or as an oral or nasal spray. The term "parenterally," as used herein, refers to modes of administration which include intravenous, intramuscular, intraperitoneal, intrastemal, subcutaneous and intraarticular injection and infusion.

In another aspect, the present invention provides a pharmaceutical composition comprising a component of the present invention and a physiologically tolerable diluent. The present invention includes one or more compounds as described above formulated into compositions together with one or more non-toxic physiologically tolerable or acceptable diluents, carriers, adjuvants or vehicles that are collectively referred to herein as diluents, for parenteral injection, for intranasal delivery, for oral administration in solid or liquid form, for rectal or topical administration, among others.

The compositions can also be delivered through a catheter for local delivery at a target site, *via* an intracoronary stent (a tubular device composed of a fine wire mesh), or via a biodegradable polymer. The compounds may also be complexed to ligands, such as antibodies, for targeted delivery.

Compositions suitable for parenteral injection may comprise physiologically acceptable, sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), vegetable oils (such as olive oil), injectable organic esters such as ethyl oleate, and suitable mixtures thereof.

These compositions can also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride and the like. Prolonged absorption of the injectable pharmaceutical form can be brought about by the use of agents delaying absorption, for example, aluminum monostearate and gelatin.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

In some cases, in order to prolong the effect of the drug, it is desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microeniulsions which are compatible with body tissues.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Solid dosage forms for oral administration include capsules, tablets, pills, powders and granules. In such solid dosage forms, the active compound may be mixed with at least one inert, pharmaceutically acceptable excipient or carrier, such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills and granules can be prepared with coatings and shells such as enteric coatings and other coatings well-known in the pharmaceutical formulating art. They may optionally contain opacifying agents and may also be of a composition such that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

Besides inert diluents, the oral compositions may also include adjuvants such as wetting agents, emulsifying and suspending agents, sweetening, flavoring and perfuming agents.

Compositions for rectal or vaginal administration are preferably suppositories which can be prepared by mixing the compounds of this invention with suitable nonirritating excipients or carriers such as cocoa butter, polyethylene glycol or a suppository wax which are solid at room temperature but liquid at body temperature and therefore melt in the rectum or vaginal cavity and release the active compound.

Compounds of the present invention can also be administered in the form of liposomes. As is known in the art, liposomes are generally derived from phospholipids or other lipid substances. Liposomes are formed by mono- or multi-lamellar hydrated liquid crystals which are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients and the like. The preferred lipids are natural and synthetic phospholipids and phosphatidyl cholines (lecithins) used separately or together.

Methods to form liposomes are known in the art. See, for example, Prescott, Ed., Methods in Cell Biology, Volume XIV, Academic Press, New York, N.Y. (1976), p. 33 *et seq.*

The term "pharmaceutically acceptable prodrugs" as used herein represents those prodrugs of the compounds of the present invention which are, within the scope of sound medical judgement, suitable for use in contact with the tissues of humans and lower animals without undue toxicity, irritation, allergic response, and the like, commensurate with a reasonable benefit/risk ratio, and effective for their intended use, as well as the zwitterionic forms, where possible, of the compounds of the invention. Prodrugs of the compounds of the present invention may be rapidly transformed *in vivo* to the parent compound of the above formula, for example, by hydrolysis in blood. A thorough discussion is provided in T. Higuchi and V. Stella, Pro-drugs as Novel Delivery Systems, V. 14 of the A.C.S. Symposium Series, and in Edward B. Roche, ed., Bioreversible Carriers in Drug Design, American Pharmaceutical Association and Pergamon Press (1987), hereby incorporated by reference.

Compounds of the present invention may exist as stereoisomers wherein asymmetric or chiral centers are present. These stereoisomers are "R" or "S" depending on the configuration of substituents around the chiral carbon atom. The present invention contemplates various stereoisomers and mixtures thereof. Stereoisomers include enantiomers and diastereomers, and mixtures of enantiomers or diastereomers. Individual stereoisomers of compounds of the present invention may be prepared synthetically from commercially available starting materials which contain asymmetric or chiral centers or by preparation of racemic mixtures followed by resolution well-known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary or (2) direct separation of the mixture of optical enantiomers on chiral chromatographic columns.

The compounds of the invention can exist in unsolvated as well as solvated forms, including hydrated forms, such as hemi-hydrates. In general, the solvated forms, with pharmaceutically acceptable solvents such as water and ethanol among others are equivalent to the unsolvated forms for the purposes of the invention.

In another aspect, the present invention contemplates the use of the compounds of Formula III or pharmaceutically acceptable salts thereof for the manufacture of a medicament for selectively inhibiting the binding of α₄β₁, integrin to VCAM-1. The inhibiting of binding can be either *in vitro* or *in vivo.* In accordance with a process of the present invention, a cell expressing α₄β₁ integrin is exposed to a cell expressing VCAM-1 in the presence of an effective inhibiting amount of a compound of the present invention.

A cell expressing α₄β₁ integrin can be a naturally occurring white blood cell, mast cell or other cell type that naturally expresses α₄β₁ on the cell surface, or a cell transfected with an expression vector that contains a poly-nucleotide (e.g., genomic DNA or cDNA) that encodes α₄β₁ integrin. In an especially preferred embodiment, α₄β₁integrin is present on the surface of a white blood cell such as a monocyte, a lymphocyte or a granulocyte (e.g., an eosinophil or a basophil).

A cell that expresses VCAM-1 can be a naturally occurring cell (e.g. an endothelial cell) or a cell transfected with an expression vector containing a polynucleotide that encodes VCAM-1. Methods for producing transfected cells that express VCAM-1 are well known in the art.

Where VCAM-1 exists on the surface of cell, the expression of that VCAM-1 is preferably induced by inflammatory cytokines such as tumor necrosis factor-α interleukin-4 and interleukin-1β.

Where the cells expressing α₄β₁ integrin and VCAM-1 are in a living organism, a compound of the present invention is administered in an effective amount to the living organism. Preferably, the compound is in a pharmaceutical composition of this invention. A process of the present invention is especially useful in treating diseases associated with uncontrolled migration of white blood cells to damaged tissue. Such diseases include, but are not limited to, asthma, atherosclerosis, rheumatoid arthritis, allergy, multiple sclerosis, lupus, inflammatory bowel disease, graft rejection, contact hypersensitivity, type I diabetes, leukemia, and brain cancer. Administration is preferably accomplished *via* intravascular, subcutaneous, intranasal, transdermal or oral delivery.

The present invention also provides the use of the compounds of Formula III or pharmaceutically acceptable salts thereof for the manufacture of a medicament for selectively inhibiting the binding of α₄β₁ integrin to a protein comprising exposing the integrin to the protein in the presence of an effective inhibiting amount of a compound of the present invention. In a preferred embodiment, the α₄β₁ integrin is expressed on the surface of a cell, either naturally occurring or a cell transformed to express α₄β₁ integrin.

The protein to which the α₄β₁, integrin binds can be expressed either on a cell surface or be part of the extracellular matrix. Especially preferred proteins are fibronectin or invasin.

The ability of compounds of the present invention to inhibit binding is described in detail hereinafter in the Examples. These Examples are presented to describe preferred embodiments and utilities of the invention and are not meant to limit the invention unless otherwise stated in the claims appended hereto.

The ability of compounds of the present invention to inhibit binding is described in detail hereinafter in the Examples. These Examples are presented to describe preferred embodiments and utilities of the invention and are not meant to limit the invention unless otherwise stated in the claims appended hereto.

### Example 1

### Synthesis of (3S)-3-{[({1-[(2-chlorophenyl)methyl]-4-ethyl-2-oxo-1,2-dihydro-3-pyridinyl}amino)carbonyl]amino}-3-(4-methylphenyl)propanoic acid (10).

Step One: Compound **1** (20.8 g, 135 mmol) was dissolved in methanol (270 mL) and palladium on carbon (10 % Pd dry weight basis, Degussa type E101 NE/W, ~50% water content, 5.75 g, 2.7 mmol Pd) was added. The atmosphere was replaced with hydrogen (toggle between vacuum and hydrogen from a balloon five times), the mixture was stirred overnight, then filtered. The filtrate was concentrated under vacuum and the residue was taken up in a 1:1 hexanes:ethyl acetate mixture and washed with a 4:1 mixture of water and saturated NaHCO₃, saturated NaHCO₃ and brine. The organic layer was dried over MgSO₄ and filtered and the filtrate was concentrated under reduced pressure to give compound 2 (12.43 g, 74%) as a white solid. This material was used without purification.

Step Two: Compound **2** (2.64 g, 21.3 mmol) was dissolved in dichloromethane (50 mL) and chilled to 0 °C. The cold solution was treated sequentially with triethylamine (3.6 mL, 25.6 mmol) and trimethylacetyl chloride (2.90 mL, 23.4 mmol). The solution was stirred at room temperature for 6 hours, then refluxed overnight. The mixture was partitioned between dichloromethane and aqueous NaOH (2N). The organic layer was washed with brine, dried over MgSO₄ and filtered and the filtrate was concentrated to give compound 3 (3.33 g, 75%).

Step Three: Compound **3** (0.50 g, 2.4 mmol) was dissolved in dry THF, (9.6 mL) and TMEDA (1.1 mL, 7.2 mmol) under a dry nitrogen atmosphere. The resulting solution was chilled to between -20 and -10 °C and treated sequentially with n-butyllithium (1.6 M in hexanes 2.25 mL) and t-butyllithium (1.7 M in pentane, 2.1 mL) dropwise *via* syringe. After 30 minutes the bath temperature was allowed to come to -5 to 0 °C and treated with ethyl iodide *via* a syringe (0.77 mL, 9.6 mmol). The solution was stirred at 0 °C for 2 hours, then room temperature overnight. The mixture was quenched with methanol and concentrated to dryness. The residue was purified by filtering through silica gel, eluting with 3:1 hexanes:ethyl acetate and then recrystallizing from hexanes to yield compound **4** (0.32 g, 56%).

Step Four: Compound **4** (0.32 g, 1.3 mmol) was dissolved in glacial acetic acid (4.5 mL) and treated with potassium iodide (0.65 g, 3.9 mmol). The resulting mixture was heated in an oil bath regulated at 115 °C for 1.0 hour. The mixture was cooled, diluted with water and adjusted to pH 6 using 2N NaOH and 2N HCl. The mixture was extracted with chloroform (4 times). The combined extracts were washed with aqueous sodium thiosulfate, dried over MgSO₄ and filtered. The filtrate was concentrated under reduced pressure to give compound **5** (0.25 g, 86%) as a white solid. This material was used without further purification.

Step Five: Compound **5** (0.25 g, 1.1 mmol) was dissolved in THF (45 mL) and treated dropwise with a solution of potassium bis(trimethylsilyl)amide (0.5 M in toluene, 2.7 mL) at 0 °C. The resulting solution was treated with 2-chlorobenzylbromide (0.16 mL, 1.2 mmol) and the solution was allowed to warm to room temperature overnight. The mixture was partitioned between 2N HCl and ethyl acetate. The organic layer was washed with brine, dried over MgSO₄ and filtered. The filtrate was concentrated under reduced pressure and the residue was purified by chromatography (SiO₂, gradient elution 4:1 switching to 2:1 hexanes:ethyl acetate) to give compound **6** (0.16 g, 41%).

Step Six: Compound **6** (0.16 g, 0.46 mmol) was suspended in 1:1 water:concentrated HCl (4.6 mL). The suspension was brought to reflux for 4 hours, during which time the compound dissolved. The mixture was cooled, diluted with water and extracted with diethyl ether. The aqueous layer adjusted basic with excess saturated sodium bicarbonate solution, and the mixture was extracted with ethyl acetate. The extracts were combined, washed with brine, dried over MgSO₄ and filtered. The filtrate was concentrated under reduced pressure to give compound **7** (0.081 g, 67%).

Step Seven: Compound **7** (0.080 g, 0.30 mmol) was dissolved in 1,2-dichloroethane (1.2 mL) and DIPEA (0.115 mL, 0.66 mmol) and chilled to 0 °C. The cold solution was treated rapidly with a solution of phosgene (1.93 M in toluene, 0.170 mL, 0.33 mmol). After 30 minutes a solution of compound **8** (0.068 g, 0.33 mmol) in 1,2-dichloroethane (0.5 mL) was added rapidly *via* syringe. The resulting mixture was heated to 55 °C. for 1 hour. The mixture was partitioned between dichloromethane and 2N HCl. The organic layer was washed with saturated aqueous NaHCO₃ and brine, dried over MgSO₄ and filtered. The filtrate was concentrated to give compound **9** (0.110 g, 74%).

Step Eight : Compound **9** (0.11 g, 0.22 mmol) was dissolved in 2:1 THF:H₂O (0.88 mL) and treated with a solution of 2N NaOH (0.33 mL). Methanol was added dropwise until a homogeneous solution was obtained. The mixture was stirred for 20 minutes, diluted with water and washed with ethyl ether. The aqueous layer was acidified with 2N HCl and extracted with ethyl acetate. The ethyl acetate layer was washed with brine, dried over MgSO₄ and filtered. The filtrate was concentrated to give (3S)-3-{[({1-[(2-chlorophenyl)methyl]-4-ethyl-2-oxo-1,2-dihydro-3-pyridinyl}amino)carbonyl]amino}-3-(4-methylphenyl)propanoic acid (10, 0.095 g, 92%).

### Example 2

### Synthesis of (3S)-3-{[({6-methyl-2-oxo-1-(phenylmethyl)-4-[(phenylmethyl)oxy]-1,2-dihydro-3-pyridinyl}amino)carbonyl]amino}-3-(4-methylphenyl)propanoic acid (15).

Step One: To a suspension of compound 11 (1.0 g, 5.9 mmol) and K₂CO₃ (2.40 g 17.6 mmol) in acetone (50 mL) was added benzylbromide (2.31 g, 13.5 mmol). After refluxing overnight, the reaction was cooled and the mixture was partitioned between ethyl acetate and saturated NaHCO₃. The organic layer was washed with dilute HCl and brine, dried over MgSO₄ and filtered and the filtrate was concentrated to give compound **12** (1.60 g, 80%).

Step Two: Compound **12** (0.30 g, 0.86 mmol), zinc powder (0.30 g, 4.6 mmol) and saturated aqueous NH₄Cl (0.30 mL) were mixed in MeOH (18 mL). This mixture was allowed to stir at room temperature for 1 hour before additional zinc (0.30 g, 4.6 mmol) was added. The resulting heterogeneous mixture was refluxed overnight. After filtration of the hot mixture and concentration of the filtrate under reduced pressure, the residue was dissolved in ethyl acetate and washed with saturated aqueous NaHCO₃ and brine. The organic layer was dried over MgSO₄ and filtered and the filtrate was concentrated under reduced pressure to give compound **13** (0.18 g, 66%).

Step Three: Compound **13** (0.30 g, 0.94 mmol.) and DIPEA (0.40 mL, 2.3 mmol.) were dissolved in CH₂Cl₂ and the mixture was cooled to 0 °C. Phosgene (1.9 M in toluene, 0.55 mL, 1.0 mmol) was added to the solution dropwise. The reaction mixture was stirred at 0 °C for 15 minutes before compound **8** (0.19 g, 0.94 mmol) in CH₂Cl₂ (2 mL) was added. The resulting solution was stirred at room temperature overnight then poured into ethyl acetate and washed with saturated aqueous NaHC0₃, 1 N HCl and brine. The organic layer was dried over MgSO₄ and filtered and the filtrate was concentrated under reduced pressure. The residue was purified by flash chromatography on silica gel, eluting with 1:1 increasing to 1:2 hexanes:ethyl acetate to give compound **14** (0.33 g, 64%).

Step Four: A solution of compound **14** ( 0.33 g, 0.6 mmol) in THF (6 mL) was treated with 2N NaOH (2 mL). MeOH was added until homogeneous solution was achieved. The reaction mixture was stirred at room temperature for 30 minutes and poured into H₂O (50 mL). The aqueous layer was washed with diethyl ether (twice), and then acidified with 1N HCl. The aqueous layer was extracted with ethyl acetate (twice). The combined ethyl acetate extracts were washed with brine (twice), dried over MgSO₄ and filtered. The filtrate was concentrated under reduced pressure to give (3S)-3-{[({6-methyl-2-oxo-1-(phenylmethyl)-4-[(phenylmethyl)oxy]-1,2-dihydro-3-pyridinyl}amino)carbonyl]amino}-3-(4-methylphenyl)propanoic acid (15, 0.26 g, 90%) as an off-white solid. Melting point: 124-126 °C.

### Example 3

Synthesis of (3S)-3-{[({4-amino-1-[(2-chlorophenyl)methyl]-6-methyl-2-oxo-1,2-dihydro-3-pyridinyl}amino)carbonyl]amino}-3-(4-methylphenyl)propanoic acid (**22**).

Step One: To a solution of compound **11** (10.00 g, 58.8 mmol) in anhydrous DMF (120 mL) at 0°C was added NaH (60% dispersion in mineral oil, 5.40 g, 135 mmol). The mixture was stirred at 0 °C for 15 minutes before the addition of 2-chlorobenzylchloride (12.3 g, 76.4 mmol). After stirring at 55 °C overnight, the mixture was poured into ice-water and washed with Et₂O twice. The aqueous layer was acidified and filtration of the resulting precipitate gave compound **16** (14.7 g, 85%).

Step Two: To a flask containing compound 16 (8.00 g, 28.6 mmol) sealed with a rubber septum and balloon at room temperature under dry nitrogen atmosphere, POCl₃ (30.0 ml, 322 mmol) was added *via* syringe. The nitrogen line was removed and the reaction mixture was stirred overnight at 70 °C, then poured over ice (300ml) and stirred for 30 minutes. The resulting mixture was extracted with dichloromethane (300 ml) and the organic phase was dried over MgSO₄ and filtered. The filtrate was concentrated under reduced pressure to give compound **17** (7.3g, 86%) as a dark brown solid.

Step Three: To a 250 ml flask equipped with condenser and rubber septum fitted with a balloon, a solution of compound **17** (2.1g, 7.05 mmol), methanol (55ml) and aqueous ammonium hydroxide (28-30%, 70.0 ml, 1.14 mol) were added at room temperature. The reaction mixture was heated to 65 °C for 60 hours open only to the balloon. The mixture was filtered and the filtrate was concentrated under reduced pressure to yield compound **18** (1.5 g, 76%) as a brown solid.

Step Four: To a solution of compound **18** (0.3g, 1.02 mmol) in methanol (50 ml) at room temperature, saturated aqueous ammonium chloride (2 ml) and zinc dust (0.30 g, 4.6 mmol) were added sequentially. After stirring 30 minutes at room temperature, additional zinc was added (0.30 g, 4.6 mmol) and the reaction mixture was refluxed overnight. The reaction mixture was filtered hot and the filtrate was concentrated under reduced pressure. The residue was partitioned between ethyl acetate and 1N NaOH. The solution was filtered and the aqueous phase extracted with ethyl acetate. The combined organic phases were dried over MgSO₄ and filtered. The filtrate was concentrated under reduced pressure to yield compound **19** (0.21g, 78%) as a brown solid.

Step Five: A solution of compound **19** (0.10 g, 0.38 mmol), NMM (0.040 mL, 0.38 mmol) and compound **20** (0.14 g, 0.38 mmol) in anhydrous DMF (5 mL) was heated to 50 °C overnight. The mixture was cooled and diluted with ethyl acetate (60 mL). The organic layer was washed with 0.5N NaOH (3 x 30 mL) and brine, dried over MgSO₄ and filtered. The filtrate was concentrated under reduced pressure and the residue was purified by flash chromatography on silica gel, eluting with 9:1 increasing to 17:3 CHCl₃:MeOH to give compound **21** (0.120 g, 65%) as a yellow foam.

Step Six: A solution of compound **21** (0.120 g, 0.25 mmol) in THF (6 mL) was treated with 2N NaOH (2 mL). Methanol was added until a homogeneous solution was achieved. The reaction mixture was stirred at room temperature for 30 minutes and poured into H₂O (50 mL). The aqueous layer was washed with diethyl ether (twice), and then acidified with 1N HCl. The aqueous layer was extracted with ethyl acetate (twice). The combined ethyl acetate extracts were washed with brine (twice), dried over MgSO₄ and filtered. The filtrate was concentrated under reduced pressure to give (3S)-3-{[({4-amino-1-[(2-chlorophenyl)methyl]-6-methyl-2-oxo-1,2-dihydro-3-pyridinyl} amino)-carbonyl]amino}-3-(4-methylphenyl)propanoic acid (22, 0.100 g, 89%) as an off-white solid. Melting point: 145-147 °C.

### Example 4

### Synthesis of (3S)-3-[({[1-[(2-chlorophenyl)methyl]-4-(methyloxy)-2-oxo-1,2-dihydro-3-pyridinyl]amino} carbonyl)amino]-3-(4-methylphenyl)propanoic acid.

Step One: To a solution of compound **23** (10.00 g, 64.0 mmol) in anhydrous DMF (130 mL) at 0°C was added NaH (60% dispersion in mineral oil, 5.90 g, 147 mmol). The mixture was stirred at 0 °C for 15 minutes before the addition of 2-chlorobenzylchloride (13.4 g, 83.3 mmol). After stirring at 55 °C overnight, the mixture was poured into ice water and washed with Et₂O (twice). The aqueous layer was acidified and filtration of the resulting precipitate gave compound **24** (13.5 g, 75%).

Step Two: A suspension of compound **24** (1.0 g, 3.6 mmol), K₂CO₃(0.85 g, 6.2 mmol) and Mel (1.18 g, 8.3 mmol) in acetone (20 mL) was refluxed overnight. The reaction mixture was diluted with ethyl acetate and washed with saturated aqueous NaHCO₃, 1N HCl and brine. The organic layer was dried over MgSO₄ and filtered and the filtrate was concentrated under reduced pressure to give Compound **25** (0.74 g, 70%).

(3S)-3-[({[1-[(2-chlorophenyl)methyl]-4-(methyloxy)-2-oxo-1,2-dihydro-3-pyridinyl]amino}carbonyl)amino]-3-(4-methylphenyl)propanoic acid was prepared from compound **25** according to procedures described in Example 3. MS: Calculated: (M+H)⁺ = 469.93; Found: (M+H)⁺ = 470.01.

### Example 5

### Synthesis of (3S)-3-{[({1-[(2-chlorophenyl)methyl]-4-fluoro-2-oxo-1,2-dihydro-3-pyridinyl}amino)carbonyl]amino}-3-(4-methylphenyl)propanoic acid.

Step One: Compound **3** (0.65 g, 3.1 mmol) was dissolved in dry THF (12.4 mL) and TMEDA (0.90 mL, 6 mmol) under a dry nitrogen atmosphere. The resulting solution was chilled to between -15 and -10 °C and n-butyllithium (1.6 M in hexanes, 7.75 mL, 12.4 mmol) was added dropwise *via* syringe. After 1.5 hours, a solution of N-fluorobenzenesulfonimide (1.07g, 3.4 mmol) in THF (5 mL) was added to the cold solution rapidly *via* syringe. The solution was stirred at 0 °C for 1 hour, then room temperature for 3 hours. The mixture was quenched with water and extracted with chloroform (4 times). The combined organic extracts were washed with brine, dried over MgSO₄ and filtered. The filtrate was concentrated under reduced pressure and the residue was purified by chromatography, (SiO₂, plug gel, using 4:1 switching to 3:1 hexanes:ethyl acetate) to yield compound **26** (0.177g, 25%).

(3S)-3-{[({1-[(2-Chlorophenyl)methyl]-4-fluoro-2-oxo-1,2-dihydro-3-pyridinyl}amino)carbonyl]amino}-3-(4-methylphenyl)propanoic acid was prepared from Compound **26** according to procedures described in Example 1. MS: Calculated: (M+H)⁺ = 458.12; Found: (M+H)⁺ = 458.01.

### Example 6

### Synthesis of (3S)-4-chloro-3-{[({1-[(2-chlorophenyl)methyl]- 2-oxo-1,2-dihydro-3-pyridinyl}amino)carbonyl]amino}-3-(4-methylphenyl)propanoic acid.

Step One: Compound **3** (0.65 g, 3.1 mmol) was dissolved in THF (21 mL) and TMEDA (1.20 mL, 7.75 mmol) and chilled to -15°C. The solution was treated with n-butyllithium (1.6 M in hexanes, 4.8 mL, 7.8 mmol). The mixture was maintained between -20 and -10 °C for 1 hour, then cooled to -78 °C. Solid N-chlorosuccinimide (0.45 g, 3.4 mmol) was added while the apparatus was under a positive flow of nitrogen. The reaction was allowed to gradually warm to room temperature then stirred overnight. The mixture was quenched with water and extracted with chloroform (4 times). The organic layers were combined, dried over MgSO₄ and filtered. The filtrate was concentrated under reduced pressure and the residue was recrystallized from hexanes to give compound **27** (0.25 g, 33%).

(3S)-4-Chloro-3-{[({1-[(2-chlorophenyl)methyl]-2-oxo-1,2-dihydro-3-pyridinyl}amino)carbonyl]amino}-3-(4-methylphenyl)propanoic acid was prepared from compound 27 according to procedures described in Example 1.

### Example 7

### Synthesis of (3S)-4-bromo-3-{[({1-[(2-chlorophenyl)methyl]- 2-oxo-1,2-dihydro-3-pyridinyl}amino)carbonyl]amino}-3-(4-methylphenyl)propanoic acid.

Step One: Compound **3** (2.00g, 9.6 mmol) was dissolved in dry THF (32 mL) and TMEDA (2.20 mL, 14.4 mmol) under a dry nitrogen atmosphere. The resulting solution was chilled to between -20 and -10 °C and n-butyl lithium (1.60 M in hexanes, 18.0 mL, 28.8 mmol) was added dropwise *via* syringe. Upon completion of the addition, the solution was chilled to -78 °C and bromine (0.49 mL, 10.5 mmol) was added dropwise *via* syringe. The solution was allowed to warm slowly to room temperature overnight, then was quenched with water and extracted with chloroform. The organic layer was dried over MgSO₄ and filtered and the filtrate was concentrated under reduced pressure. The residue was recrystallized from hexanes to give compound **28** (1.32 g, 48%) as a tannish white solid.

(3S)-4-Bromo-3-{[({1-[(2-chlorophenyl)methyl]-2-oxo-1,2-dihydro-3-pyridinyl}amino)carbonyl]amino}-3-(4-methylphenyl)propanoic acid was prepared from compound **28** according to procedures described in Example 1.

### Example 8

### Synthesis of (3S)-3-{[({1-[(2-chlorophenyl)methyl]-4-hydroxy-2-oxo-1,2-dihydro-3-pyridinyl}amino)carbonyl]amino}-3-(4-methylphenyl)propanoic acid (32).

Step One: To a solution of compound **24** (1.5 g, 5.3 mmol) in methanol (50 ml) at room temperature, saturated ammonium chloride (1.5 mL) and zinc dust (1.5 g, 23 mmol) were added sequentially. After stirring 30 minutes at room temperature, additional zinc dust (1.5 g, 23 mmol) was added and the reaction mixture was refluxed overnight. The reaction mixture was filtered while hot and the filtrate was concentrated under reduced pressure. HCl (1N) was added to the resulting residue until the pH was approximately 4 and the resulting precipitate was collected by filtration to give compound **29** (0.80 g, 57%) as a brown solid.

Step Two: A solution of compound **29** (0.26 g, 1.0 mmol) and CDI (0.25 g, 1.6 mmol) in DMF (10 mL) was heated to 70 °C overnight. After cooling to room temperature, the mixture was diluted with ethyl acetate and washed with 1N HCl (3 times) and brine. The organic layer was dried over MgSO₄ and filtered and the filtrate was concentrated under reduced pressure to give compound **30** (0.14 g, 50%) as a brown solid.

Step Three: A solution of compound **30** (0.1 g, 0.36 mmol) and compound **8** (0.082 g, 0.40 mmol) in anhydrous DMF (5 mL) was heated to 70 °C overnight. The mixture was cooled, diluted with ethyl acetate and washed with 1N HCl (3 times) and brine. The organic layer was dried over MgSO₄ and filtered and the filtrate was concentrated under reduced pressure. The residue was purified by flash chromatography (SiO₂), eluting with 9:1 CHCl₃:MeOH to give compound **31** (0.17 g, 97%).

Step Four: A solution of compound **31** (0.170 g, 0.35 mmol) in THF (3 mL) was treated with 2N NaOH (1 mL). Methanol was added until a homogeneous solution was achieved. The reaction mixture was stirred at room temperature for 30 minutes and poured into H₂O (50 mL). The aqueous layer was washed with diethyl ether (twice), and then acidified with 1N HCl. The aqueous layer was extracted with ethyl acetate (twice). The combined ethyl acetate extracts were washed with brine (twice), dried over MgSO₄ and filtered. The filtrate was concentrated under reduced pressure to give (3S)-3-{[({1-[(2-chlorophenyl)methyl]-4-hydroxy-2-oxo-1,2-dihydro-3-pyridinyl}amino)carbonyl]amino}-3-(4-methylphenyl)propanoic acid (32, 0.150 g, 94%) as an off-white solid. Melting point: 113-115 °C.

### Example 9

### Synthesis of (3S)-3-{[({1-[(2-chlorophenyl)methyl]-2-oxo-4-phenyl-1,2-dihydro-3-pyridinyl}amino)carbonyl]amino}-3-(4-methylphenyl)propanoic acid.

Step One: Compound **33** (prepared from compound **28** according to procedures described in Example 1, 0.20 g, 0.50 mmol) was dissolved in DMF (1.8 mL) and water (0.7 mL) and treated with K₃PO₄ (0.39 g, 1.86 mmol) and phenyl boronic acid (0.113 g, 0.93 mmol). The resulting mixture was deoxygenated (switching between vacuum and nitrogen 5 times), then tetrakis(triphenylphosine)palladium(0) (8.7 mg, 0.050 mmol) was added. The mixture was deoxygenated as before and heated at 90 °C overnight. The mixture was cooled, diluted with water and extracted with ethyl acetate (2 times). The combined extracts were washed with brine, dried over MgSO₄ and filtered through silica gel and concentrated under reduced pressure. The residue was suspended in 1:1 water:concentrated HCl (2 mL) and acetonitrile (0.5 mL). The suspension was brought to reflux for 1 hour, then cooled, and partitioned between ethyl acetate and saturated aqueous NaHCO₃. The ethyl acetate layer was washed with brine, dried over MgSO₄, filtered, and concentrated under reduced pressure. The residue was purified by flash chromatography (SiO₂, 3:1 hexanes/ethyl acetate) to give compound **34** (0.115 g, 94%). This material was used without purification.

(3S)-3-{[({1-[(2-Chlorophenyl)methyl]-2-oxo-4-phenyl-1,2-dihydro-3-pyridinyl}amino)carbonyl]amino}-3-(4-methylphenyl)propanoic acid was prepared from Compound **34** according procedures described in Example 1. ¹H NMR (400 MHz, CD₃OD): 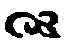 2.25 (s, 3H), 2.50 (m, 2H), 4.89 (t, J = 5.9 Hz, 1H), 5.34 (s, 2H), 6.40 (d, J = 7.0Hz, 1H), 7.0 (d, J = 8.0 Hz, 2H), 7.10 (d, J = 8.0 Hz, 2H), 7.18 (m, 1H), 7.28 (m, 2H), 7.35 (m, 3H), 7.43 (m, 1H), 7.49 (m, 3H).

### Example 10

### Synthesis of (3S)-3-[({[2-methyl-4-(2-methylpropyl)-6-oxo-1-(phenylmethyl)-1,6-dihydro-5-pyrimidinyl]amino}carbonyl)amino]-3-(4-methylphenyl)propanoic acid (43).

Step One: Compound **35** (2.00 g 18.2 mmol) was dissolved in 30 mL of dry methanol. To this was added benzylamine (1.97 g 18.2 mmol) and triethylamine (2.0 g 20.0 mmol). The reaction mixture was stirred at 50 °C for 3 hours, and then concentrated under reduced pressure. The residue was partitioned between H₂O and CH₂Cl₂. The organic layer was dried over MgSO₄ and filtered and the filtrate was concentrated under reduced pressure to give compound **36** (2.3 g, 82%).

Step Two: To a solution of compound **37** (3.50 g, 26.5 mmol) in ethanol (10 mL) and pyridine (5 mL) was added isovaleraldehyde (2.8 mL 27 mmol) and piperidine (1 mL). The reaction mixture was heated to reflux for 3 hours and concentrated under reduced pressure. The residue was partitioned between 2N HCl (15 mL) and ethyl acetate (30 mL). The organic layer was dried over MgSO₄, and filtered and the filtrate was concentrated under reduced pressure. The residue was purified by silica gel chromatography, eluting with 2:1 hexanes:ethyl acetate to give compound **38** (3.6 g, 67%).

Step Three: A solution of compound **38** (2.5 g, 12.48 mmol) and compound 36 (2.52 g, 13.7 mmol) in dry methanol (25 mL) was heated to vigorous reflux for 3 hours, cooled and concentrated under reduced pressure. The residue was chromatographed on silica gel eluting with 2:1 hexanes:ethylacetate to give compound **39** (2.75 g, 69%).

Step Four: To a solution of compound **39** (2.5 g, 7.9 mmol) in CCl₄ (15 mL) was added NBS (1.4 g, 8.0 mmoL), K₂CO₃ (11.0 g, 80.0 mmol), and benzoyl peroxide (50 mg, 0.20 mmol). The reaction mixture was heated to reflux for 1 hour, cooled to room temperature, diluted with H₂O and extracted with CH₂Cl₂. The organic layer was dried over MgSO₄ and filtered and the filtrate was concentrated under reduced pressure. The residue was chromatographed on silica gel eluting with 3:1 hexanes:ethyl acetate to give compound **40** (0.62 g, 25%).

Step Five: Compound **40** (0.60 g, 1.9 mmol) was treated with 2N NaOH (5mL) and THF (3 mL). The resulting mixture was stirred at room temperature for 2 hours, acidified with 2N HCl and extracted with ethyl acetate. The organic layer was dried over MgSO₄ and filtered and the filtrate was concentrated under reduced pressure to give compound **41** (560 mg, 98%).

Step Six: To a solution of compound **41** (0.56 g, 1.86 mmol) in dry benzene (10 mL), diphenylphosphorylazide (0.56 g, 2.0 mmol) and triethylamine (2.02 g, 2.0 mmol) were added. The reaction mixture was heated to 90 °C for 1 hour then a solution of compound 8 (0.39 g, 1.9 mmol) in benzene (2 mL) was added. The reaction was stirred at 90 °C for an additional 1 hour, cooled to room temperature, diluted with 10% aqueous ammonium chloride and extracted with ethyl acetate. The organic layer was dried over MgSO₄ and filtered and the filtrate was concentrated under reduced pressure. The residue was chromatographed on silica gel, eluting with 7:3 ethyl acetate:hexane to give compound **42** (0.38 g, 40%).

Step Seven: To a solution of compound **42** (0.35 g 0.7 mmol) in 1:1 mixture of THF:MeOH (8 mL) was added 2N NaOH (8 mL). The reaction was stirred at room temperature for 3 hours, acidified with 2N HCl (10 mL) and extracted with ethyl acetate (20 mL). The organic layer was dried over MgSO₄ and filtered and the filtrate was concentrated under reduced pressure to give (3S)-3-[({[2-methyl-4-(2-methylpropyl)-6-oxo-1-(phenylmethyl)-1,6-dihydro-5-pyrimidinyl]amino}carbonyl)amino]-3-(4-methylphenyl)propanoic acid (**43**, 250 mg, 75%). MS: Calculated: (M+H)⁺ = 477.25 m/z; Found: (M+H)⁺ = 477.17 m/z.

### Example 11

### Synthesis of (3S)-3-[({[2-methyl-6-oxo-1-(phenylmethyl)-1,6-dihydro-5-pyrimidinyl]amino}carbonyl)amino]-3-(4-methylphenyl)propanoic acid

Step One: A solution of compound **36** (2.3 g, 15.5 mmol) and compound 44 (3.36 g, 15.5 mmol) in absolute ethanol (35 mL) was refluxed for 3 hours and concentrated. The residue was chromatographed on silica gel, eluting with 1:1 ethyl acetate:hexane to give compound **45** (1.87 g, 55% yield).

(3S)-3-[({[2-Methyl-6-oxo-1-(phenylmethyl)-1,6-dihydro-5-pyrimidinyl]amino}carbonyl)amino]-3-(4-methylphenyl)propanoic acid was prepared from compound 45 according to procedures described in Example 10. ¹H NMR (400 MHz, CD₃OD) 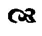 2.28 (s, 3H), 2.35 (s, 3H), 2.57 (m, 2H), 5.16 (m, 1H), 5.30 (s, 2H), 7.13 (m, 4H), 7.30 (m, 5H), 8.50 (s, 1H).'

### Example 12

### Synthesis of (3S)-3-{[({1-[(2-chlorophenyl)methyl]-4-[({ethyl[(ethylamino) carbonyl]amino}carbonyl)amino]-2-oxo-1,2-dihydro-3-pyridinyl}amino)carbonyl]amino}-3-(4-methylphenyl)propanoic acid.

Step One: To a solution of compound **46** (prepared according to procedures described in Example 3, 0.50 g, 1.8 mmol) in THF (10 mL) at 0 °C was added NaH (60% dispersion in mineral oil, 0.23 g, 5.1 mmol). The mixture was stirred for 10 minutes at 0 °C, then ethyl isocyanate (0.65 g, 9.15 mmol) was added. The mixture was stirred at room temperature over the weekend, was quenched with 1 N HCl and extracted with ethyl acetate. The organic layer was dried over MgSO₄ and filtered and the filtrate was concentrated under reduced pressure to give compound **47** (0.60 g). This material was used without purification.

(3S)-3-{[({1-[(2-Chlorophenyl)methyl]-4-[({ethyl[(ethylamino)carbonyl] amino }carbonyl)amino]-2-oxo-1,2-dihydro-3-pyridinyl}amino)carbonyl]amino}-3-(4-methylphenyl)propanoic acid was prepared from compound **47** according to procedures described in Example 3. Melting point: 128-130 °C.

### Example 13

### Synthesis of (3S)-3-{[({1-[(2-chlorophenyl)methyl]-4-hydroxy-2-oxo-1,2-dihydro-3-quinolinyl}amino)carbonyl]amino}-3-(4-methylphenyl)propanoic acid.

Step One: To a solution of compound **48** (2.00 g, 9.70 mmol) in anhydrous DMF (25 mL) at 0°C was added NaH (60% dispersion in mineral oil, 0.89 g, 22 mmol). The mixture was stirred at 0 °C for 15 minutes before the addition of 2-chlorobenzylchloride (2.03 g, 12.6 mmol). After stirring at 55 °C overnight, the mixture was poured into ice-water and washed with Et₂O (twice). The aqueous layer was acidified and filtration of the resulting precipitate gave compound **49** (3.45 g). This material was used without purification.

(3S)-3-{[({1-[(2-chlorophenyl)methyl]-4-hydroxy-2-oxo-1,2-dihydro-3-quinolinyl}amino)carbonyl]amino}-3-(4-methylphenyl)propanoic acid was prepared from compound 49 according to procedures described in Example 8. Melting point: 134-136 °C.

### Example 14

### Synthesis of (3S)-3-{[({1-[(2-chlorophenyl)methyl]-5-methyl-2-oxo-1,2-dihydro-3-pyridinyl}amino)carbonyl]amino}-3-(4-methylphenyl)propanoic acid (56).

Step One: To a suspension of compound **51** (1.67 g, 9.81 mmol) in DMF (33 mL) at room temperature under a dry, nitrogen atmosphere, 2-chlorobenzylamine (1.30 mL, 10.8 mmol) and EDCI (2.35 g, 12.3 mmol) were added sequentially. The resulting mixture was vigorously stirred at room temperature for 5 hours, diluted with ethyl acetate and washed with 2 N HCl, H₂O (3 times), saturated aqueous NaHCO₃ and brine. The organic layer was dried over MgSO₄ and filtered and the filtrate was concentrated under reduced pressure to give compound 52 (2.55 g, 100%) as a pale yellow solid.

Step Two: A solution of compound **52** (555 mg, 2.17 mmol) and 3-dimethylamino-2-methylpropenal (738 mg, 6.5 mmol) in absolute ethanol (4.3 mL) and glacial acetic acid (0.22 mL) was heated to reflux overnight. The resulting mixture was cooled to room temperature, diluted with ethyl acetate and washed with 2 N HCl (twice), H₂O and brine. The organic layer was dried over MgSO₄ and filtered and the filtrate was concentrated under reduced pressure. The pressure was purified by chromatography on silica gel, eluting with 7:3 increasing to 1:1 hexanes:ethyl acetate and finally 19:19:2 hexanes:ethyl acetate:methanol to yield compound **53** (182 mg, 27%) as a yellow oil.

Step Three: To a solution of compound **53** (167 mg, 0.55 mmol) in THF (3 mL), 2 N NaOH (1 mL) and methanol (2 mL) were added. The resulting mixture was stirred for 15 minutes, diluted with H₂O and extracted with ethyl ether. The aqueous layer was acidified with 2 N HCl and extracted with ethyl acetate. The ethyl acetate layer was washed with H₂O and brine, dried over MgSO₄ and filtered. The filtrate was concentrated under reduced pressure to give compound **54** (139 mg, 91%) as a white solid.

Step Four: To a suspension of compound **54** (175 mg, 0.63 mmol) in THF (6.7 mL) and DIPEA (0.23 mL, 1.34 mmol) at room temperature under a dry, nitrogen atmosphere, DPPA (0.29 mL, 1.34 mmol) was added *via* syringe. The resulting mixture was stirred at room temperature for 15 minutes, then heated to reflux for 3.5 hours. The mixture was allowed to cool to room temperature and a solution of compound 8 (278 mg, 1.34 mmol) in THF (6.0 mL) was added *via* cannula along with a THF (0.7 mL) rinse. The resulting mixture was stirred at room temperature overnight, diluted with ethyl acetate and washed with 2 N HCl (twice), saturated aqueous NaHCO₃ and brine. The organic layer was dried over MgSO₄ and filtered and the filtrate was concentrated under reduced pressure. The residue was purified by silica gel chromatography, eluting with 7:3 then 3:2 and finally 1:1 hexanes:ethyl acetate to yield compound **55** (60 mg, 20%) as a colorless oil.

Step Five: To a solution of compound **55** (60 mg, 0.12 mmol) in THF (3 mL), 0.192 N NaOH (0.65 mL, 0.12 mmol) and methanol (2 mL) were added. The resulting mixture was stirred at room temperature for 24 hours, then was diluted with H₂O. The organic solvents were removed under reduced pressure and the resulting aqueous mixture was extracted with ethyl ether. The aqueous phase was lyophilized to give (3S)-3-{[({1-[(2-chlorophenyl)methyl]-5-methyl-2-oxo-1,2-dihydro-3-pyridinyl}amino)carbonyl]amino}-3-(4-methylphenyl)propanoic acid, sodium salt **(56,** 56 mg, 95%) as an off-white solid. MS: Calculated for (C₂₄H₂₃ClN₃O₄): 452.14 m/z; Found: 451.99 m/z.

### Example 15

### Synthesis of (3S)-3-(1,3-benzodioxol-5-yl)-3-[({[2-oxo-1-(2-thienylmethyl)-1,2-dihydro-3-pyridinyl]amino}carbonyl)amino]propanoic acid (62).

Step One: To a solution of 2-thiophenemethanol (1.015 g, 8.89 mmol) in CH₂Cl₂ (17.8 ml) cooled to °C under a dry nitrogen atmosphere, triethylamine (2.98 ml, 21.4 mmol) and methanesulfonyl chloride (0.69 ml, 8.9 mmol) were added sequentially by syringe. The resulting mixture was stirred at 0°C for 15 minutes, then 2-hydroxy-3-nitropyridine (1.496 g, 10.7 mmol) and 4-dimethylaminopyridine (catalytic) were added. The mixture was allowed to gradually warm to room temperature and then was stirred overnight. The mixture was diluted with ethyl acetate and washed with 2N HCl, H₂O, saturated NaHCO₃ and brine. The organic phase was dried over MgSO₄ and filtered and the filtrate was concentrated under reduced pressure to give **58** (395 mg) as a yellow waxy solid. This material was used without purification.

Step Two: To a solution of **58** (330 mg, 1.40 mmol) in glacial acetic acid (6.6 ml) at room temperature under a dry nitrogen atmosphere, iron powder (154 mg, 2.8 mmol, -325 mesh) was added. The resulting solution was heated to 60°C in an oil bath with vigorous stirring for 20 minutes. The mixture was cooled to room temperature, diluted with ethyl acetate and filtered through Celite. The filtrate was washed with H₂O, saturated NaHCO₃ and brine. The organic phase was dried over MgSO₄ and filtered and the filtrate was concentrated under reduced pressure. The residue was filtered through silica gel, eluting with 1:1 hexanes:ethyl acetate increasing to 1:3 hexanes:ethyl acetate to yield 59 (188 mg, 12% for two steps) as a greenish solid.

Step Three: To a solution of **59** (111 mg, 0.54 mmol) in CH₂Cl₂ (2.7 ml) cooled to 0°C under a dry nitrogen atmosphere, N,N-diisopropylethylamine (0.23 ml, 1.30 mmol) and phosgene (0.31 ml, 1.9M in toluene, 0.59 mmol) were added sequentially by syringe. The resulting mixture was stirred at 0°C for 15 minutes, then a solution of β-amino ester **60** (167 mg, 0.70 mmol) in CH₂Cl₂ (2.7 ml) was added by cannula along with a CH₂Cl₂ rinse (1.0 ml). The resulting mixture was allowed to warm to room temperature, was stirred for 2 hours, was diluted with ethyl acetate and washed with 2N HCl, H₂O, saturated NaHCO₃ and brine. The organic phase was dried over MgSO₄ and filtered and the filtrate was concentrated under reduced pressure. The residue was purified by silica gel chromatography, eluting with 1:1 hexanes:ethyl acetate to yield **61** (231 mg, 91%) as a purple foam.

Step Four: To a solution of ester **61** (227 mg, 0.48 mmol) in THF (6 ml) at room temperature, NaOH (2 ml, 2N in H₂O, 4 mmol) and methanol (enough to give a clear solution, approximately 2 ml) were added. The resulting mixture was stirred for 15 minutes, then was diluted with water and extracted with ether. The aqueous phase was acidified with HCl (2N) and extracted with ethyl acetate. The organic phase was washed with brine, dried over MgSO₄ and filtered and the filtrate was concentrated under reduced pressure to give **62** (191 mg, 90%) as a white solid. ¹H NMR (400 MHz, CD₃SOCD₃) δ 2.63 (d, J = 7.3 Hz, 2H), 4.99 (dt, J = 8.4, 7.3 Hz, 1H), 5.30 (s, 2H), 5.98 (m, 2H), 6.21 (dd, J = 7.5, 7.0 Hz, 1H), 6.78 (dd, J = 8.1, 1.6 Hz, 1H), 6.85 (d, J = 8.1 Hz, 1H), 6.88 (d, J = 1.6 Hz, 1H), 6.97 (dd, J = 5.1, 3.5 Hz, 1H), 7.17 (dd, J = 3.5, 1.1 Hz, 1H), 7.35 (dd, J = 7.0, 1.8 Hz, 1H), 7.44 (dd, J = 5.1, 1.1 Hz, 1H), 7.67 (d, J = 8.4 Hz, 1H), 7.94 (dd, J = 7.5, 1.8 Hz, 1H), 8.40 (s, 1H).

### Example 16

### Synthesis of (3S)-3-(1,3-benzodioxol-5-yl)-3-[({[(3S)-2-oxo-1-(2-thienylmethyl)hexahydro-3-pyridinyl]amino} carbonyl)amino]propanoic acid (68).

Step One: To a solution of N-α-*tert*-butoxycarbonyl-N-δ-benzyloxycarbonyl-L-ornithine **63** (1.00 g, 2.73 mmol) and cesium carbonate (1.33 g, 4.1 mmol) in DMF (10 ml) at room temperature under a dry nitrogen atmosphere, iodomethane (0.22 ml, 3.3 mmol) was added by syringe. The resulting mixture was stirred at room temperature for 18 hours then was diluted with ethyl acetate and washed with H₂O, 10% Na₂S₂O₅, saturated NaHCO₃ and brine. The organic phase was dried over MgSO₄ and filtered and the filtrate was concentrated under reduced pressure to give ester **64** (1.21g) as a pale yellow oil. This material contained DMF but was used without purification.

Step Two: To a solution of 64 (0.86 g of crude material prepared in previous procedure, 1.94 mmol theoretical) in methanol (10 ml) at 0°C under a dry nitrogen atmosphere, palladium on charcoal (300 mg, 10% Pd, Degussa type E101 NE/W, wet, 50% water by weight) was added. The nitrogen atmosphere was replaced by hydrogen (alternate five times between vacuum and hydrogen supplied by balloon) and the mixture was stirred at 0°C for 30 minutes then filtered directly into a flask containing 2-thiophenecarboxaldehyde (177 mg, 1.58 mmol). The mixture was concentrated (water bath at room temperature) and the residue was taken up in dichloroethane (6 ml). To this solution, sodium triacetoxyborohydride (479 mg, 2.26 mmol) was added and the mixture was stirred for 2 hours, diluted with ethyl acetate and washed with saturated NaHCO₃ (2 times) and brine. The organic phase was dried over MgSO₄ and filtered and the filtrate was concentrated under reduced pressure. The residue was filtered through silica gel, eluting with 7:3 hexanes:ethyl acetate to yield lactam 65 (75 mg, 12% for two steps) as a colorless oil.

Step Three: To a flask containing **65** (89 mg, 0.29 mmol) sealed with a rubber septum at room temperature under a dry nitrogen atmosphere, HCl (7.2 ml, 4.0M in dioxane, 28.8 mmol) was added by syringe. The nitrogen needle was removed and the mixture in the sealed flask was stirred overnight. The mixture was diluted with CH₂Cl₂ and washed with saturated NaHCO₃. The organic phase was dried over MgSO₄ and filtered and the filtrate was concentrated under reduced pressure to give amine 66 (60 mg, 100%) as a light yellow oil. This material was used without purification.

Step Four: To a solution of β-amino ester **60** (75 mg, 0.32 mmol) in CH₂Cl₂ (0.6 ml) at room temperature under a dry nitrogen atmosphere, carbonyldiimidazole (51 mg, 0.32 mmol) was added. The resulting mixture was stirred at room temperature for 5 minutes and a solution of amine **66** (60 mg, 0.29 mmol) in CH₂Cl₂ (0.6 ml) was added by cannula along with a CH₂Cl₂ (0.2 ml) rinse. The resulting mixture was stirred at room temperature for 3 days, then was diluted with ethyl acetate and washed with 2N HCl (2 times), H₂O, saturated NaHCO₃ and brine. The organic phase was dried over MgSO₄ and filtered and the filtrate was concentrated under reduced pressure. The residue was filtered through silica gel, eluting with 1:1 hexanes:ethyl acetate increasing to 2:3 hexanes:ethyl acetate to yield urea **67** (110 mg, 80%).

Step Five: To a solution of urea **67** (108 mg, 0.23 mmol) in THF (3 ml) at room temperature, NaOH (1 ml, 2N in H₂O, 2 mmol) and methanol (enough to give a clear solution, approximately 2 ml) were added. The resulting mixture was stirred for 15 minutes, then was diluted with water and extracted with ether. The aqueous phase was acidified with HCl (2N) and extracted with ethyl acetate. The ethyl acetate layer was washed with brine, dried over MgSO₄ and filtered and the filtrate was concentrated under reduced pressure to give **68** (92 mg, 90%) as a white foam. ¹H NMR (400 MHz, CD₃SOCD₃) δ 1.45 (m, 1H), 1.76 (m, 2H), 2.62 (m, 2H), 3.25 (m overlapping H₂O, 2H), 4.01 (m, 1H), 4.59 (d, J = 15.0 Hz, 1H), 4.68 (d, J =15.0 Hz, 1H), 4.96 (m, 1H), 5.97 (s, 2H), 6.24 (d, J = 6.6 Hz, 1H), 6.71 (d, J = 8.4 Hz, 1H), 6.75 (dd, J = 8.1, 1.5 Hz, 1H), 6.82 (d, J = 8.1 Hz, 1H), 6.85 (d, J = 1.5 Hz, 1H), 6.97 (dd, J = 5.1, 3.3 Hz, 1H), 7.03 (dd, J = 3.3, 1.5 Hz, 1H), 7.42 (dd, J = 5.1, 1.5 Hz, 1H), 12.06 (br. s, 1H).

### Example 17

### Synthesis of (3S)-3-(1,3-benzodioxol-5-yl)-3-[({[(3S)-2-oxo-1-(2-thienylmethyl)tetrahydro-1H-pyrrol-3-yl]amino}carbonyl)amino]propanoic acid (74).

Step One : To a solution of N-*tert*-butoxycarbonyl-L-aspartic acid α-benzylester (2.10 g, 6.5 mmol) in dimethoxyethane (15 ml) cooled to -15°C (bath temperature) under a dry nitrogen atmosphere, 4-methylmorpholine (0.71 ml, 6.5 mmol) and isobutyl chloroformate (0.84 ml, 6.5 mmol) were added sequentially by syringe. The resulting mixture was stirred for 2 minutes, then was filtered, washing the solid cake with dimethoxyethane (10 ml). The filtrate was recooled to -15°C (bath temperature) and a solution of sodium borohydride (370 mg, 9.7 mmol) in H₂O (3 ml) was added followed immediately by the addition of H₂O (100 ml). The mixture was extracted with ethyl acetate (3 times) and the organic layers were combined and washed with cold (0°C) HCl (0.2N), H₂O, saturated NaHCO₃ and brine. The resulting organic layer was dried over MgSO₄ and filtered and the filtrate was concentrated under reduced pressure to give 69 (2.50 g) as a colorless oil. This material contains some of the unreduced mixed-anhydride but was used without purification.

Step Two: To a solution of oxalyl chloride (2.4 ml, 2.0 M in CH₂Cl₂, 4.8 mmol) in CH₂Cl₂ (30 ml) cooled to -65°C under a dry nitrogen atmosphere, a solution of methylsulfoxide (0.55 ml, 7.8 mmol) in CH₂Cl₂ (8 ml) was added by syringe. The resulting mixture was stirred at -65°C for 15 minutes, then a solution of alcohol 69 (1.00 g, 3.2 mmol) in CH₂Cl₂ (29 ml) was added by cannula along with a CH₂Cl₂ (3 ml) rinse. The mixture was stirred at -65°C for 3 hours, then was allowed to warm to -20°C (bath temperature). Triethylamine (0.96 ml, 6.9 mmol) was added, followed by H₂O (20 ml). The aqueous layer was extracted with CH₂Cl₂ and the combined organic phases were dried over MgSO₄ and filtered. The filtrate was concentrated under reduced pressure to give aldehyde 70 as a white solid. This material was used immediately without purification.

Step Three: To a solution of the crude aldehyde **70** (3.2 mmol theoretical) and 2-aminomethylthiophene (402 mg, 3.55 mmol) in dichloroethane (13 ml) at room temperature under a dry nitrogen atmosphere, sodium triacetoxyborohydride (959 mg, 4.5 mmol) was added. The resulting mixture was stirred at room temperature overnight, then was diluted with ethyl acetate and washed with saturated NaHCO₃ and brine. The organic phase was dried over MgSO₄ and filtered and the filtrate was concentrated under reduced pressure. The residue was purified by silica gel chromatography, eluting with 1:1 hexanes:ethyl acetate to yield lactam **71** (220 mg, 23% for 3 steps) as a white solid.

Step Four: To a solution **of 71** (220 mg, 0.74 mmol) in dioxane (1.5 ml) sealed with a rubber septum at room temperature under a dry nitrogen atmosphere, HCl (1.50 ml, 4.0M in dioxane, 6.0 mmol) was added by syringe. The nitrogen needle was removed and the mixture in the sealed flask was stirred for 5 hours. The mixture was diluted with CH₂Cl₂ and washed with saturated NaHCO₃. The organic phase was dried over MgSO₄ and filtered and the filtrate was concentrated under reduced pressure to give amine **72** (129 mg, 89%) as a light yellow oil. This material was used without purification.

Step Five: To a solution of amine **72** (123 mg, 0.63 mmol) in CH₂Cl₂ (1.5 ml) at room temperature under a dry nitrogen atmosphere, carbonyldiimidazole (112 mg, 0.69 mmol) was added. The resulting mixture was stirred at room temperature for 5 minutes and a solution of β-amino ester **60** (164 mg, 0.69 mmol) in CH₂Cl₂ (0.8 ml) was added by cannula along with a CH₂Cl₂ (0.2 ml) rinse. The resulting mixture was stirred at room temperature overnight, then was diluted with ethyl acetate and washed with 2N HCl (2 times), H₂O, saturated NaHCO₃ and brine. The organic phase was dried over MgSO₄ and filtered and the filtrate was concentrated under reduced pressure. The residue was filtered through silica gel, eluting with 49:1 chloroform:methanol to yield urea **73** (230 mg, 80%) as a colorless oil which slowly solidified on standing.

Step Six: To a solution of urea **73** (230 mg, 0.50 mmol) in THF (3 ml) at room temperature, NaOH (1 ml, 2N in H₂O, 2 mmol) and methanol (1 ml) were added. The resulting mixture was stirred for 1 hour, then was diluted with water and extracted with ether. The aqueous phase was acidified with HCl (2N) and extracted with ethyl acetate. The ethyl acetate layer was washed with brine, dried over MgSO₄ and filtered and the filtrate was concentrated under reduced pressure to give **74** (181 mg, 84%) as a white foam. ¹H NMR (400 MHz, CD₃SOCD₃) δ 1.64 (m, 1H), 2.30 (m, 1H), 2.64 (m, 2H), 3.20 (m, 2H), 4.17 (dd, J = 8.8, 8.4 Hz, 1H), 4.56 (s, 2H), 4.96 (m, 1H), 5.97 (s, 2H), 6.30 (d, J = 7.0 Hz, 1H), 6.58 (d, J = 8.8 Hz, 1H), 6.77 (m, 1H), 6.80-6.90 (m, 2H), 6.96-7.04 (m, 2H), 7.45 (dd, J = 5.1, 0.7 Hz, 1H), 12.10 (br. s, 1H).

### Example 18

### Synthesis of (3S)-3-[({[5-chloro-2-hydroxy-3-(phenylmethyl)phenyl]amino}carbonyl)amino]-3-(4-methylphenyl)propanoic acid.

Step One: To a mixture of 2-phenylmethyl-3-chlorophenol (5.00 g, 22.9 mmol) in Et₂O (20 mL) and 6N HCl (50 mL), KNO₃ (2.30 g, 22.9 mmol) and NaNO₂ (20 mg, catalytic) were added sequentially. The resulting mixture was stirred for 2 hours, diluted with water and extracted with ethyl acetate. The organic layer was washed with water and brine, dried over MgSO₄ and filtered. The filtrate was concentrated under reduced pressure to give **99** (6.0 g, 100%).

Step Two: To a solution of **99** (6.0 g, 22.8 mmol) in methanol (360 mL), zinc powder (6.0 g, 92 mmol) and saturated aqueous NH₄Cl (6 mL) were added. The resulting heterogeneous mixture was refluxed overnight. After filtration of the hot mixture and concentration of the filtrate under reduced pressure, the residue was dissolved in ethyl acetate and washed with saturated aqueous NaHCO₃ and brine. The organic layer was dried over MgSO₄ and filtered and the filtrate was concentrated under reduced pressure to give compound **100** (2.93 g, 55%).

Step Three: To a solution of **25** (0.20 g, 0.96 mmol) in CH₂Cl₂ at 0 °C, DIPEA (0.40 mL, 2.4 mmol) and phosgene (1.93 M in toluene, 0.60 mL, 1.2 mmol) were added sequentially. The resulting mixture was allowed to warm to room temperature, stirred for 20 minutes, then recooled to 0 °C. To this mixture, a solution of **100** (0.25 g, 1.1 mmol) in CH₂Cl₂ was added dropwise. The resulting mixture was allowed to warm to room temperature overnight, was diluted with water and was extracted with CH₂Cl₂. The organic layer was washed with water and brine, dried over MgSO₄ and filtered. The filtrate was concentrated under reduced pressure and the residue was purified by silica gel chromatography, eluting with 9:1 and increasing to 5:1 hexanes:ethyl acetate to give **101** (60 mg, 12%).

(3S)-3-[({[5-Chloro-2-hydroxy-3-(phenylmethyl)phenyl]amino}carbonyl)amino]-3-(4-methylphenyl)propanoic acid was prepared from **101** by procedures described in Example 1. ¹H NMR (400 MHz, CD₃SO₂CD₃) 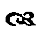 2.26 (s, 3H), 2.58 (dd, J = 15.8, 6.6 Hz, 1H), 2.67 (dd, J = 15.8, 8.4 Hz, 1H), 3.49 (s, 2H), 4.88 (m, 1H), 7.00-7.70 (m, 13H), 11.95 (br. s, 1H).

### Example 19

### Synthesis of (3S)-3-(1,3-benzodioxol-5-yl)-3-[({butyl[2,5-dioxo-1-(phenylmethyl)tetrahydro-1H-pyrrol-3-yl]amino}carbonyl)amino]propanoic acid.

Step One: A solution of N-benzylmaleimide (2.60 g, 13.9 mmol) and n-butylamine (1.00 g, 13.7 mmol) in THF (15 mL) was stirred at room temperature overnight and concentrated under reduced pressure. The residue was purified by silica gel chromatography, eluting with 4:1 increasing to 2:1 hexanes:ethyl acetate to give **102** (3.25 g, 90%).

(3S)-3-(1,3-Benzodioxol-5-yl)-3-[({butyl[2,5-dioxo-1-(phenylmethyl)tetrahydro-1H-pyrrol-3-yl]amino}carbonyl)amino]propanoic acid was prepared from **102** according to procedures described in Example 1. MP: 80-85 °C.

### Example 20

### Synthesis of (3S)-3-(1,3-benzodioxol-5-yl)-3-[({[1-(cyaopentylmethyl)-2-oxo-1,2-dihydro-3-pyridinyl]amino}carbonyl)amino]propanoic acid.

Step One: To a solution of 2-hydroxy-3-nitropyridine (200 mg, 1.4 mmol) in CH₂Cl₂ (14 mL) at 0 °C under a nitrogen atmosphere, cyclopentanemethanol (178 mg, 1.78 mmol) was added followed by triphenylphosphine (551 mg, 2.1 mmol). The solution was stirred at 0 °C for 15 minutes and diethyl azodicarboxylate (366 mg, 2.1 mmol) was added dropwise *via* syringe. The reaction was allowed to stir at 0 °C for one hour and then at room temperature overnight. The mixture was quenched with methanol (20 mL) and washed with water (twice). The aqueous layer was extracted with dichloromethane and the combined organic layers were dried over magnesium sulfate and filtered. The filtrate was concentrated and the residue was purified by silica gel chromatography, eluting with 1:1 hexanes:ethyl acetate to afford **103** (299 mg, 96% yield) as a yellow solid.

(3S)-3-(1,3-Benzodioxol-5-yl)-3-[({[1-(cyclopentylmethyl)-2-oxo-1,2-dihydro-3-pyridinyl]amino}carbonyl)amino]propanoic acid was prepared from 103 according to procedures described in Example 1. ¹H NMR (400 MHz, CDCl₃): 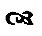 1.2-1.7 (m, 8H), 2.34 (m, 1H), 2.81 (dd, J = , 1H), 2.95 (dd, J = , 1H), 3.92 (d, J = 7.7 Hz, 2H), 5.30 (m, 1H), 5.92 (m, 2H), 6.30 (t, J = 7.1 Hz, 1H), 6.68-7.00 (m, 5H), 8.33 (d, J = 7.7 Hz, 1H), 8.89 (s, 1H).

### Example 21

### Synthesis of (3S)-3-(1,3-benzodioxol-5-yl)-3-{[({3-[(2-thiophenylmethyl)amino] phenyl} amino)carbonyl]amino} propanoic acid.

Step One: To a solution of 2-thiophenecarboxaldehyde (0.48 g, 4.0 mmol) in dichloromethane was added 3-nitroaniline (0.51 g, 3.7 mmol). The solution was concentrated to dryness and brought up in 1,2-dichloroethane (16 mL). Molecular sieves (3Å, 1.1 g) were added followed by NaBH(OAC)₃ (1.01 g, 4.8 mmol). The solution was stirred overnight at room temperature, diluted with chloroform and washed with water. The organic layer was dried over MgSO₄ and filtered and the filtrate was concentrated under reduced pressure to give **104** (0.72 g, 84%).

Step Two: To a solution of **104** (0.30 g, 1.3 mmol) in CH₂Cl₂ (5.2 mL) and triethylamine (0.215 mL, 1.5 mmol) at 0 °C was added trifluoroacetic anhydride (0.193 mL, 1.4 mmol). The solution was stirred 15 minutes at 0 °C, the ice bath was removed and the mixture was stirred for an additional 15 minutes. The mixture was diluted with CH₂Cl₂, washed with 2N HCl, water and brine. The organic layer was dried over Na₂SO₄ and filtered and the filtrate was concentrated under reduced pressure to give **105** (0.38 g, 100 %) as a yellow solid.

Step Three: To a solution of **105** (0.38 g, 1.4 mmol) in ethanol (2.6 mL) and acetic acid (2.6 mL) at room temperature, Fe powder (0.36 g, 6.5 mmol) was added and the suspension was stirred vigorously at 40 °C until TLC indicated complete consumption of **105.** The mixture was filtered through Celite, washing with chloroform. The filtrate was diltuted with saturated sodium bicarbonate and the chloroform layer was dried over Na₂SO₄ and filtered. The filtrate was concentrated under reduced pressure and the residue was purified by chromatography on silica gel (gradient elution 6:1 to 4:1 hexanes:ethyl acetate) to give compound **106** (0.102 g, 25%)

(3S)-3-(1,3-Benzodioxol-5-yl)-3-{[({3-[(2-thiophenylmethyl)amino]phenyl} amino)carbonyl]amino}propanoic acid was prepared from **106** according to procedures described in Example 1. ¹H NMR (400 MHz, CD₃SO₂CD₃) 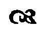 2.50 (m, 2H overlapping DMSO), 4.37 (d, J = 5.9 Hz, 2H), 4.94 (m, 1H), 5.94 (m, 2H), 6.06 (t, J = 5.8 Hz, 1H), 6.16 (m, 1H), 6.59 (d, J = 8.8 Hz, 1H), 6.78 (m, 3H), 6.85 (dd, J = 8.8, 7.7 Hz, 1H), 6.90 (s, 1H), 6.94 (dd, J = 5.2, 3.7 Hz, 1H), 7.00 (d, J = 3.3 Hz, 1H), 7.33 (dd, J = 5.1, 1.1 Hz, 1H), 8.5 (s, 1H).

### Example 22

### Synthesis of 3-(1,3-benzodioxol-5-yl)-2,2-difluoro-3-[({[2-oxo-1-(2-thiophenylmethyl) 1,2-dihydro-3-pyridinyl]amino} carbonyl)amino]propanoic acid.

Step One: To a solution of (1S,2R,5S)-(+)-menthyl (R)-p-toluenesulfinate (3.00 g, 10.2 mmol) in THF (25.5 mL) chilled to -78 °C, lithium bis(trimethylsilyl)amide (1.0 M in THF, 15.3 mL) was added dropwise over 15 minutes. The resulting mixture was stirred at room temperature for 6 hours, then chilled to 0 °C. Piperonal (3.06 g, 20.4 mmol) and CsF (3.10 g, 20.4 mmol) were added rapidly and the suspension stirred 36 hours at room temperature. The reaction was quenched with saturated NH₄Cl and extracted with ethyl acetate. The organic layer was washed with brine, dried over Na₂SO₄ and filtered and the filtrate was concentrated under reduced pressure. The residue was recrystallized from hexanes and dichloromethane to give compound **108** (1.36 g, 46 %)

Step Two: Ethyl bromodifluoroacetate (0.78 mL, 6.1 mmol) was added to a suspension of Zn dust (2.00 g, 30.5 mmol) in THF (20.2 mL) and refluxed for 15 minutes. The suspension was chilled to 0 ° C and **108** (0.87 g, 3.0 mmol) was added. The suspension was allowed to warm to room temperature and stirred overnight. The mixture was quenched with a minimum amount of saturated NH₄Cl and extracted with ethyl acetate. The organic layer was washed with saturated aqueous NaHCO₃ and brine, dried over Na₂SO₄ and filtered. The filtrate was concentrated under reduced pressure and the residue was purified by chromatography on silica gel (gradient elution 6:1 to 4:1 hexanes:ethyl acetate to give **109** (0.607 g, 61 % at 80% conversion).

Step Three: To a solution of **109** (0.700 g, 1.70 mmol) in methanol (4.3 mL) at 0 °C, trifluoroacetic acid (0.26 mL 3.4 mmol) was added. The solution was stirred at 0 °C for 2 hours, then concentrated to dryness under reduced pressure, while maintaining the external temperature below 30 °C. The residue was taken up in diethyl ether and washed with 2N HCl (2 times). The combined aqueous layers were carefully basified with excess saturated NaHCO₃ and extracted with diethyl ether. The ether layer was dried over MgSO₄ and filtered and the filtrate was concentrated under reduced pressure to give **110** (0.326 g, 80 %).

3-(1,3-Benzodioxol-5-yl)-2,2-difluoro-3-[({[2-oxo-1-(2-thiophenylmethyl)-1,2-dihydro-3 -pyridinyl] amino}carbonyl)amino]propanoic acid was prepared from **110** according to procedures described in Example 1. MS: Calculated (M-H)⁻ = 476.07; Found (M-H)⁻ = 476.00.

### Example 23

### Synthesis of (3S)-3-(1,3-benzodioxol-5-yl)-3-({[9-oxo-8-(phenylmethyl)-2,3,4,5,8,9-hexahydro-1H-pyrido[3,4-b]azepin-1-yl]carbonyl}amino)propanoic acid.

Step One: To a solution of 3 (0.74 g, 3.6 mmol) in THF (14.4 mL) and TMEDA (1.60 mL, 10.8 mmol) at -20 °C, n-butyllithium (1.6 M in hexanes, 3.4 mL, 5.4 mmol) and tert-butyllithium (1.7M in pentane, 2.5 mL, 4.3 mmol) were sequentially added dropwise by syringe. The temperature was allowed to warm to between -10 and 0 °C and maintained there for 2 hours. To the resulting mixture, 1,4-dibromobutane (1.75 mL, 14.7 mmol) was added rapidly and the solution was allowed to warm to room temperature and stirred for 4 days. The reaction was quenched with water and extracted with CHCl₃ (3 times). The combined extracts were washed with brine, dried over NaSO₄ and filtered. The filtrate was concentrated under reduced pressure and the residue was purified by chromatography on silica gel, eluting with 4:1 hexanes:ethyl acetate to give 111 (0.41g, 44%).

(3S)-3-(1,3-Benzodioxol-5-yl)-3-({[9-oxo-8-(phenylmethyl)-2,3,4,5,8,9-hexahydro-1H-pyrido[3,4-b]azepin-1-yl]carbonyl}amino)propanoic acid was prepared from **111** according to the procedures described in Example 4. MS: Calculated (M-H)⁻= 488.18; Found (M-H)⁻= 488.21.

### Example 24

### Synthesis of (3S)-3-{[({1-[(2-chlorophenyl)methyl]-2-oxo-1,2-dihydro-3-pyridinyl}amino)carbonyl]amino}-3-(4-hydroxyphenyl)propanoic acid.

Step One: To a solution of **112** (prepared according to procedures described in Example 15, 0.19 g, 0.39 mmol) in CH₂Cl₂ at 0 °C under nitrogen, BBr₃ (1.0 M in CH₂Cl₂, 1.2 mL, 1.2 mmol) was added by syringe. The mixture was allowed to gradually warm to room temperature and then stirred overnight. The mixture was diluted with water and stirred for 30 minutes and further diluted with saturated aqueous NaHCO₃. The organic layer was washed with water and the aqueous layers were combined and acidified with 2N HCl and extracted with ethyl acetate (3 times). The combined ethyl acetate layers were dried over MgSO₄ and filtered and the filtrate was concentrated under reduced pressure to yield (3S)-3-{[({1-[(2-chlorophenyl)methyl]-2-oxo-1,2-dihydro-3-pyridinyl}amino)carbonyl]amino}-3-(4-hydroxyphenyl)propanoic acid (**113**, 120 mg, 70%). ¹H NMR (400 MHz, CD₃SO₂CD₃) δ 2.95 (d, J = 5.2 Hz, 2H), 5.28 (s, 2H), 5.35 (ddd, J = 9.2, 4.8, 4.4 Hz, 1H), 6.33 (t, J = 7.1 Hz, 1H), 6.60 (d, J = 8.8 Hz, 2H), 7.04 (m, 5H), 7.22 (m, 3H), 7.37 (dd, J = 7.7, 1.5 Hz, 1H), 8.35 (dd, J = 7.6, 1.5 Hz, 1H), 8.80 (s, 1H).

Synthetic procedures similar to those described above may be utilized to obtain the compounds of Tables 1, 2 and 3.

### Example 25

A procedure in which a 26-amino acid peptide containing the CS1 sequence of fibronectin with an N-terminal Cys (CDELPQLVTLPHPNLHGPEILDVPST) was coupled to maleimide activated ovalbumin was used to determine the efficacy of the compounds synthesized. Bovine serum albumin (BSA) and CS1 conjugated ovalbumin were coated onto 96-well polystyrene plates at 0.5 ③g/ml in TBS (50 mM TRIS, pH 7.5; 150 mM NaCl) at 4°C for 16 hours. The plates were washed three times with TBS and blocked with TBS containing 3% BSA at room temperature for 4 hours. Blocked plates were washed three times in binding buffer (TBS; 1 mM MgCl₂; 1 mM CaCl₂; 1 mM MnCl₂) prior to assay. Ramos cells fluorescently labeled with calcein AM were resuspended in binding buffer (10⁷ cells/ml) and diluted 1:2 with same buffer with or without compound. 100 ③ M of compound was added. The cells were added immediately to the wells (2.5 x 10⁵ cells/well) and incubated for 30 minutes at 37°C. Following three washes with binding buffer, adherent cells were lysed and quantitated using a fluorometer. The results are shown in Tables 1-3. IC₅₀ is defined as the dose required to give 50% inhibition, measured in µM for Tables 1 and 3. The lower the IC₅₀ value and the greater the percentage of inhibition, the more efficient the compound is at prevention of cell adhesion.

**Table 1**

| Name | IC₅₀ | Mass Spectral Data (m/z) |
|---|---|---|
| (3S)-3-(1,3-benzodioxol-5-yl)-3-[({[(3S)-2-oxo-1-(2-thienylmethyl)hexahydro-3-pyridinyl]amino}carbonyl)amino]propanoic acid | 0.2 | Calc'd (M-H)⁻ =444.12; |
| | | Found (M-H)⁻= 444.08 |
| (3S)-3-(1,3-benzodioxol-5-yl)-3-[({[(3S)-2-oxo-1-(2-thienylmethyl)tetrahydro-1H-pyrrol-3-yl]amino}carbonyl)amino]propanoic acid | 15 | Calc'd (M-H)⁻ =430.11; |
| | | Found (M-H)⁻= 430.06 |
| (3S)-3-(1,3-benzodioxol-5-yl)-3-[({[(3R)-2-oxo-1-(2-thienylmethyl)hexahydro-3-pyridinyl]amino}carbonyl)amino]propanoic acid | 2 | Calc'd (M-H)⁻ =444.12; |
| | | Found (M-H)⁻= 444.05 |
| (3S)-3-(1,3-benzodioxol-5-yl)-3-[({[2-oxo-1-(2-thienylmethyl)-1,2-dihydro-3-pyridinyl]amino}carbonyl)amino]propanoic acid | 0.9 | Calc'd (M-H)⁻ =440.09; |
| | | Found (M-H)⁻= 439.98 |
| (3S)-3-(1,3-benzodioxol-5-yl)-3-({[((3S)-2-oxo-1-{4-[(2-toluidinocarbonyl)amino]benzyl}hexahydro-3-pyridinyl)amino]carbonyl}amino)propanoic acid | 0.0003 | Calc'd (M-H)⁻ =586.23; |
| | | Found (M-H)⁻= 586.17 |
| (3S)-3-(1,3-benzodioxol-5-yl)-3-[({[2-oxo-1-{4-[(2-toluidinocarbonyl)amino]benzyl}-1,2-dihydro-3-pyridinyl]amino}carbonyl)amino]propanoic acid | 0.001 | Calc'd (M-H)⁻ =582.20; |
| | | Found (M-H)⁻= 582.20 |
| (3S)-3-(1,3-benzodioxol-5-yl)-3-({[((3S)-1-{4-[(2-methylbenzyl)amino]benzyl}-2-oxohexahydro-pyridinyl)amino]carbonyl} amino)propanoic acid | nd | nd |
| (3S)-3-(1,3-benzodioxol-5-yl)-3-[({butyl[2-oxo-1-(2-thienylmethyl)-1,2-dihydro-3-pyridinyl]amino} carbonyl)amino]propanoic acid | 20 | Calculated (M-H)⁻ = 496.15; |
| | | Found (M-H)⁻ = 496.10 |
| (3S)-3-(1,3-benzodioxol-5-yl)-3-[({[(3S)-2-oxo-1-(2-thienylmethyl)azepanyl]amino}carbonyl)amino]propanoic acid | 0.015 | Calculated (M-H)⁻ = 458.13; |
| | | Found (M-H)⁻ = 458.09 |

**Table 2**

| Compound | IC₅₀ (nM) | Mass Spectral Data |
|---|---|---|
| (3S)-3-[({[2-methyl-4-(2-methylpropyl)-6-oxo-1-(phenylmethyl)-1,6-dihydro-5-pyrimidinyl]amino}carbonyl)amino]-3-(4-methylphenyl)propanoic acid | 10 | Calculated (M-H)⁻ = 475.23 m/z; |
| | | Found (M-H)⁻ = 475.02 m/z. |
| (3S)-3-(1,3-benzodioxol-5-yl)-3-[({[2-oxo-1-(phenylmethyl)-4-propyl-1,2-dihydro-3-pyridinyl]amino}carbonyl)amino]propanoic acid | 10 | Calculated (M-H)⁻ = 476.18 m/z; |
| | | Found (M-H)⁻= 475.99 m/z. |
| (3S)-3-(1,3-benzodioxol-5-yl)-3-({[9-oxo-8-(phenylmethyl)-2,3,4,5,8,9-hexahydro-1H-pyrido[3,4-b]azepin-1-yl]carbonyl}amino)propanoic acid | 4000 | Calculated (M-H)⁻= 488.18 m/z; |
| | | Found (M-H)⁻ = 488.19 m/z. |
| (3S)-3-{[({1-[(2-chlorophenyl)methyl]-4-ethyl-2-oxo-1,2-dihydro-3-pyridinyl} amino)carbonyl]amino}-3-(4-methylphenyl)propanoic acid | 10 | Calculated (M-H)⁻ = 466.15 m/z; |
| | | Found (M-H)⁻ = 465.95 m/z. |
| (3S)-3 -{[({1-[(2-chlorophenyl)methyl]-2-oxo-4- propyl-1,2-dihydro-3-pyridinyl}amino)carbonyl]amino}-3-(4-methylphenyl)propanoic acid | 4 | Calculated (M-H)⁻ = 480.17 m/z; |
| | | Found (M-H)⁻ = 480.00 m/z. |
| (3S)-3-{[({1-[(2-chlorophenyl)methyl]-4-methyl- 2-oxo-1,2-dihydro-3-pyridinyl}amino)carbonyl]amino}-3-(4-methylphenyl)propanoic acid | 5 | Calculated (M+H)⁺= 454.15 m/z; |
| | | Found (M+H)⁺ = 454.09 m/z. |
| (3S)-3-{[({6-methyl-2-oxo-1-(phenylmethyl)-4-[(phenylmethyl)oxy]-1,2-dihydro-3-pyridinyl} amino)carbonyl]amino}-3-(4-methylphenyl)propanoic acid | 5 | Calculated (M-H)⁻= 524.22 m/z; |
| | | Found (M-H)⁻ = 524.02 m/z. |
| (3S)-3-{[({1-[(2-chlorophenyl)methyl]-2,4-dimethyl-6-oxo-1,6-dihydro-5-pyrimidinyl} amino)carbonyl]amino} -3-(4-methylphenyl)propanoic acid | 10 | Calculated (M-H)⁻= 467.15 m/z; Found (M-H)⁻ = 467.00 m/z. |
| (3S)-3-{[({1-[(2,4-dichlorophenyl)methyl]-4-methyl-2-oxo-1,2-dihydro-3-pyridinyl}amino)carbonyl]amino}-3-(4-methylphenyl)propanoic acid | 30 | Calculated (M-H)⁻ = 486.10 m/z; |
| | | Found (M-H)⁻ = 485.95 m/z. |
| (3S)-3-{[({4-amino-1-[(2-chlorophenyl)methyl]-6-methyl-2-oxo-1,2-dihydro-3-pyridinyl} amino)carbonyl]amino}-3-(4-methylphenyl)propanoic acid | 10 | Calculated (M-H)⁻ = 467.15 m/z; |
| | | Found (M-H)⁻ = 467.14 m/z. |
| (3S)-3-[({[1-[(2-chlorophenyl)methyl]-4-(methyloxy)-2-oxo-1,2-dihydro-3-pyridinyl]amino} carbonyl)amino]-3-(4-methylphenyl)propanoic acid | 20 | Calculated (M-H)⁻ = 468.13 m/z; |
| | | Found (M-H)⁻ = 467.97 m/z. |
| (3S)-3-{[({4-chloro-1-[(2-chlorophenyl)methyl]- 2-oxo-1,2-dihydro-3-pyridinyl}amino)carbonyl]amino}-3-(4-methylphenyl)propanoic acid | 20 | Calculated (M-H)⁻ = 472.08 m/z; |
| | | Found (M-H)⁻ = 471.91 m/z. |
| (3S)-3-{[({1-[(2-chlorophenyl)methyl]-4-methyl-2-oxo-1,2-dihydro-3-pyridinyl}amino)carbonyl]amino}-3-[3-methyl-4-(methyloxy)phenyl]propanoic acid | 15 | Calculated (M-H)⁻ = 482.15 m/z; |
| | | Found (M-H)⁻ = 481.93 m/z. |
| (3S)-3-{[({1-[(2-chlorophenyl)methyl]-4-methyl- 2-oxo-1,2-dihydro-3-pyridinyl}amino)carbonyl]amino}-3-[4-(methyloxy)phenyl]propanoic acid | 3 | Calculated (M-H)⁻=470.15 m/z; |
| | | Found (M-H)⁻ = 470.01 m/z. |
| (3S)-3-{[({1-[(2-chlorophenyl)methyl]-4-methyl-2-oxo-1,2-dihydro-3-pyridinyl}amino)carbonyl]amino}-3-(3,4-dimethylphenyl)propanoic acid | 10 | Calculated (M-H)⁻=468.17 m/z; |
| | | Found (M-H)⁻ = 468.05 m/z. |
| (3S)-3-{[({4-amino-1-[(2-chlorophenyl)methyl]-2-oxo-1,2-dihydro-3-pyridinyl}amino)carbonyl]amino}-3-(4-methylphenyl)propanoic acid | 10 | Calculated (M-H)⁻ = 453.13 m/z; |
| | 15 | Found (M-H)⁻ = 453.01 m/z. |
| (3S)-3-{[({1-[(2-chlorophenyl)methyl]-4-fluoro- 2-oxo-1,2-dihydro-3- pyridinyl} amino)carbonyl] amino}-3-(4-methylphenyl)propanoic acid | 10 | Calculated (M-H)⁻ = 456.12 m/z; |
| | | Found (M-H)⁻ = 455.94 m/z. |
| (3S)-3-[({[1-[(2-chlorophenyl)methyl]-2-oxo-4-(phenylamino)-1,2-dihydro-3-pyridinyl] amino} carbonyl)amino]-3-(4-methylphenyl)propanoic acid | 20 | Calculated (M-H)⁻ = 529.16 m/z; |
| | | Found (M-H)⁻ = 529.02 m/z. |
| (3S)-3-[({[1-[(2-chlorophenyl)methyl]-2-oxo-4-(2-pyridinylamino)-1,2-dihydro-3-pyridinyl]amino} carbonyl)amino]-3-(4-methylphenyl)propanoic acid | 15 | Calculated (M-H)⁻ = 530.16 m/z; |
| | | Found (M-H)⁻ = 529.99 m/z. |
| (3S)-3- {[({1-[(2-chlorophenyl)methyl]-4-hydroxy-2-oxo-1,2-dihydro-3-pyridinyl}amino)carbonyl]amino}-3-(4-methylphenyl)propanoic acid | 10 | Calculated (M-H)⁻ = 454.11 m/z; |
| | | Found (M-H)⁻ = 454.05 m/z. |
| (3S)-3-{[({1-[(2-chlorophenyl)methyl]-2-oxo-4-[(2-pyridinylmethyl)amino]-1,2-dihydro-3-pyridinyl}amino)carbonyl]amino}-3-(4-methylphenyl)propanoic acid | 15 | Calculated (M-H)⁻ = 544.17 m/z; |
| | | Found (M-H)⁻ = 544.03 m/z. |
| (3S)-3-{[({1-[(2-chlorophenyl)methyl]-2-oxo-4-[(3-pyridinylmethyl)amino]-1,2-dihydro-3-pyridinyl}amino)carbonyl]amino}-3-(4-methylphenyl)propanoic acid | 20 | Calculated (M-H)⁻ = 544.17 m/z; |
| | | Found (M-H)⁻ = 544.02 m/z. |
| (3S)-3-[({[1-[(2-chlorophenyl)methyl]-4-(1,4-oxazinan-4-yl)-2-oxo-1,2-dihydro-3-pyridinyl]amino}carbonyl)amino]-3-(4-methylphenyl)propanoic acid | 1 | Calculated (M-H)⁻ = 523.17 m/z; |
| | | Found (M-H)⁻ = 523.02 m/z. |
| (3S)-3-[({[1-[(2-chlorophenyl)methyl]-2-oxo-4-(propylamino)-1,2-dihydro-3-pyridinyl] amino}carbonyl)amino]-3-(4-methylphenyl)propanoic acid | 10 | Calculated (M-H)⁻ = 495.18 m/z; |
| | | Found (M-H)⁻ = 495.04 m/z. |
| (3S)-3-{[({1-[(3-fluorophenyl)methyl]-4-methyl-2-oxo-1,2-dihydro-3-pyridinyl}amino)carbonyl]amino}-3-(4-methylphenyl)propanoic acid | 20 | Calculated (M-H)⁻ = 436.17 m/z; |
| | | Found (M-H)⁻ = 435.99 m/z. |
| (3S)-3- {[({1-[(2,6-dichlorophenyl)methyl]-4-methyl-2-oxo-1,2-dihydro-3-pyridinyl} amino)carbonyl]amino} -3-(4-methylphenyl)propanoic acid | 20 | Calculated (M-H)⁻ = 486.10 m/z; |
| | | Found (M-H)⁻ = 485.95 m/z. |
| (3R)-3-{[({1-[(2-chlorophenyl)methyl]-4-methyl-2-oxo-1,2-dihydro-3-pyridinyl} amino)carbonyl]amino }butanoic acid | 30 | Calculated (M-H)⁻ = 376.11 m/z ; |
| | | Found (M-H)⁻ = 376.00 m/z. |
| (3S)-3-{[({1-[(2-bromophenyl)methyl]-4-methyl-2-oxo-1,2-dihydro-3-pyridinyl} amino)carbonyl]amino }-3-(4-methylphenyl)propanoic acid | 10 | Calculated (M-H)⁻=496.09 m/z; |
| | | Found (M-H)⁻ = 495.87 m/z. |
| (3S)-3-[({[4-methyl-2-oxo-1-(phenyhnethyl)-1,2-dihydro-3-pyridinyl]amino}carbonyl)amino]-3-(4-methylphenyl)propanoic acid | 30 | Calculated (M-H)⁻ = 418.17 m/z; |
| | | Found (M-H)⁻ = 417.96 m/z. |
| (3S)-3-{[({1-[(2-chlorophenyl)methyl]-4-hydroxy-2-oxo-1,2-dihydro-3-pyridinyl}amino)carbonyl]amino}-3-[3-methyl-4-(methyloxy)phenyl]propanoic acid | 8 | Calculated (M-H)⁻ = 484.12 m/z; |
| | | Found (M-H)⁻ = 484.03 m/z. |
| (3S)-3-{[({1-[(2-chlorophenyl)methyl]-2-oxo-4-phenyl-1,2-dihydro-3-pyridinyl}amino)carbonyl]amino}-3-(4-methylphenyl)propanoic acid | 10 | Calculated (M-H)⁻ = 514.15 m/z; |
| | | Found (M-H)⁻ = 514.00 m/z. |
| (3S)-3-{[({4-bromo-1-[(2-chlorophenyl)methyl]-2-oxo-1,2-dihydro-3-pyridinyl} amino)carbonyl]amino}-3-(4-methylphenyl)propanoic acid | 20 | Calculated (M-H)⁻ = 516.03 m/z; |
| | | Found (M-H)⁻ = 515.90 m/z. |
| (3S)-3-(1,3-benzodioxol-5-yl)-3-{[({1-[(2-chlorophenyl)methyl]-4-hydroxy-2-oxo-1,2-dihydro-3-pyridinyl} amino)carbonyl] amino}propanoic acid | 20 | Calculated (M-H)⁻ = 484.09 m/z; |
| | | Found (M-H)⁻= 484.03 m/z. |
| (3S)-3-{[({1-[(2-chlorophenyl)methyl]-4-[(2-{[2-(methyloxy)ethyl]oxy}ethyl)oxy]-2-oxo-1,2-dihydro-3-pyridinyl} amino)carbonyl]amino} -3-(4-methylphenyl)propanoic acid | 2 | Calculated (M-H)⁻ = 556.18 m/z; |
| | | Found (M-H)⁻ = 556.03 m/z. |
| (3S)-3-{[({1-[(2-chlorophenyl)methyl]-4-hydroxy-6-methyl-2-oxo-1,2-dihydro-3-pyridinyl} amino)carbonyl]amino} -3-(4-methylphenyl)propanoic acid | 15 | Calculated (M-H)⁻ = 468.13 m/z; |
| | | Found (M-H)⁻ = 468.05 m/z. |
| (3S)-3-{[({1-[(2-chlorophenyl)methyl]-4-[(1,1-dimethylethyl)amino]-2-oxo-1,2-dihydro-3-pyridinyl}amino)carbonyl]amino}-3-(4-methylphenyl)propanoic acid | 3 | Calculated (M-H)⁻ = 509.20 m/z; |
| | | Found (M-H)⁻ = 509.06 m/z. |
| (3S)-3-{[({1-[(2-chlorophenyl)methyl]-4-hydroxy-2-oxo-1,2-dihydro-3-pyridinyl} amino)carbonyl] amino }-3-phenylpropanoic acid | 10 | Calculated (M-H)⁻=440.10 m/z; |
| | | Found (M-H)⁻ = 440.04 m/z. |
| (3S)-3-{[({1-[(2-chlorophenyl)methyl]-4-[4-methyltetrahydro-1(2H)-pyrazinyl]-2-oxo-1,2-dihydro-3-pyridinyl}amino)carbonyl]amino}-3-(4-methylphenyl)propanoic acid | 3 | Calculated (M-H)⁻ =536.20 m/z; |
| | | Found (M-H)⁻ = 536.12 m/z. |
| (3S)-3-{[({1-[(2-chlorophenyl)methyl]-4-hydroxy-2-oxo-1,2-dihydro-3-pyridinyl} amino)carbonyl]amino} -3-[4-(methyloxy)phenyl]propanoic acid | 5 | Calculated (M-H)⁻ = 470.11 m/z; |
| | | Found (M-H)⁻ = 470.05 m/z. |
| (3S)-3- {[({1-[(2-chlorophenyl)methyl]-4-hydroxy-2-oxo-1,2-dihydro-3-pyridinyl} amino)carbonyl]amino} -3-[3,4,5-tris(methyloxy)phenyl]propanoic acid | 20 | Calculated (M-H)⁻ = 530.13 m/z; |
| | | Found (M-H)⁻ = 530.05 m/z. |
| (3S)-3-{[({1-[(2-chlorophenyl)methyl]-4-hydroxy-2-oxo-1,2-dihydro-3-pyridinyl}amino)carbonyl]amino}-3-(3,5-dimethylphenyl)propanoic acid | 15 | Calculated (M-H)⁻ = 468.13 m/z; |
| | | Found (M-H)⁻ = 468.08 m/z. |
| (3S)-3-{[({1-[(2-chlorophenyl)methyl]-4-[(3-methyl-5-isoxazolyl)amino]-2-oxo-1,2-dihydro-3-pyridinyl} amino)carbonyl] amino} -3 -(4-methylphenyl)propanoic acid | 15 | Calculated (M-H)⁻ = 534.15 m/z; |
| | | Found (M-H)⁻ = 534.01 m/z. |
| (3S)-3- {[({1-[(2-chlorophenyl)methyl]-4-hydroxy-2-oxo-1,2-dihydro-3-pyridinyl}amino)carbonyl]amino}-3-(3-methylphenyl)propanoic acid | 20 | Calculated (M-H)⁻ = 454.17 m/z; |
| | | Found (M-H)⁻ = 454.04 m/z. |
| (3S)-3-{[({1-[(2-chlorophenyl)methyl]-4-hydroxy-2-oxo-1,2-dihydro-3-pyridinyl} amino)carbonyl] amino}-3-[3-(methyloxy)phenyl]propanoic acid | 5 | Calculated (M-H)⁻ = 470.11 m/z; |
| | | Found (M-H)⁻ = 470.03 m/z. |
| (3S)-3-[3,5-bis(methyloxy)phenyl]-3-{[({1-[(2-chlorophenyl)methyl]-4-hydroxy-2-oxo-1,2-dihydro-3-pyridinyl} amino)carbonyl]amino}propanoic acid | 3 | Calculated (M-H)⁻ =500.12 m/z; |
| | | Found (M-H)⁻= 500.07 m/z. |
| (3S)-3-{[({1-[(2-chlorophenyl)methyl]-4-hydroxy-2-oxo-1,2-dihydro-3-quinolinyl}amino)carbonyl]amino}-3-(4-methylphenyl)propanoic acid | 8 | Calculated (M-H)⁻ = 504.13 m/z; |
| | | Found (M-H)⁻ = 504.06 m/z. |
| (3S)-3-{[({1-[(2-chlorophenyl)methyl]-4-hydroxy-2-oxo-1,2-dihydro-3-pyridinyl} amino)carbonyl]amino} -3-[3 -(trifluoromethyl)phenyl]propanoic acid | 20 | Calculated (M-H)⁻ = 508.04 m/z; |
| | | Found (M-H)⁻ = 508.09 m/z. |
| (3S)-3-{[({1-[(2-chlorophenyl)methyl]-4-[({ethyl[(ethylamino)carbonyl]amino}carbonyl) amino]-2-oxo-1,2-dihydro-3-pyridinyl} amino)carbonyl]amino} -3-(4-methylphenyl)propanoic acid | 2 | Calculated (M-H)⁻= 595.21 m/z; |
| | | Found (M-H)⁻ = 594.97 m/z. |
| (3S)-3-{[({4-(1-azetanyl)-1-[(2-chlorophenyl)methyl]-2-oxo-1,2-dihydro-3-pyridinyl} amino)carbonyl]amino}-3-(4-methylphenyl)propanoic acid | 5 | Calculated (M-H)⁻=493.16 m/z; |
| | | Found (M-H)⁻ = 493.05 m/z. |
| (3S)-3-{[({1-[(2-chlorophenyl)methyl]-4-hydroxy-2-oxo-1,2-dihydro-3-pyridinyl} amino)carbonyl]amino} -3-(4-fluorophenyl)propanoic acid | 30 | Calculated (M-H)⁻ = 458.09 m/z; |
| | | Found (M-H)⁻ = 458.03 m/z. |
| (3S)-3-{[({1-[(2-chlorophenyl)methyl]-4-hydroxy-2-oxo-1,2-dihydro-3-pyridinyl}amino)carbonyl]amino}-3-(3-fluorophenyl)propanoic acid | 40 | Calculated (M-H)⁻ = 458.09 m/z; |
| | | Found (M-H)⁻ = 458.06 m/z. |
| (3S)-3-[({[1-[(2-chlorophenyl)methyl]-4-({2-[(2-{[2-(methyloxy)ethyl]oxy}ethyl)oxy]ethyl}oxy)-2-oxo-1,2-dihydro-3-pyridinyl]amino}carbonyl)amino]-3-(4methylphenyl)propanoic acid | 2 | Calculated (M-H)⁻ = 600.21 m/z; |
| | | Found (M-H)⁻ = 600.10 m/z. |
| (3S)-3-{[({1-[(2-chlorophenyl)methyl]-4-hydroxy-2-oxo-1,2-dihydro-3- pyridinyl} amino)carbonyl]amino}-3-[4-(trifluoromethyl)phenyl]propanoic acid | 25 | Calculated (M-H)⁻= 508.09 m/z; |
| | | Found (M-H)⁻ =508.02 m/z. |
| (3S)-3-{[({1-[(2-fluorophenyl)methyl]-4-hydroxy-2-oxo-1,2-dihydro-3-pyridinyl} amino)carbonyl]amino} -3-(4-methylphenyl)propanoic acid | 30 | Calculated (M-H)⁻ = 438.15 m/z; |
| | | Found (M-H)⁻ = 438.07 m/z. |
| (3S)-3-{[({1-[(2-chloro-6-fluorophenyl)methyl]-4-hydroxy-2-oxo-1,2-dihydro-3-pyridinyl}amino)carbonyl]amino} -3-(4-methylphenyl)propanoic acid | 10 | Calculated (M-H)⁻=472.11 m/z; |
| | | Found (M-H)⁻ = 472.06 m/z. |
| (3S)-3-{[({1-[(2-chlorophenyl)methyl]-4-hydroxy-2-oxo-1,2-dihydro-3-pyridinyl}amino)carbonyl]amino}-3-[4-(1,1-dimethylethyl)phenyl]propanoic acid | 400 | Calculated (M-H)⁻ = 496.16 m/z; |
| | | Found (M-H)⁻ = 496.11 m/z. |
| (3S)-3-{[({1-[(2-chlorophenyl)methyl]-5-methyl-2-oxo-1,2-dihydro-3-pyridinyl}amino)carbonyl]amino}-3-(4-methylphenyl)propanoic acid | 70 | Calculated (M-H)⁻ = 452.14 m/z; |
| | | Found (M-H)⁻ = 451.99 m/z. |
| 3-(4-chlorophenyl)-3-{[({1-[(2-chlorophenyl)methyl]-4-hydroxy-2-oxo-1,2-dihydro-3-pyridinyl}amino)carbonyl]amino}propanoic acid | 30 | Calculated (M-H)⁻ = 474.06 m/z; |
| | | Found (M-H)⁻ = 474.07 m/z. |
| (3S)-3-[({[2-methyl-6-oxo-1-(phenylmethyl)-4-(2-pyridinyl)-1,6-dihydro-5-pyrimidinyl]amino}carbonyl)amino]-3-(4-methylphenyl)propanoic acid | 25 | Calculated (M+H)⁺= 498.22 m/z; |
| | | Found (M+H)⁺ = 498.10 m/z. |
| 3-(3-chlorophenyl)-3-{[({1-[(2-chlorophenyl)methyl]-4-hydroxy-2-oxo-1,2-dihydro-3-pyridinyl} amino)carbonyl] amino} propanoic acid | 30 | Calculated (M-H)⁻ = 474.06 m/z; |
| | | Found (M-H)⁻ = 474.03 m/z. |
| 3-{[({1-[(2-chlorophenyl)methyl]-4-hydroxy-2-oxo-1,2-dihydro-3-pyridinyl}amino)carbonyl]amino}-3-(3,4-dichlorophenyl)propanoic acid | 40 | Calculated (M-H)⁻= 508.02 m/z; |
| | | Found (M-H)⁻ = 507.97 m/z. |

**Table 3**

| Name | IC₅₀ | Mass Spectral Data |
|---|---|---|
| (3S)-3-(1,3-benzodioxol-5-yl)-3-[({[2-oxo-1- (phenylmethyl)-3-azepanyl]amino}carbonyl)amino]propanoic acid | 0.015 | Calculated (M-H)⁻=452.18 m/z; |
| | | Found (M-H)⁻ = 452.10 m/z. |
| (3S)-3-(1,3-benzodioxol-5-yl)-3-{[({1-[(3- cyanophenyl)methyl]-2-oxo-3-azepanyl}amino)carbonyl]amino}propanoic acid | 0.04 | Calculated (M-H)⁻= 477.18 m/z; |
| | | Found (M-H)⁻ = 477.14 m/z. |
| (3S)-3-(4-methylphenyl)-3-[({[2-oxo-1-(2- thiophenylmethyl)-1,2-dihydro-3-pyridinyl]amino}carbonyl)amino]propanoic acid | 0.6 | Calculated (M-H)⁻ = 410.11 m/z; |
| | | Found (M-H)⁻ = 410.00 m/z. |
| (3S)-3-(1,3-benzodioxol-5-yl)-3-[({[2-oxo-1- (phenylmethyl)-1,2-dihydro-3-pyridinyl]amino}carbonyl)amino]propanoic acid | 0.5 | Calculated (M-H)⁻ = 434.13 m/z; |
| | | Found (M-H)⁻ = 434.05 m/z. |
| (3S)-3-(1,3-benzodioxol-5-yl)-3-{[({1-[(4- methylphenyl)methyl]-2-oxo-1,2-dihydro-3- pyridinyl}amino)carbonyl]amino}propanoic acid | 1 | Calculated (M-H)⁻ = 448.14 m/z; |
| | | Found (M-H)⁻ = 448.02 m/z. |
| (3S)-3-(1,3-benzodioxol-5-yl)-3-({[(1-{[4- (methyloxy)phenyl]methyl}-2-oxo-1,2-dihydro-3-pyridinyl)amino]carbonyl} amino)propanoic acid | 3 | Calculated (M-H)⁻ = 464.14 m/z; |
| | | Found (M-H)⁻ = 464.03 m/z. |
| (3S)-3-(1,3-benzodioxol-5-yl)-3-{[({1-[(3- methylphenyl)methyl]-2-oxo-1,2-dihydro-3-pyridinyl}amino)carbonyl]amino}propanoic acid | 1.5 | Calculated (M-H)⁻ = 448.15 m/z; |
| (3S)-3-[3,5-bis(methyloxy)phenyl]-3-[({[2-oxo-1-(2-thiophenylmethyl)-1,2-dihydro-3-pyridinyl]amino}carbonyl)amino]propanoic acid | 0.7 | Found (M-H)⁻ = 448.04 m/z. Calculated (M-H)⁻ = 456.12 m/z; Found (M-H)⁻ = 456.00 m/z. |
| (3S)-3-[4-(methyloxy)phenyl]-3-[({[2-oxo-1-(2-thiophenylmethyl)-1,2-dihydro-3-pyridinyl]amino}carbonyl)amino]propanoic acid | 0.8 | Calculated (M-H)⁻ = 426.11 m/z; |
| | | Found (M-H)⁻ = 426.00 m/z. |
| (3S)-3-[({[2-oxo-1-(2-thiophenylmethyl)-1,2-dihydro-3-pyridinyl]amino}carbonyl)amino]-3-[3-(trifluoromethyl)phenyl]propanoic acid | 2.5 | Calculated (M-H)⁻ = 464.09 m/z; |
| | | Found (M-H)⁻ = 463.99 m/z. |
| (3S)-3-(1,3-benzodioxol-5-yl)-3-[({[3-(phenyloxy)phenyl]amino}carbonyl)amino] propanoic acid | 50 | Calculated (M-H)⁻=419.12 m/z; |
| | | Found (M-H)⁻ = 418.97 m/z. |
| (3S)-3-(1,3-benzodioxol-5-yl)-3- {[({3-[(2- thiophenylmethyl)amino]phenyl}amino)carbonyl] amino}propanoic acid | 5 | Calculated (M-H)⁻ = 438.11 m/z; |
| | | Found (M-H)⁻ = 438.00 m/z. |
| (3S)-3-(1,3-benzodioxol-5-yl)-3-{[({1-[(3-chlorophenyl)methyl]-2-oxo-1,2-dihydro-3-pyridinyl} amino)carbonyl]amino}propanoic acid | 0.8 | Calculated (M-H)⁻ = 468.09 m/z; |
| | | Found (M-H)⁻ = 468.01 m/z. |
| (3S)-3-(1,3-benzodioxol-5-yl)-3-({[(2-oxo-1-{[3-(trifluoromethyl)phenyl]methyl}-1,2-dihydro-3-pyridinyl)amino]carbonyl} amino)propanoic acid | 0.8 | Calculated (M-H)⁻ = 502.12 m/z; |
| | | Found (M-H)⁻ = 502.03 m/z. |
| (3S)-3-(1,3-benzodioxol-5-yl)-3-({[(2-oxo-1- {[4-(trifluoromethyl)phenyl]methyl}-1,2-dihydro-3-pyridinyl)amino]carbonyl}amino)propanoic acid | 1.6 | Calculated (M-H)⁻ = 502.12 m/z; |
| | | Found (M-H)⁻= 502.01 m/z. |
| (3S)-3-(4-fluorophenyl)-3-[({[2-oxo-1-(2-throphenylmethyl)-1,2-dihydro-3-pyridinyl]amino}carbonyl)amino]propanoic acid | 1.6 | Calculated (M-H)⁻ = 414.09 m/z; |
| | | Found (M-H)⁻ = 414.01 m/z. |
| (3S)-3-(1,3-benzodioxol-5-yl)-3-{[({1-[(4-chlorophenyl)methyl]-2-oxo-1,2-dihydro-3-pyridinyl} amino)carbonyl]amino}propanoic acid | 3 | Calculated (M-H)⁻ = 468.09 m/z; |
| | | Found (M-H)⁻ = 467.99 m/z. |
| (3S)-3-(1,3-benzodioxol-5-yl)-3-({[(1-{[2-(methyloxy)phenyl]methyl}-2-oxo-1,2-dihydro-3-pyridinyl)amino]carbonyl}amino)propanoic acid | 0.5 | Calculated (M-H)⁻ = 464.14 m/z; |
| | | Found (M-H)⁻ = 464.04 m/z. |
| (3S)-3-[3-(methyloxy)phenyl]-3-[({[2-oxo-1-(2-thiophenylmethyl)-1,2-dihydro-3-pyridinyl]amino}carbonyl)amino]propanoic acid | 1.4 | Calculated (M-H)⁻ = 426.11 m/z; |
| | | Found (M-H)⁻ = 426.02 m/z. |
| (3S)-3-[({[2-oxo-1-(2-thiophenylmethyl)-1,2-dihydro-3-pyridinyl]amino}carbonyl)amino]-3-phenylpropanoic acid | 1 | Calculated (M-H)⁻ = 396.10 m/z; |
| | | Found (M-H)⁻ = 396.01 m/z. |
| (3S)-3-[({[2-oxo-1-(2-thiophenylmethyl)-1,2-dihydro-3-pyridinyl]amino}carbonyl)amino]-3-[3,4,5-tris(methyloxy)phenyl]propanoic acid | 0.3 | Calculated (M-H)⁻= 486.13 m/z; |
| | | Found (M-H)⁻ = 485.98 m/z. |
| (3S)-3-(1,3-benzodioxol-5-yl)-3-{[({1-[(2-chlorophenyl)methyl]-2-oxo-1,2-dihydro-3-pyridinyl} amino)carbonyl]amino} propanoic acid | 0.3 | Calculated (M-H)⁻=468.08 m/z; |
| | | Found (M-H)⁻= 468.03 m/z. |
| (3S)-3-(1,3-benzodioxol-5-yl)-3-{[({1-[(4-fluorophenyl)methyl]-2-oxo-1,2-dihydro-3-pyridinyl} amino)carbonyl]amino}propanoic acid | 2 | Calculated (M-H)⁻ = 452.12 m/z; |
| | | Found (M-H)⁻ = 452.00 m/z. |
| 3-(1,3-benzodioxol-5-yl)-2,2-difluoro-3-[({[2-oxo-1-(2-thiophenylmethyl)-1,2-dihydro-3-pyridinyl]amino} carbonyl)amino]propanoic acid | >100 | Calculated (M-H)- = 476.07 m/z; |
| | | Found (M-H)⁻ = 476.00 m/z. |
| (3S)-3-(1,3-benzodioxol-5-yl)-3-{[({2-oxo-1-[3-(phenyloxy)propyl]-1,2-dihydro-3-pyridinyl} amino)carbonyl]amino} propanoic acid | 14 | Calculated (M-H)⁻ = 478.16 m/z; |
| | | Found (M-H)⁻ = 478.09 m/z. |
| (3S)-3-(1,3-benzodioxol-5-yl)-3-{[({1-[(3,4-dichlorophenyl)methyl]-2-oxo-1,2-dihydro-3-pyridinyl} amino)carbonyl] amino }propanoic acid | 4 | Calculated (M-H)⁻= 502.05 m/z; |
| | | Found (M-H)⁻ = 501.98 m/z. |
| (3S)-3-(1,3-benzodioxol-5-yl)-3-{[({1-[(3,5-dichlorophenyl)methyl]-2-oxo-1,2-dihydro-3-pyridinyl}amino)carbonyl]amino}propanoic acid | 5 | Calculated (M-H)⁻ = 502.05 m/z; |
| | | Found (M-H)⁻ = 501.94 m/z. |
| (3S)-3-(1,3-benzodioxol-5-yl)-3-[({[1-(cyclopentylmethyl)-2-oxo-1,2-dihydro-3-pyridinyl]amino}carbonyl)amino]propanoic acid | 6 | Calculated (M-H)⁻= 426.16 m/z; |
| | | Found (M-H)⁻ = 426.09 m/z. |
| (3S)-3-(1,3-benzodioxol-5-yl)-3-{[({2-oxo-1-[2-(2-thiophenyl)ethyl]-1,2-dihydro-3-pyridinyl}amino)carbonyl]amino}propanoic acid | 15 | Calculated (M-H)⁻=454.09 m/z; |
| | | Found (M-H)⁻ = 453.99 m/z. |
| (3S)-3-{[({1-[(2-chlorophenyl)methyl]-2-oxo-1,2-dihydro-3- pyridinyl}amino)carbonyl]amino}-3-(4-methylphenyl)propanoic acid | 0.1 | Calculated (M+H)⁺ = 440.14 m/z; |
| | | Found (M+H)⁺ = 440.09 m/z. |
| (3S)-3-(2,3-dihydro-1-benzofuran-5-yl)-3-[({[2-oxo-1-(2-thiophenylmethyl)-1,2-dihydro-3-pyridinyl]amino}carbonyl)amino]propanoic acid | 0.14 | Calculated (M-H)⁻= 438.11 m/z; |
| | | Found (M-H)⁻ = 437.99 m/z. |
| (3S)-3-(3-fluorophenyl)-3-[({[2-oxo-1-(2-thiophenylmethyl)-1,2-dihydro-3-pyridinyl]amino}carbonyl)amino]propanoic acid | 3 | Calculated (M-H)⁻ = 414.09 m/z; |
| | | Found (M-H)⁻ = 413.99 m/z. |
| (3S)-3-[({[2-oxo-1-(2-thiophenylmethyl)-1,2-dihydro-3-pyridinyl]amino} carbonyl)amino]-3-[4-(trifluoromethyl)phenyl]propanoic acid | 1.5 | Calculated (M-H)⁻ = 464.09 m/z; |
| | | Found (M-H)⁻ = 463.99 m/z. |
| (3S)-3-(1,3-benzodioxol-5-yl)-3-[({[6-oxo-1-(phenyhnethyl)-1,6-dihydro-3-pyridinyl]amino}carbonyl)amino]propanoic acid | 0.5 | Calculated (M-H)⁻ = 434.13 m/z; |
| | | Found (M-H)⁻ = 434.02 m/z. |
| (3S)-3-[4-fluoro-3-(trifluoromethyl)phenyl]-3-[({[2-oxo-1-(2-thiophenylmethyl)-1,2-dihydro-3-pyridinyl]amino}carbonyl)amino]propanoic acid | 0.35 | Calculated (M-H)⁻= 482.08 m/z; |
| | | Found (M-H)⁻ = 481.97 m/z. |
| (3S)-3-[4-(1,1-dimethylethyl)phenyl]-3-[({[2-oxo-1-(2-thiophenylmethyl)-1,2-dihydro-3-pyridinyl]amino}carbonyl)amino]propanoic acid | 2 | Calculated (M-H)⁻= 452.16 m/z; |
| | | Found (M-H)⁻ = 452.02 m/z. |
| (3S)-3-(1,3-benzodioxol-5-yl)-3-[({butyl[2,5-dioxo-1-(phenylmethyl)tetrahydro-1H-pyrrol-3-yl]amino}carbonyl)amino]propanoic acid | 70 | Calculated (M-H)⁻ = 494.19 m/z; |
| | | Found (M-H)⁻ = 494.12 m/z. |
| (3S)-3-{[({1-[(2-chlorophenyl)methyl]-2-oxo-1,2-dihydro-3-pyridinyl}amino)carbonyl]amino}-3-[3,4,5-tris(methyloxy)phenyl]propanoic acid | 0.04 | Calculated (M+H)⁺ = 516.16 m/z; |
| | | Found (M+H)⁺ = 516.02 m/z. |
| (3S)-3-{[({1-[(2,6-dichlorophenyl)methyl]-2-oxo-1,2-dihydro-3-pyridinyl}amino)carbonyl]amino}-3-(4methylphenyl)propanoic acid | 0.2 | Calculated (M+H)⁺ = 474.10 m/z; |
| | | Found (M+H)⁺ = 474.04 m/z. |
| (3S)-3-{[({1-[(2-chlorophenyl)methyl]-2-oxo-1,2-dihydro-3-pyridinyl}amino)carbonyl]amino}-3-[4-fluoro-3-(trifluoromethyl)phenyl]propanoic acid | 0.2 | Calculated (M+H)⁺= 512.10 m/z; |
| | | Found (M+H)⁺ = 512.04 m/z. |
| (3S)-3-{[({1-[(2-fluorophenyl)methyl]-2-oxo-1,2-dihydro-3-pyridinyl}amino)carbonyl]amino} -3-(4-methylphenyl)propanoic acid | 0.1 | Calculated (M-H)⁻ = 422.15 m/z; |
| | | Found (M-H)⁻ = 422.01 m/z. |
| (3S)-3-(4-methylphenyl)-3-{[({1-[(2-methylphenyl)methyl]-2-oxo-1,2-dihydro-3-pyridinyl}amino)carbonyl]amino}propanoic acid | 0.1 | Calculated (M-H)⁻ = 418.18 m/z; |
| | | Found (M-H)⁻ = 418.02 m/z. |
| (3S)-3-{[({1-[(2-bromophenyl)methyl]-2-oxo-1,2-dihydro-3-pyridinyl} amino)carbonyl] amino}-3-(4-methylphenyl)propanoic acid | 0.05 | Calculated (M+H)⁺ = 484.09 m/z; |
| | | Found (M+H)⁺ = 484.03 m/z. |
| (3S)-3-{[({1-[(2,4-dichlorophenyl)methyl]-2-oxo-1,2-dihydro-3-pyridinyl}amino)carbonyl]amino}-3-(4-methylphenyl)propanoic acid | 0.4 | Calculated (M+H)⁺ = 474.10 m/z; |
| | | Found (M+H)⁺ = 474.05 m/z. |
| (3S)-3-{[({1-[(2-chlorophenyl)methyl]-2-oxo-1,2-dihydro-3-pyridinyl} amino)carbonyl]amino}-3-(2,3-dihydro-1-benzofuran-5-yl)propanoic acid | 0.04 | Calculated (M-H)⁻ = 466.11 m/z; |
| | | Found (M-H)⁻ = 466.00 m/z. |
| (3S)-3-(1,3-benzodioxol-5-yl)-3-{[({1-[(2-chlorophenyl)methyl]-2-oxo-1,2-dihydro-3-pyridinyl} amino)carbonyl]amino}propanoic acid | 2 | Calculated (M-H)⁻ = 468.09 m/z; |
| | | Found (M-H)⁻ = 467.97 m/z. |
| (3S)-3-(4-methylphenyl)-3-({[(2-oxo-1-{[2-(trifluoromethyl)phenyl]methyl}-1,2-dihydro-3- pyridinyl)amino]carbonyl} amino)propanoic acid | 1 | Calculated (M+H)⁺= 474.10 m/z ; |
| | | Found (M+H)⁺ = 474.09 m/z. |
| (3S)-3-{[({1-[(2,5-dichlorophenyl)methyl]-2-oxo-1,2-dihydro-3-pyridinyl}amino)carbonyl]amino}-3-(4-methylphenyl)propanoic acid | 0.15 | Calculated (M+H)⁺ = 474.10 m/z; |
| | | Found (M+H)⁺ = 474.04 m/z. |
| (2R)-2-{[({1-[(2-chlorophenyl)methyl]-2-oxo-1,2-dihydro-3-pyridinyl}amino)carbonyl]amino}-3-phenylpropanoic acid | 50 | Calculated (M-H)⁻ = 424.10 m/z ; |
| | | Found (M-H)⁻ = 423.99 m/z. |
| (2R)-2-{[({1-[(2-chlorophenyl)methyl]-2-oxo-1,2-dihydro-3-pyridinyl}amino)carbonyl]amino} -2-phenylethanoic acid | 80 | Calculated (M-H)⁻ = 410.08 m/z ; |
| | | Found (M-H)⁻ = 409.95 m/z. |
| (3S)-3-{[({1-[(2-chlorophenyl)methyl]-2-oxo-1,2-dihydro-3-pyridinyl} amino)carbonyl]amino} -3-(3,5-dimethylphenyl)propanoic acid | 0.1 | Calculated (M-H)⁻ = 452.14 m/z ; |
| | | Found (M-H)⁻ = 451.96 m/z. |
| (3S)-3-{[({1-[(2-chlorophenyl)methyl]-2-oxo-1,2-dihydro-3-pyridinyl}amino)carbonyl]amino} -3-phenylpropanoic acid | 0.1 | Calculated (M. H)⁻ = 424.10 m/z; |
| | | Found (M-H)⁻ = 424.07 m/z. |
| (3S)-3-{[({1-[(2-chlorophenyl)methyl]-2-oxo-1,2-dihydro-3-pyridinyl} amino)carbonyl]amino}-3-[4-(methyloxy)phenyl]propanoic acid | 0.1 | Calculated (M-H)⁻ = 454.11 m/z; |
| | | Found (M-H)⁻ = 454.01 m/z. |
| (3S)-3- {[({1-[(2-chlorophenyl)methyl]-2-oxo-1,2-dihydro-3-pyridinyl} amino)carbonyl]amino}-3-(4-hydroxyphenyl)propanoic acid | 0.1 | Calculated (M-H)⁻ = 440.10 m/z; |
| | | Found (M-H)⁻ = 440.00 m/z. |
| (3S)-3-({[(1-{[3-(methyloxy)phenyl]methyl}-2-oxo-1,2-dihydro-3-pyridinyl)amino]carbonyl}amino)-3-(4-methylphenyl)propanoic acid | 0.2 | Calculated (M-H)⁻= 434.17 m/z; |
| | | Found (M-H)⁻ = 434.01 m/z. |
| (3S)-3-{[({1-[(2-bromophenyl)methyl]-2-oxo-1,2-dihydro-3-pyridinyl} amino)carbonyl]amino}-3-[3,4,5-tris(methyloxy)phenyl]propanoic acid | 0.08 | Calculated (M-H)⁻ = 558.09 m/z; |
| | | Found (M-H)⁻ = 557.87 m/z. |
| (3S)-3-{[({1-[(2-chlorophenyl)methyl]-2-oxo-1,2-dihydro-3-pyridinyl}amino)carbonyl]amino}-3 -(3,4-dimethylphenyl)propanoic acid | 0.09 | Calculated (M+H)⁺ = 454.15 m/z; |
| | | Found (M+H)⁺ = 454.07 m/z. |
| (3S)-3-[({[5-chloro-2-hydroxy-3-(phenylmethyl)phenyl]amino}carbonyl)amino]-3-(4-methylphenyl)propanoic acid | 0.8 | Calculated (M-H)⁻ = 437.12 m/z; |
| | | Found (M-H)⁻ = 437.06 m/z. |
| (3S)-3-(4-methylphenyl)-3-[({[3-(phenylmethyl)phenyl]amino}carbonyl)amino] propanoic acid | 10 | Calculated (M-H)⁻ =387.17 m/z ; |
| | | Found (M-H)⁻ = 387.00 m/z. |
| (3S)-3-{[({1-[(2-chlorophenyl)methyl]-2-oxo-1,2-dihydro-3-pyridinyl} amino)carbonyl]amino} -3-[3-methyl-4-(methyloxy)phenyl]propanoic acid | 0.04 | Calculated (M-H)⁻ = 468.13 m/z; |
| | | Found (M-H)⁻ = 468.01 m/z. |
| (3S)-3-{[({1-[(2-chlorophenyl)methyl]-2-oxo-1,2-dihydro-3-pyridinyl}amino)carbonyl]amino} -3-(4-hydroxy-3-metliylphenyl)propanoic acid | 0.07 | Calculated (M-H)⁻ = 454.11 m/z; |
| | | Found (M-H)⁻ = 454.00 m/z. |
| (3S)-3-{[({1-[(2,3-dichlorophenyl)methyl]-2-oxo-1,2-dihydro-3-pyridinyl} amino)carbonyl]amino}-3-(4-methylphenyl)propanoic acid | 0.35 | Calculated (M-H)⁻ = 472.08 m/z; |
| | | Found (M-H)⁻ = 471.94 m/z. |
| (3S)-3-[({[1-([1,1'-biphenyl]-2-ylmethyl)-2-oxo-1,2-dihydro-3-pyridinyl]amino} carbonyl)amino]-3-(4-methylphenyl)propanoic acid | 2.5 | Calculated (M-H)⁻ = 480.19 m/z; |
| | | Found (M-H)⁻ = 480.05 m/z. |
| (3S)-3-{[({1-[(2-chlorophenyl)methyl]-2-oxo-1,2-dihydro-3-pyridinyl} amino)carbonyl]amino} -3-(3-methylphenyl)propanoic acid | 0.2 | Calculated (M-H)⁻=438.12 m/z; |
| | | Found (M-H)⁻ = 438.00 m/z. |
| (3S)-3-{[({1-[(2-chlorophenyl)methyl]-2-oxo-1,2-dihydro-3-pyridinyl}amino)carbonyl]amino}-3-(2-methylphenyl)propanoic acid | 3 | Calculated (M-H)⁻ = 438.12 m/z; |
| | | Found (M-H)⁻ = 437.99 m/z. |
| (3S)-3-{[({1-[(2-chlorophenyl)methyl]-2-oxo-1,2-dihydro-3-pyridinyl}amino)carbonyl]amino}-3-(2,3-dihydro-1H-inden-5-yl)propanoic acid | 0.3 | Calculated (M-H)⁻ = 464.13 m/z; |
| | | Found (M-H)⁻ = 464.03 m/z. |
| (3S)-3-{[({1-[(2-cyanophenyl)methyl]-2-oxo-1,2-dihydro-3-pyridinyl}amino)carbonyl]amino}-3-(4-methylphenyl)propanoic acid | 0.1 | Calculated (M+H)⁺ = 431.18 m/z; |
| | | Found (M+H)⁺ = 431.09 m/z. |
| (3S)-3-[2,6-bis(methyloxy)phenyl]-3-{[({1-[(2-chlorophenyl)methyl]-2-oxo-1,2-dihydro-3-pyridinyl}amino)carbonyl]amino}propanoic acid | 6 | Calculated (M-H)⁻ = 484.14 m/z; |
| | | Found (M-H)⁻= 483.96 m/z. |
| (3S)-3-{[({1-[(3-hydroxyphenyl)methyl]-2-oxo-1,2-dihydro-3-pyridinyl} amino)carbonyl]amino}-3-(4-methylphenyl)propanoic acid | 0.2 | Calculated (M+H)⁺ = 420.18 m/z; |
| | | Found (M+H)⁺ = 422.05 m/z. |
| (3S)-3-[({[2-methyl-6-oxo-1-(phenylmethyl)-1,6-dihydro-5-pyrimidinyl]amino} carbonyl)amino]-3-(4-methylphenyl)propanoic acid | 0.1 | Calculated (M-H)⁻ = 419.17 m/z; |
| | | Found (M-H)⁻ = 419.03 m/z. |
| (3S)-3-{[({1-[(2-chlorophenyl)methyl]-4-oxo-1,4-dihydro-3-pyridinyl} amino)carbonyl]amino}-3-(4-methylphenyl)propanoic acid | 0.1 | Calculated (M-H)⁻ = 438.12 m/z; |
| | | Found (M-H)⁻ = 438.10 m/z. |
| (3S)-3-(4-methylphenyl)-3- {[({1-[(2nitrophenyl)methyl]-2-oxo-1,2-dihydro-3-pyridinyl}amino)carbonyl]amino}propanoic acid | 1 | Calculated (M+H)⁺ = 451.17 m/z; |
| | | Found (M+H)⁺ = 451.07 m/z. |
| (3S)-3-(4-methylphenyl)-3- {[({1 -[(4-nitrophenyl)methyl]-2-oxo-1,2-dihydro-3-pyridinyl} amino)carbonyl]amino}propanoic acid | 1 | Calculated (M+H)⁺ = 451.17 m/z; |
| | | Found (M+H)⁺ = 451.09 m/z. |
| (3S)-3-{[({1-[(2-chlorophenyl)methyl]-2-oxo-1,2-dihydro-3-pyridinyl} amino)carbonyl]amino }-3-(2,6-dihydroxyphenyl)propanoic acid | 3 | Calculated (M-H)⁻ = 456.10 m/z; |
| | | Found (M-H)⁻ = 456.04 m/z. |
| (3S)-3-{[({1-[(2,6-difluorophenyl)methyl]-2-oxo-1,2-dihydro-3-pyridinyl} amino)carbonyl]amino}-3-(4-methylphenyl)propanoic acid | 0.3 | Calculated (M-H)⁻ = 440.14 m/z ; |
| | | Found (M-H)⁻ = 440.00 m/z. |
| (3S)-3-{[({1-[(2,4-difluorophenyl)methyl]-2-oxo-1,2-dihydro-3-pyridinyl} amino)carbonyl]amino}-3-(4-methylphenyl)propanoic acid | 1.3 | Calculated (M-H)⁻ = 440.14 m/z; |
| | | Found (M-H)⁻ = 439.96 m/z. |
| (3S)-3-{[({1-[(2,5-difluorophenyl)methyl]-2-oxo-1,2-dihydro-3-pyridinyl}amino)carbonyl]amino}-3-(4-methylphenyl)propanoic acid | 0.8 | Calculated (M-H)⁻ = 440.14 m/z ; |
| | | Found (M-H)⁻= 439.96 m/z. |
| (3S)-3-{[({1-[(2-chlorophenyl)methyl]-2-methyl-6-oxo-1,6-dihydro-5-pyrimidinyl} amino)carbonyl]amino}-3-(4-methylphenyl)propanoic acid | 0.09 | Calculated (M-H)⁻ = 453.13 m/z; |
| | | Found (M-H)⁻ = 453.00 m/z. |
| (3S)-3-{[({1-[(2-chloro-6-fluorophenyl)methyl]-2-oxo-1,2-dihydro-3-pyridinyl} amino)carbonyl]amino} -3-(4-methylphenyl)propanoic acid | 0.1 | Calculated (M-H)⁻ = 456.11 m/z; |
| | | Found (M-H)⁻ = 455.94 m/z. |
| (3S)-3-{[({1-[(2-bromo-5-fluorophenyl)methyl]-2-oxo-1,2-dihydro-3-pyridinyl} amino)carbonyl] amino}-3-(4-methylphenyl)propanoic acid | 0.5 | Calculated (M-H)⁻= 500.06 m/z; |
| | | Found (M-H)⁻ = 499.91 m/z. |
| (3S)-3-{[({1-[(2-chloro-4-fluorophenyl)methyl]-2-oxo-1,2-dihydro-3--pyridinyl} amino)carbonyl]amino} -3-(4-methylphenyl)propanoic acid | 0.35 | Calculated (M-H)⁻ = 456.11 m/z; |
| | | Found (M-H)⁻ = 455.93 m/z. |
| (3S)-3-{[({1-[(2-bromophenyl)methyl]-2-oxo-1,2-dihydro-3-pyridinyl}amino)carbonyl]amino}-3-[3-methyl-4-(methyloxy)phenyl]propanoic acid | 0.2 | Calculated (M-H)⁻ = 512.08 m/z; |
| | | Found (M-H)⁻ = 511.96 m/z. |
| (3S)-3-{[({1-[(3,5-dimethyl-4-isoxazolyl)methyl]-2-oxo-1,2-dihydro-3-pyridinyl} amino)carbonyl]amino}-3-(4-methylphenyl)propanoic acid | 3 | Calculated (M-H)⁻=423.17 m/z; |
| | | Found (M-H)⁻ = 423.02 m/z. |
| (3S)-3-(4-methylphenyl)-3-{[({2-oxo-1-[(2,4,6- trimethylphenyl)methyl]-1,2-dihydro-3-pyridinyl}amino)carbonyl]amino}propanoic acid | 2.5 | Calculated (M-H)⁻=446.21 m/z; |
| | | Found (M-H)⁻ = 446.08 m/z. |
| (3S)-3-(4-methylphenyl)-3-{[({1-[(2-methyl-1,3-thiazol-4-yl)methyl]-2-oxo-1,2-dihydro-3-pyridinyl} amino)carbonyl]amino}propanoic acid | 1 | Calculated (M-H)⁻ = 425.13 m/z; |
| | | Found (M-H)⁻ = 424.99 m/z. |
| (3S)-3-({[(1-{[4-(1,1-dimethylethyl)phenyl]methyl}-2-oxo-1,2-dihydro-3-pyridinyl)amino]carbonyl}amino)-3-(4-methylphenyl)propanoic acid | 6 | Calculated (M-H)⁻ = 460.22 m/z; |
| | | Found (M-H)⁻ = 460.07 m/z. |
| (3S)-3-[({[1-(1,3-benzoxazol-2-ylmethyl)-2-oxo-1,2-dihydro-3-pyridinyl]amino}carbonyl)amino]-3-(4-methylphenyl)propanoic acid | >10 | Calculated (M-H)⁻ = 445.15 m/z; |
| | | Found (M-H)⁻ = 445.01 m/z. |
| (3S)-3-({[(1-{2-[(2-hydroxyphenyl)amino]-2-oxoethyl}-2-oxo-1,2-dihydro-3-pyridinyl)amino]carbonyl}amino)-3-(4-methylphenyl)propanoic acid | >10 | Calculated (M-H)⁻ = 463.16 m/z; |
| | | Found (M-H)⁻ = 463.06 m/z. |
| (3S)-3-{[({1-[(2-chloro-6-nitrophenyl)methyl]-2-oxo-1,2-dihydro-3-pyridinyl}amino)carbonyl]amino}-3-(4-methylphenyl)propanoic acid | 4 | Calculated (M-H)⁻=483.11 m/z; |
| | | Found (M-H)⁻ = 483.01 m/z. |
| (3S)-3-{[({1-[(5-chloro-2-fluorophenyl)methyl]-2-oxo-1,2-dihydro-3-pyridinyl}amino)carbonyl]amino} -3-(4-methylphenyl)propanoic acid | 2.5 | Calculated (M-H)⁻= 456.11 m/z; |
| | | Found (M-H)⁻ = 456.00 m/z. |
| (3S)-3-{[({1-[(2-amino-6-chlorophenyl)methyl]-2-oxo-1,2-dihydro-3-pyridinyl} amino)carbonyl]amino} -3-(4-methylphenyl)propanoic acid | 2 | Calculated (M-H)⁻=453.13 m/z; |
| | | Found (M-H)⁻ = 453.02 m/z. |
| (3S)-3-({[(1-{[2-fluoro-4-(trifluoromethyl)phenyl]methyl}-2-oxo-1,2-dihydro-3-pyridinyl)amino]carbonyl}amino)-3-(4-methylphenyl)propanoic acid | 3 | Calculated (M-H)⁻=490.14 m/z; |
| | | Found (M-H)⁻ = 489.99 m/z. |
| (3S)-3-{[({1-[(5-chloro-2-thiophenyl)methyl]-2-oxo-1,2-dihydro-3-pyridinyl} amino)carbonyl]amino}-3-(4-methylphenyl)propanoic acid | 1.3 | Calculated (M-H)⁻ = 444.08 m/z ; |
| | | Found (M-H)⁻ = 443.97 m/z. |
| (3S)-3-{[({1-[(2-bromo-5-nitrophenyl)methyl]-2-oxo-1,2-dihydro-3-pyridinyl}amino)carbonyl]amino}-3-(4-methylphenyl)propanoic acid | 2 | Calculated (M-H)⁻= 527.06 m/z ; |
| | | Found (M-H)⁻ = 526.95 m/z. |
| 3-(4-chlorophenyl)-3-{[({1-[(2-chlorophenyl)methyl]-4-hydroxy-2-oxo-1,2-dihydro-3-pyridinyl}amino)carbonyl]amino}propanoic acid | 0.03 | Calculated (M-H)⁻ = 474.06 m/z; |
| | | Found (M-H)⁻ = 474.07 m/z. |
| (3S)-3-[({[2-methyl-6-oxo-1-(phenylmethyl)-4-(2-pyridinyl)-1,6-dihydro-5-pyrimidinyl]amino}carbonyl)amino]-3-(4-methylphenyl)propanoic acid | 0.025 | Calculated (M+H)⁺ = 498.22 m/z; |
| | | Found (M+H)⁺ = 498.10 m/z. |
| (3S)-3-{[({1-[(5-amino-2-bromophenyl)methyl]-2-oxo-1,2-dihydro-3-pyridinyl}amino)carbonyl]amino} -3-(4-methylphenyl)propanoic acid | 0.08 | Calculated (M-H)⁻=497.08 m/z; |
| | | Found (M-H)⁻ = 497.02 m/z. |
| (3S)-3-{[({1-[(2,5-dimethylphenyl)methyl]-2-oxo-1,2-dihydro-3-pyridinyl }amino)carbonyl] amino} -3-(4-methylphenyl)propanoic acid | 0.15 | Calculated (M-H)⁻ = 432.19 m/z; |
| | | Found (M-H)⁻ = 432.04 m/z. |
| 3-(3-chlorophenyl)-3-{[({1-[(2-chlorophenyl)methyl]-4-hydroxy-2-oxo-1,2-dihydro-3-pyridinyl} amino) carbonyl]amino}propanoic acid | 0.03 | Calculated (M-H)⁻ = 474.06 m/z; |
| | | Found (M-H)⁻ = 474.03 m/z. |
| 3-{[({1-[(2-chlorophenyl)methyl]-4-hydroxy-2-oxo-1,2-dihydro-3-pyridinyl} amino)carbonyl]amino}-3-(3,4-dichlorophenyl)propanoic acid | 0.04 | Calculated (M-H)⁻ = 508.02 m/z; |
| | | Found (M-H)⁻ = 507.97 m/z. |
| (3S)-3-({[(1-{[5-(acetylamino)-2-bromophenyl]methyl}-2-oxo-1,2-dihydro-3-pyridinyl)amino]carbonyl}amino)-3-(4-methylphenyl)propanoic acid | 0.2 | Calculated (M-H)⁻= 539.09 m/z; |
| | | Found (M-H)⁻ = 539.02 m/z. |
| (3S)-3-[({[1-({2-bromo-5-[(methylsulfonyl)amino]phenyl}methyl)-2-oxo-1,2-dihydro-3-pyridinyl]amino} carbonyl)amino]-3-(4-methylphenyl)propanoic acid | 0.25 | Calculated (M-H)⁻= 575.06 m/z; |
| | | Found (M-H)⁻ = 575.01 m/z. |
| 3-(4-chlorophenyl)-3-{[({1-[(2-chlorophenyl)methyl]-2-oxo-1,2-dihydro-3-pyridinyl} amino)carbonyl]amino} propanoic acid | 0.4 | Calculated (M-H)⁻ = 458.07 m/z; |
| | | Found (M-H)⁻ = 457.96 m/z. |
| 3-(3-chlorophenyl)-3-{[({1-[(2-chlorophenyl)methyl]-2-oxo-1,2-dihydro-3-pyridinyl} amino)carbonyl]amino}propanoic acid | 1 | Calculated (M-H)⁻=458.07 m/z; |
| | | Found (M-H)⁻ = 457.93 m/z. |
| 3-{[({1-[(2-chlorophenyl)methyl]-2-oxo-1,2-dihydro-3-pyridinyl}amino)carbonyl]amino}-3-(3,4-dichlorophenyl)propanoic acid | 1 | Calculated (M-H)⁻ = 492.03 m/z; |
| | | Found (M-H)⁻= 491.85 m/z. |
| (3S)-3-{[({1-[(2-bromo-4-chlorophenyl)methyl]-2-oxo-1,2-dihydro-3-pyridinyl}amino)carbonyl]amino}-3-(4-methylphenyl)propanoic acid | 1 | Calculated (M-H)⁻ = 516.03 m/z; |
| | | Found (M-H)⁻ = 515.91 m/z. |
| (3S)-3-{[({1-[(4-chlorophenyl)methyl]-2-oxo-1,2-dihydro-3-pyridinyl}amino)carbonyl]amino}-3-(4-methylphenyl)propanoic acid | 2 | Calculated (M-H)⁻ = 438.12 m/z; |
| | | Found (M-H)⁻ = 437.88 m/z. |
| (3S)-3-{[({1-[(2-chlorophenyl)methyl]-4-hydroxy-2-oxo-1,2-dihydro-3-pyridinyl}amino)carbonyl]amino}-3-[2,3-dimethyl-4-(methyloxy)phenyl]propanoic acid | 0.035 | Calculated (M-H)⁻ = 498.14 m/z; |
| | | Found (M-H)⁻ = 498.05 m/z. |
| (3S)-3-{[({1-[(2-chlorophenyl)methyl]-4-hydroxy-2-oxo-1,2-dihydro-3-pyridinyl}amino)carbonyl]amino}-3-{4-[(trifluoromethyl)oxy]phenyl}propanoic acid | 0.015 | Calculated (M-H)⁻= 524.08 m/z; |
| | | Found (M-H)⁻= 524.03 m/z. |
| (3R)-3-[({[1-[(2-chlorophenyl)methyl]-4-(1,4- oxazinan-4-yl)-2-oxo-1,2-dihydro-3-pyridinyl]amino}carbonyl)amino]-5-methylhexanoic- acid | 0.1 | Calculated (M-H)⁻=489.19 m/z; |
| | | Found (M-H)⁻ = 489.13 m/z. |
| (3S)-3-[({[4-hydroxy-6-methyl-2-oxo-1-(phenylmethyl)-1,2-dihydro-3-pyridinyl]amino} carbonyl)amino]-3-(4-methylphenyl)propanoic acid | 0.1 | Calculated (M-H)⁻ = 434.17 m/z; |
| | | Found (M-H)⁻ = 434.08 m/z. |
| (3S)-3-{[({1-[(2-chlorophenyl)methyl]-2-oxo-4-[(propylsulfonyl)amino]-1,2-dihydro-3-pyridinyl} amino)carbonyl] amino }-3-(4-methylphenyl)propanoic acid | 0.030 | Calculated (M-H)⁻ = 559.14 m/z; |
| | | Found (M-H)⁻ = 559.04 m/z. |
| (3S)-3-{[({1-[(2-chlorophenyl)methyl]-4-hydroxy-2-oxo-1,2-dihydro-3-pyridinyl}amino)carbonyl]amino}-3-(4-ethylphenyl)propanoic acid | 0.025 | Calculated (M-H)⁻ = 468.13 m/z; |
| | | Found (M-H)⁻ = 468.06 m/z. |
| (3S)-3-{[({1-[(2-chlorophenyl)methyl]-4-hydroxy-2-oxo-1,2-dihydro-3-pyridinyl}amino)carbonyl]amino}-3-[4-(ethyloxy)phenyl]propanoic acid | 0.02 | Calculated (M-H)⁻ = 484.13 m/z; |
| | | Found (M-H)⁻ = 484.06 m/z. |
| (3S)-3-[({[4-hydroxy-2-oxo-1-(phenylmethyl)-1,2-dihydro-3-pyridinyl]amino}carbonyl)amino]-3-(4-methylphenyl)propanoic acid | 0.030 | Calculated (M-H)⁻ = 420.16 m/z; |
| | | Found (M-H)⁻ = 420.08 m/z. |

The present invention is illustrated by way of the foregoing description and examples. The foregoing description is intended as a non-limiting illustration, since many variations will become apparent to those skilled in the art in view thereof. It is intended that all such variations within the scope of the appended claims be embraced thereby.

## Claims

1. A compound of the structure 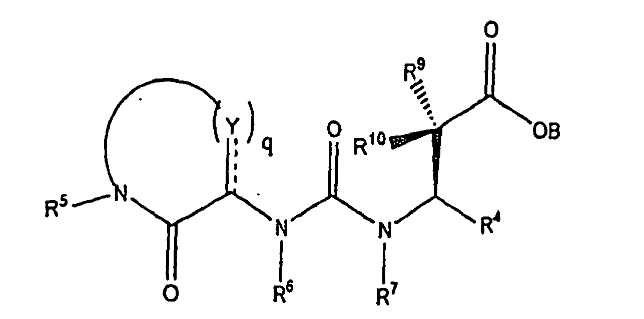 or a pharmaceutically acceptable salt thereof,
wherein Y at each occurrence, is independently selected from the group consisting of C (0), N, CR¹, C (R²) (R³), and CH; and the ring containing Y may optionally be a bicyclic ring;
q is an integer of from 2 to 5;
B and R⁶ are independently selected from the group consisting of hydrogen and alkyl;
R¹ at each occurrence is independently selected from the group consisting of hydrogen, halogen, hydroxyl, alkyl, alkenyl, alkynyl, alkoxy, alkenoxy, alkynoxy, thioalkoxy, hydroxyalkyl, alkyl-C(0)-, alkenyl-C(O)-, alkynyl-C(O)-, -CF₃, nitro, amino, cyano, carboxy, -N(C₁-C₃ alkyl)-C(O)(C₁-C₃alkyl), -NHC(O)N(C₁-C₃ alkyl)C(O)NH(C₁-C₃ alkyl), -NHC(O)NH(C₁-C₆ alkyl), alkylamino, alkenylamino, di(C₁-C₃)amino, -C(O)O-(C₁-C₃) alkyl, -C(O)NH-(C₁-C₃) alkyl, -C(O)N(C₁-C₃ alkyl)₂, -CH=NOH, -PO₃H₂, -OPO₃H₂, haloalkyl, alkoxyalkoxy, carboxaldehyde, carboxamide, cycloalkyl, cycloalkenyl, cycloalkylalkyl, aryl, aroyl, aryloxy, arylamino, biaryl, thioaryl, diarylamino, heterocyclyl, alkylaryl, aralkenyl, aralkyl, alkylheterocyclyl, heterocyclylalkyl, sulfonyl, -SO₂ (C₁-C₃ alkyl), -SO₃ (C₁-C₃alkyl), sulfonamido, carbamate, aryloxyalkyl, carboxyl and -C(O)NH(benzyl) groups; wherein each R¹ may be unsubstituted or substituted with one or more substituents selected from the group consisting of aryl, C₁-C₆ alkoxy, alkoxyalkoxy and alkyl;
and wherein when at least one Y is CR¹ R¹ and R⁶ taken
together may form a ring;
R² and R³ when present are hydrogen;
R⁴ is selected from the group consisting of alkyl and aryl;
R⁵ is selected from the group consisting of aralkyl, aryloxyalkyl and cycloalkylalkyl;
R⁷ is hydrogen;
R⁹ and R¹⁰ are selected from the group consisting of hydrogen and halogen; and wherein, unless otherwise indicated,
alkyl, alone or in combination, refers to C₁-C₁₂ straight or branched saturated chain radicals derived from saturated hydrocarbons by the removal of one hydrogen atom; alkenyl, alone or in combination, refers to a straight-chain or branched-chain alkenyl radical containing from 2 to 10 carbon atoms;
alkynyl, alone or in combination, refers to a straight branched chain alkynyl radical containing from 2 to 10 carbon atoms;
cycloalkyl refers to an aliphatic ring system having 3 to 10 carbon atoms and 1 to 3 rings which can be optionally substituted with one, two or three substituents independently selected from C₁-C₆ alkyl, haloalkyl, alkoxy, thioalkoxy, amino, alkylamino, dialkylamino, hydroxy, halo, mercapto, nitro, carboxaldehyde, carboxy, alkoxycarbonyl and carboxamide;
cycloalkenyl, alone or in combination, refers to a cyclic carbocycle containing from 4 to 8 carbon atoms and one or more double bonds;
cycloalkylalkyl refers to a cycloalkyl group appended to a C₁-C₆ alkyl radical; haloalkyl refers to a C₁-C₆ alkyl radical, to which is appended at least one halogen substituent; alkoxy, alone or in combination, refers to an alkyl ether radical, wherein alkyl is as defined above;
alkenoxy refers to a radical of formula alkenyl-O, provided that the radical is not an enol ether, wherein alkenyl is as defined above;
alkynoxy refers to a radical of formula alkynyl-O, provided that the radical is not an -ynol ether,
thioalkoxy refers to a thioether radical of formula alkyl-S-. wherein alkyl is as defined above;
alkoxyalkoxy refers to R_{g}-Rₕ O-wherein R_{g} is a C₁-C₆ alkyl and Rₕ is -(CH₂)ₙ,- wherein n' is an integer from 1 to 6;
alkylamino refers to RᵢNH- wherein Rᵢ is a C₁-C₆ alkyl group, provided that the radical is not an enamine;
alkenylamino refers to a radical of formula alkenyl-NH- or (alkenyl)₂N-, wherein alkenyl is as defined above, provided that the radical is not an enamine;
dialkylamino refers to RⱼRₖN- wherein Rⱼ and Rₖ are independently selected from C₁-C₆ alkyl ;
aryl, alone or in combination, refers to a carbocyclic aromatic group having 6 to 12 carbon atoms or a heterocyclic aromatic group containing at least one endocyclic N, O or S atom, said aryl being optionally substituted with at least one group selected from the group consisting of alcohols, ethers, esters, amides, sulfones, sulfides, hydroxyl, nitro, cyano, carboxy, amines, heteroatoms, C₁-C₆ alkyl, C₁-C₆ alkoxy, C₁-C₆ alkoxy-C(O)-, alkoxyalkoxy, acyloxy, halogens, trifluoromethoxy, trifluoromethyl, alkyl, aralkyl, alkenyl, alkynyl, aryl, cyano, carboxy, carboalkoxy, carboxyalkyl, cycloalkyl, cycloalkylalkyl, heterocyclyl, alkylheterocyclyl, heterocyclylalkyl, oxo, arylsulfonyl and aralkylaminocarbonyl wherein said substituents are optionally attached by means of a linker selected from a chain of 1-3 atoms containing any combination of -C-, -C(O)-, -NH-, -S-, -S(O)-, -O-, -C(O)O- or -S(O)O-;
thioaryl refers to a radical of formula aryl-S-, wherein aryl is as defined above; heterocyclyl, alone or in combination, refers to a non-aromatic 3 to 10-membered ring containing at least one endocyclic N, O, or S atom, said heterocyclyl being optionally aryl-fused and optionally substituted with at least one substituent independently selected from the group consisting of hydrogen, halogen, hydroxyl, amino, nitro, trifluoromethyl, trifluoromethoxy, alkyl, aralkyl, alkenyl, alkynyl, aryl, cyano, carboxy, carboalkoxy, carboxyalkyl, oxo, arylsulfonyl and aralkylaminocarbonyl.

2. A compound of claim 1 wherein 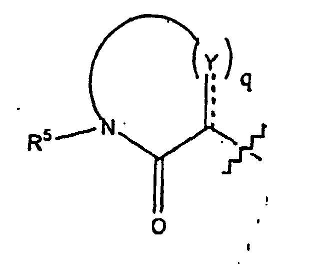 is selected from the group consisting of 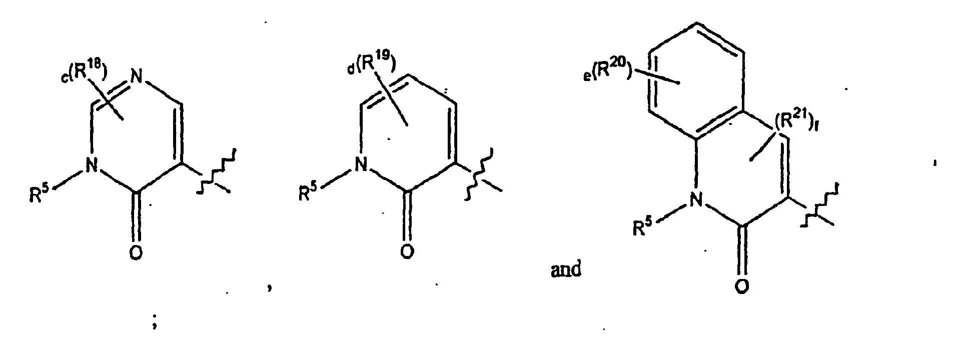 wherein R¹⁸, R¹⁹, R²⁰ and R²¹ at each occurrence are independently selected from the group consisting of hydrogen, halogen, hydroxyl, alkyl, alkenyl, alkynyl, alkoxy, alkenoxy, alkynoxy, thioalkoxy, hydroxyalkyl, aliphatic acyl, -CF₃, nitro, amino, cyano, carboxy, -N(C₁-C₃ alkyl)-C (O)(C₁-C₃ alkyl), -NHC(O)N(C₁-C₃ alkyl)C(O)NH(C₁-C₃alkyl), -NHC(O)NH(C₁-C₆ alkyl), alkylamino, alkenylamino, di(C₁-C₃) amino, -C(O)O-(C₁-C₃)alkyl, -C(O)NH-(C₁-C₃) alkyl, -C(O)N (C₁-C₃ alkyl)₂, -CH=NOH, -PO₃H₂, -OPO₃H₂, haloalkyl, alkoxyalkoxy, carboxaldehyde, carboxamide, cycloalkyl, cycloalkenyl, cycloalkynyl, cycloalkylalkyl, aryl, aroyl, aryloxy, arylamino, biaryl, thioaryl, diarylamino, heterocyclyl, alkylaryl, aralkenyl, aralkyl, alkylheterocyclyl, heterocyclylalkyl, sulfonyl, -SO₂-(C₁-C₃ alkyl), -SO₃-(C₁-C₃ alkyl), sulfonamido, carbamate, aryloxyalkyl, carboxyl and -C(O)NH(benzyl) groups;
c is an integer of zero to two;
d is an integer of zero to three;
e is an integer of zero to four; and
f is an integer of zero or one.

3. The compound of claim 1 wherein R⁵ is arylalkyl;
R⁴ is aryl; and
B and R⁶ are each independently hydrogen.

4. A compound of Claim 1 wherein two independent R¹, R², R³ or R⁵ groups taken together may be linked to form a ring.

5. A compound of Claim 1 selected from the group consisting of
(3S)-3-[({[2-methyl-4-(2-methylpropyl)-6-oxo-1-(phenylmethyl)-1,6-dihydro-5-pyrimidinyl]amino}carbonyl)amino]-3-(4-methylphenyl)propanoic acid,
(3S)-3-(1,3-benzodioxol-5-yl)-3-[({[2-oxo-1-(phenylmethyl)-4-propyl-1,2-dihydro-3-pyridinyl]amino}carbonyl)amino]propanoic acid,
(3S)-3-{[({1-[(2-chlorophenyl)methyl]-4-ethyl-2-oxo-1,2-dihydro-3-pyridinyl}amino)carbonyl]amino}-3-(4-methylphenyl)propanoic acid,
(3S)-3-{[({1-[(2-chlorophenyl)methyl]-2-oxo-4-propyl-1,2-dihydro-3-pyridinyl}amino)carbonyl]amino}-3-(4-methylphenyl)propanoic acid,
(3S)-3-{[({1-[(2-chlorophenyl)methyl]-4-methyl-2-oxo-1,2-dihydro-3-pyridinyl}amino)carbonyl]amino}-3-(4-methylphenyl)propanoic acid,
(3S)-3-{[({6-methyl-2-oxo-1-(phenylmethyl)-4-[(phenylmethyl)oxy]-1,2-dihydro-3-pyridinyl}amino)carbonyl]amino}-3-(4-methylphenyl)propanoic
(3S)-3-{[({1-[(2-chlorophenyl)methyl]-2,4-dimethyl-6-oxo-1,6-dihydro-5-pyrimidinyl}amino)carbonyl]amino}-3-(4-methylphenyl)propanoic acid,
(3S)-3-{[({4-amino-1-[(2-chlorophenyl)methyl]-6-methyl-2-oxo-1,2-dihydro-3-pyridinyl} amino)carbonyl]amino}-3-(4-methylphenyl)propanoic acid,
(3S)-3- {[({1-[(2-chlorophenyl)methyl]-4-methyl-2-oxo-1,2-dihydro-3-pyridinyl}amino)carbonyl]amino}-3-[4-(methyloxy)phenyl]propanoic acid,
(3S)-3- {[({1-[(2-chlorophenyl)methyl]-4-methyl-2-oxo-1,2-dihydro-3-pyridinyl} amino)carbonyl]amino}-3-(3,4-dimethylphenyl)propanoic acid,
(3S)-3- {[({4-amino-1-[(2-chlorophenyl)methyl]-2-oxo-1.2-dihydro-3-pyridinyl}amino)carbonyl]amino}-3-(4-methylphenyl)propanoic acid,
(3S)-3-{[({1-[(2-chlorophenyl)methyl]-4-hydroxy-2-oxo-1,2-dihydro-3-pyridinyl} amino)carbonyl]amino}-3-(4-methylphenyl)propanoic acid,
(3S)-3-[({[1-[(2-chlorophenyl)methyl]-4-(1,4-oxazinan-4-yl)-2-oxo-1,2-dihydro-3-pyridinyl]amino}carbonyl)amino]-3-(4-methylphenyl)propanoic acid,
(3S)-3-[({[1-[(2-chlorophenyl)methyl]-2-oxo-4-(propylamino)-1,2-dihydro-3-pyridinyl]amino}carbonyl)amino]-3-(4-methylphenyl)propanoic acid,
(3S)-3-{[({1-[(2-bromophenyl)methyl]-4-methyl-2-oxo-1,2-dihydro-3-pyridinyl}amino)carbonyl]amino-3-(4-methylphenyl)propanoic acid,
(3S)-3-{[({1-[(2-chlorophenyl)methyl]-4-hydroxy-2-oxo-1,2-dihydro-3-pyridinyl}amino)carbonyl]amino}-3-[3-methyl-4-(methyloxy)phenyl]propanoic acid,
(3S)-3-{[({1-[(2-chlorophenyl)methyl]-2-oxo-4-phenyl-1,2-dihydro-3-pyridinyl}amino)carbonyl]amino}-3-(4-methylphenyl)propanoic acid,
(3S)-3-{[({1-[(2-chlorophenyl)methyl]-4-[(2-{[2-(methyloxy)ethyl]oxy}ethyl)oxy]-2-oxo-1,2-dihydro-3-pyridinyl}amino)carbonyl]amino}-3-(4-methylphenyl)propanoic acid,
(3S)-3-{[({1-[(2-chlorophenyl)methyl]-4-hydroxy-9-methyl-2-oxo-1,2-dihydro-3-pyridinyl}amino)carbonyl]amino}-3-(4-methylphenyl)propanoic acid,
(3S)-3- {[({-[(2-chlorophenyl)methyl]-4-[(1, 1 -dimethylethyl)amino]-2-oxo-1,2-dihydro-3-pyridinyl}amino)carbonyl]amino}-3-(4-methylphenyl)propanoic acid,
(3S)-3-{[({1-[(2-chlorophenyl)methyl]-4-hydroxy-2-oxo-1,2-dihydro-3-pyridinyl}amino)carbonyl]amino}-3-phenylpropanoic acid,
(3S)-3-{[({1-[(2-chlorophenyl)methyl]-4-[4-methyltetrahydro-1(2H)-pyrazinyl]-2-oxo-1,2-dihydro-3-pyridinyl}amino)carbonyl]amino}-3-(4-methylphenyl)propanoic acid,
(3S)-3-{[({1-[(2-chlorophenyl)methyl]-4-hydroxy-2-oxo-1,2-dihydro-3-pyridinyl}amino)carbonyl]amino}-3-[4-(methyloxy)phenyl]propanoic acid,
(3S)-3-{[({1-[(2-chlorophenyl)methyl]-4-hydroxy-2-oxo-1,2-dihydro-3-pyridinyl}amino)carbonyl]amino}-3-(3,5-dimethylphenyl)propanoic acid,
(3S)-3-{[({1-[(2-chlorophenyl)methyl]-4-hydroxy-2-oxo-1,2-dihydro-3-pyridinyl}amino)carbonyl]amino}-3-(3-methylphenyl)propanoic acid,
(3S)-3-{[({1-[(2-chlorophenyl)methyl]-4-hydroxy-2-oxo-1,2-dihydro-3-pyridinyl}amino)carbonyl]amino}-3-[3-(methyloxy)phenyl]propanoic acid,
(3S)-3-[3,5-bis(methyloxy)phenyl]-3-{[({1-[(2-chlorophenyl)methyl]-4-hydroxy-2-oxo-1,2-dihydro-3-pyridinyl}amino)carbonyl]amino}propanoic acid,
(3S)-3-{[({1-[(2-chlorophenyl)methyl]-4-hydroxy-2-oxo-1,2-dihydro-3-quinolinyl}amino)carbonyl]amino}-3-(4-methylphenyl)propanoic acid,
(3S)-3-{[({1-[(2-chlorophenyl)methyl]4-hydroxy-2-oxo-1,2-dihydro-3-pyridinyl}amino)carbonyl]amino}-3-[3-(trifluoromethyl)phenyl]propanoic acid,
(3S)-3-{[({1-[(2-chlorophenyl)methyl]-4-[({ethyl[(ethylamino)carbonyl]amino}carbonyl)amino]-2-oxo-1,2-dihydro-3-pyridinyl}amino)carbonyl]amino}-3-(4-methylphenyl)propanoic acid,
(3S)-3-{[({4-(1-azetanyl)-1-[(2-chlorophenyl)methyl]-2-oxo-1,2-dihydro-3-pyridinyl}amino)carbonyl]amino}-3-(4-methylphenyl)propanoic acid,
(3S)-3-[({[1-[(2-chlorophenyl)methyl]-4-({2-[(2-{[2-(methyloxy)ethyl]oxy} ethyl)oxy]ethyl} oxy)-2-oxo-1,2-dihydro-3-pyridinyl]amino}carbonyl)amino]-3-(4-methylphenyl)propanoic acid,
(3S)-3-{[({1-[(2-fluorophenyl)methyl]-4-hydroxy-2-oxo-1,2-dihydro-3-pyridinyl}amino)carbonyl]amino}-3-(4-methylphenyl)propanoic acid,
(3S)-3-{[({1-[(2-chloro-6-fluorophenyl)methyl]-4-hydroxy-2-oxo-1,2-dihydro-3-pyridinyl}amino)carbonyl]amino}-3-(4-methylphenyl)propanoic acid,
(3S)-3-{[({1-[(2-chlorophenyl)methyl]-5-methyl-2-oxo-1,2-dihydro-3-pyridinyl}amino)carbonyl]amino}-3-(4-methylphenyl)propanoic acid,
(3S)-3-(1,3-benzodioxol-5-yl)-3-((((2-oxo-1-((4-(trifluoromethyl)phenyl)methyl)-1,2 dihydro-3-pyridinyl)amino)carbonyl)amino)propanoic acid, (3S)-3-((((1-((2-chlorophenyl)methyl)-2-oxo-1,2-dihydro-3-pyridinyl)amino)carbonyl)amino)-3-(4-methylphenyl)propanoic acid,
(3S)-3-((((1-((2-fluorophenyl)methyl)-2-oxo-1,2-dihydro-3-pyridinyl)amino)carbonyl)amino)-3-(4-methylphenyl)propanoic acid, (3S)-3-((((1-((2-bromophenyl)methyl)-2-oxo-1,2-dihydro-3-pyridinyl)amino)carbonyl)amino)-3-(4-methylphenyl)propanoic acid,
(3S)-3-((((1-((2,4-dichlorophenyl)methyl)-2-oxo-1,2-dihydro-3-pyridinyl)amino)carbonyl)amino)-3-(4-methylphenyl)propanoic acid, (3S)-3-((((1-((2-chloro-6-fluorophenyl)methyl)-2-oxo-1,2-dihydro-3-pyridinyl)amino)carbonyl)amino)-3-(4-methylphenyl)propanoic acid,
(3S)-3-((((1-((2-chlorophenyl)methyl)-4-hydroxy-2-oxo-1,2-dihydro-3-pyridinyl)amino)carbonyl)amino)-3-(4-trifluoromethyl)oxy)phenyl)propanoic acid and pharmaceutically acceptable salts thereof.

6. A pharmaceutical composition comprising a compound of Claim 1 in a pharmaceutically acceptable carrier.

7. Use of a compound of Claim 1 for the manufacture of a medicament for selectively inhibiting α₄β₁ integrin binding in a mammal wherein a therapeutic amount thereof is to be administered to said manual.

8. A compound of Claim 1 for use as a medicament.

## Patentansprüche

1. Verbindung mit der Struktur 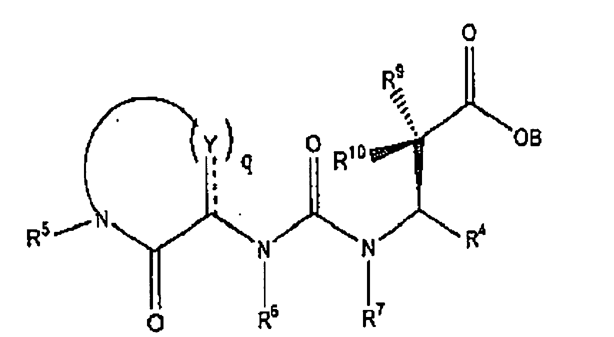 oder ein pharmazeutisch akzeptables Salz davon,
worin Y bei jedem Vorkommen jeweils unabhängig ausgewählt ist aus der Gruppe bestehend aus C(O), N, CR¹, C(R²)(R³) und CH, und der Ring mit Y wahlweise ein bizyklischer Ring sein kann,
q eine ganze Zahl von 2 bis 5 ist,
B und R⁶ jeweils unabhängig ausgewählt sind aus der Gruppe bestehend aus Wasserstoff und Alkyl,
R¹ bei jedem Vorkommen jeweils unabhängig ausgewählt ist aus der Gruppe bestehend aus Wasserstoff, Halogen, Hydroxyl, Alkyl, Alkenyl, Alkinyl, Alkoxy, Alkenoxy, Alkinoxy, Thioalkoxy, Hydroxyalkyl, Alkyl-C(O)-, Alkenyl-C(O)-, Alkinyl-C(O)-, -CF₃, Nitro, Amino, Cyano, Carboxy, -N(C₁-C₃-Alkyl)-C(O)(C₁-C₃-Alkyl), -NHC(O)N(C₁-C₃-Alkyl)C(O)NH(C₁-C₃-Alkyl), NHC(O)NH(C₁-C₆-Alkyl), Alkylamino, Alkenylamino, Di(C₁-C₃)amino, -C(O)O-(C₁-C₃)alkyl, -C(O)NH-(C₁-C₃)alkyl, -C(O)N-(C₁-C₃-Alkyl)₂, -CH=NOH, -PO₃H₂, -OPO₃H₂, Halogenalkyl, Alkoxyalkoxy, Carboxaldehyd, Carboxamid, Cycloalkyl, Cycloalkenyl, Cycloalkylalkyl, Aryl, Aroyl, Aryloxy, Arylamino, Biaryl, Thioaryl, Diarylamino, Heterocyclyl, Alkylaryl, Aralkenyl, Aralkyl, Alkylheterocyclyl, Heterocyclylalkyl, Sulfonyl, - SO₂-(C₁-C₃-Alkyl), -SO₃-(C₁-C₃-Alkyl), Sulfonamido, Carbamat, Aryloxyalkyl, Carboxyl und -C(O)NH(Benzyl)-Gruppen, worin jedes R¹ nicht substituiert oder mit einem oder mehreren Substituenten, ausgewählt aus der Gruppe bestehend aus Aryl, C₁-C₆-Alkoxy, Alkoxyalkoxy und Alkyl, substituiert sein kann
und worin, wenn mindestens ein Y CR¹ ist, R¹ und R⁶ zusammen einen Ring bilden können, R² und R³, falls vorhanden, Wasserstoff sind,
R⁴ ausgewählt ist aus der Gruppe bestehend aus Alkyl und Aryl,
R⁵ ausgewählt ist aus der Gruppe bestehend aus Aralkyl, Aryloxyalkyl und Cycloalkylalkyl, R⁷ Wasserstoff ist,
R⁹ und R¹⁰ ausgewählt sind aus der Gruppe bestehend aus Wasserstoff und Halogen,
und worin, solange nichts anderes angegeben ist,
Alkenyl, alleine oder in Kombination, einen geradkettigen oder verzweigtkettigen Alkenylrest mit 2 bis 10 Kohlenstoffatomen bedeutet,
Alkinyl, alleine oder in Kombination, einen geraden verzweigtkettigen Alkinylrest mit 2 bis 10 Kohlenstoffatomen bedeutet,
Cycloalkyl ein aliphatisches Ringsystem mit 3 bis 10 Kohlenstoffatomen und 1 bis 3 Ringen bedeutet, das wahlweise mit einem, zwei oder drei Substituenten substituiert sein kann, die jeweils unabhängig ausgewählt sind aus C₁-C₆-Alkyl, Halogenalkyl, Alkoxy, Thioalkoxy, Amino, Alkylamino, Dialkylamino, Hydroxy, Halogen, Mercapto, Nitro, Carboxaldehyd, Carboxy, Alkoxycarbonyl und Carboxamid,
Cycloalkenyl alleine oder in Kombination, einen zyklischen Carbozyklus mit 4 bis 8 Kohlenstoffatomen und einer oder mehreren Doppelbindungen bedeutet,
Cycloalkylalkyl eine Cycloalkylgruppe bedeutet, die an einen C₁-C₆-Alkylrest angefügt ist, Halogenalkyl einen C₁-C₆-Alkylrest bedeutet, an den mindestens ein Halogensubstituent angefügt ist,
Alkoxy, alleine oder in Kombination, einen Alkyletherrest bedeutet, worin Alkyl wie oben definiert ist,
Alkenoxy einen Rest mit der Formel Alkenyl-O bedeutet, mit der Maßgabe, dass der Rest kein Enolether ist, worin Alkenyl wie oben definiert ist,
Alkinoxy einen Rest mit der Formel Alkinyl-O bedeutet, mit der Maßgabe, dass der Rest kein -Inolether ist,
Thioalkoxy einen Thioetherrest mit der Formel Alkyl-S- bedeutet, worin Alkyl wie oben definiert ist,
Alkoxyalkoxy R_{g}O-RₕO- bedeutet, worin R_{g} ein C₁-C₆-Alkyl ist und Rₕ-(CH₂)ₙ- ist, worin n'eine ganze Zahl von 1 bis 6 ist,
Alkylamino RᵢNH- bedeutet, worin Rᵢ eine C₁-C₆-Alkylgruppe ist, mit der Maßgabe, dass der Rest kein Enamin ist,
Alkenylamino einen Rest mit der Formel Alkenyl-NH- oder (Alkenyl)₂N- bedeutet, worin Alkenyl wie oben definiert ist, mit der Maßgabe, dass der Rest kein Enamin ist,
Dialkylamino RⱼR_{K}N- bedeutet, worin Rⱼ und R_{K} jeweils unabhängig ausgewählt ist aus C₁-C₆-Alkyl,
Aryl, alleine oder in Kombination, eine carbozyklische aromatische Gruppe mit 6 bis 12 Kohlenstoffatomen oder eine heterozyklische aromatische Gruppe mit mindestens einem N-, O- oder S-Atom im Ring bedeutet, wobei das Aryl wahlweise mit mindestens einer Gruppe substituiert ist, die ausgewählt ist aus der Gruppe bestehend aus Alkoholen, Ethern, Estern, Amiden, Sulfonen, Sulfiden, Hydroxyl, Nitro, Cyano, Carboxy, Aminen, Heteroatomen, C₁-C₆-Alkyl, C₁-C₆-Alkoxy, C₁-C₆-Alkoxy-C(O)-, Alkoxyalkoxy, Acyolxy, Halogenen, Trifluormethoxy, Trifluormethyl, Alkyl, Aralkyl, Alkenyl, Alkinyl, Aryl, Cyano, Carboxy, Carboalkoxy, Carboxyalkyl, Cycloalkyl, Cycloalkylalkyl, Heterocyclyl, Alkylheterocyclyl, Heterocyclylalkyl, Oxo, Arylsulfonyl und Aralkylaminocarbonyl, worin die Substituenten wahlweise durch einen Linker angefügt sind, der ausgewählt ist aus einer Ketter mit 1-3 Atomen, die eine beliebige Kombination von -C-, -C(O)-, -NH-, -S-, -S(O)-, -O-, -C(O)O- oder -S(O)O- enthält,
Thioaryl einen Rest mit der Formel Aryl-S- bedeutet, worin Aryl wie oben definiert ist, Heterocyclyl, alleine oder in Kombination, einen nicht-aromatischen 3-10-gliedrigen Ring mit mindestens einem N-, O- oder S-Atom im Ring bedeutet, wobei das Heterocyclyl wahlweise mit Aryl fusioniert und wahlweise mit mindestens einem Substituenten substituiert ist, der jeweils unabhängig ausgewählt ist aus der Grupp bestehend aus Wasserstoff, Halogen, Hydroxyl, Amino, Nitro, Trifluormethyl, Trifluormethoxy, Alkyl, Aralkyl, Alkenyl, Alkinyl, Aryl, Cyano, Carboxy, Carboalkoxy, Carboxyalkyl, Oxo, Arylsulfonyl und Aralkylaminocarbonyl.

2. Verbindung nach Anspruch 1, worin 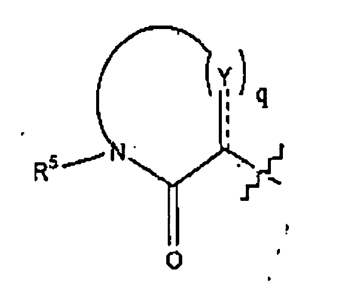 ausgewählt ist aus der Gruppe bestehend aus 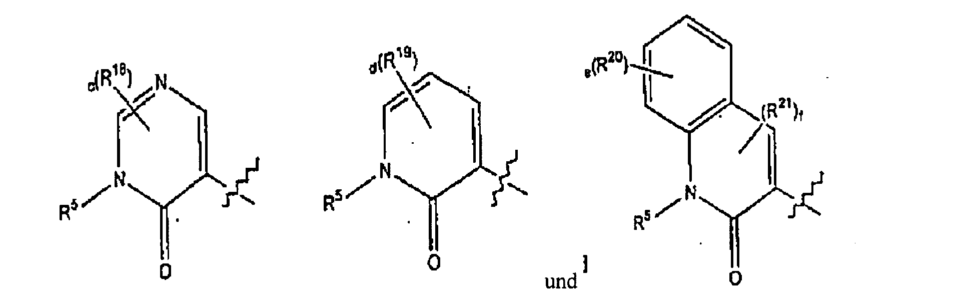 worin R¹⁸, R¹⁹, R²⁰ und R²¹ bei jedem Vorkommen jeweils unabhängig ausgewählt sind aus der Gruppe bestehend aus Wasserstoff, Halogen, Hydroxyl, Alkyl, Alkenyl, Alkinyl, Alkoxy, Alkenoxy, Alkinoxy, Thioalkoxy, Hydroxyalkyl, aliphatischem Acyl, -CF₃, Nitro, Amino, Cyano, Carboxy, -N(C₁-C₃-Alkyl)-C(O)( C₁ -C₃-Alkyl), -NHC(O)N(C₁-C₃-Alkyl)C(O)NH(C₁-C₃-Alkyl), -NHC(O)NH(C₁-C₆-Alkyl), Alkylamino, Alkenylamino, Di(C₁-C₃)amino, -C(O)O-(C₁-C₃)Alkyl, -C(O)NH(C₁-C₃-Alkyl), -C(O)N(C₁-C₃-Alkyl)₂, -CH=NOH, -PO₃H₂, -OPO₃H₂, Halogenalkyl, Alkoxyalkoxy, Carboxaldehyd, Carboxamid, Cycloalkyl, Cycloalkenyl, Cycloalkinyl, Cycloalkylalkyl, Aryl, Aroyl, Aryloxy, Arylamino, Biaryl, Thioaryl, Diarylamino, Heterocyclyl, Alkylaryl, Aralkenyl, Aralkyl, Alkylheterocyclyl, Haterocyclylalkyl, Sulfonyl, -SO₂-(C₁-C₃-Alkyl), -SO₃-(C₁-C₃-Alkyl), Sulfonamido, Carbamat, Aryloxyalkyl, Carboxyl und -C(O)NH(Benzyl)-Gruppen,
c eine ganze Zahl von Null bis Zwei ist,
d eine ganze Zahl von Null bis Drei ist,
e eine ganze Zahl von Null bis Vier ist und
f eine ganze Zahl von Null bis Eins ist.

3. Verbindung nach Anspruch 1, worin R⁵ Arylalkyl ist, R⁴ Aryl ist und B und R⁶ jeweils unabhängig Wasserstoff sind.

4. Verbindung nach Anspruch 1, worin zwei unabhängige R¹-, R²-, R³- oder R⁵-Gruppen zusammen unter Ringbildung verbunden sein können.

5. Verbindung nach Anspruch 1, ausgewählt aus der Gruppe bestehend aus
(3S)-3-[({[2-Methyl-4-(2-methylpropyl)-6-oxo-1-(phenylmethyl)-1,6-dihydro-5-pyrimidinyl]-amino }carbonyl)amino]-3-(4-methylphenyl)propansäure,
(3S)-3-(1,3-Benzodioxol-5-yl)-3-[({[2-oxo-1-(phenylmethyl)-4-propyl-1,2-dihydro-3-pyridinyl]amino}carbonyl)amino]propansäure,
(3S)-3-{[({1-[(2-Chlorphenyl)methyl]-4-ethyl-2-oxo-1,2-dihydro-3-pyridinyl}amino)-carbonyl]amino}-3-(4-methyIphenyl)propansäure,
(3S)-3-{[({1-[(2-Chlorphenyl)methyl]-2-oxo-4-propyl-1,2-dihydro-3-pyridinyl}amino)-carbonyl]amino}-3-(4-methylphenyl)propansäure,
(3S)-3-{[({1-[(2-Chlorphenyl)methyl]-4-methyl-2-oxo-1,2-dihydro-3-pyridinyl}amino)-carbonyl]amino}-3-(4-methylphenyl)propansäure,
(3S)-3-{[({6-Methyl-2-oxo-1-(phenylmethyl)-4-[(phenylmethyl)oxy]-1,2-dihydro-3-pyridinyl}amino)carbonyl]amino} -3-(4-methylphenyl)propansäure,
(3S)-3-{ [({1-[(2-Chlorphenyl)methyl]-2,4-dimethyl-6-oxo-1,6-dihydro-5-pyrimidinyl}amino)carbonyl]amino}-3-(4-methylphenyl)propansäure,
(3S)-3-{[({4-Amino-1-[(2-chlorphenyl)methyl]-6-methyl-2-oxo1,2-dihydro-3-pyridinyl}amino)carbonyl]amino}-3-(4-methylphenyl)propansäure,
(3S)-3-{[({1-[(2-Chlorphenyl)methyl]-4-methyl-2-oxo-1,2-dihydro-3-pyidinyl}amino)-carbonyl]amino}-3-[4-(methyloxy)phenyl]propansäure,
(3S)-3-{[({1-[(2-Chlorphenyl)methyl]-4-methyl-2-oxo-1,2-dihydro-3-pyridinyl}amino)-carbonyl]amino}-3-(3,4-dimethyIphenyl)propansäure,
(3S)-3-{[({4-Amino-1-[(2-chlorphenyl)methyl]-2-oxo-1,2-dihydro-3-pyridinyl}amino)-carbonyl]amino}-3-(4-methylphenyl)propansäure,
(3S)-3-{[({{1-[(2-Chlorphenyl)methyl]-4-hydroxy-2-oxo-1,2-dihydro-3-pyridinyl}amino)-carbonyl]amino}-3-(4-methylphenyl)propansäure,
(3S)-3-{[({1-[(2-Chlorphenyl)methyl]-4-(oxazinan-4-yl)-2-oxo-1,2-dihydro-3-pyridinyl]-amino}carbonyl)amino]-3-(4-methylphenyl)propansäure,
(3S)-3-[({[1-[(2-Chlorphenyl)methyl]-2-oxo-4-(propylamino)-1,2-dihydro-3-pyridinyl]-amino}carbonyl)amino]-3-(4-methylphenyl)propansäure,
(3S)-3-{[({1-[(2-Bromphenyl)methyl]-4-methyl-2-oxo-1,2-dihydro-3-pyridinyl)amino)-carbonyl]amino}-3-(4-methylphenyl)propansäure,
(3S)-3-{[({1-[(2-Chlorphenyl)methyl]-4-hydroxy-2-oxo-1,2-dihydro-3-pyridinyl}amino)-carbonyl]amino}-3-[3-methyl-4-(methyloxy)phenyl]propansäure,
(3S)-3-{[({1-[(2-Chlorphenyl)methyl]-2-oxo-4-phenyl-1,2-dihydro-3 -pyridinyl)amino)-carbonyl]amino}-3-(4-methylphenyl)propansäure,
(3 S)-3-{[([{1- [(2-Chlorphenyl)methyl]-4-[(2-[2-(methyloxy)ethyl]oxy}ethyl)oxy]-2-oxo-1,2-dihydro-3-pyridinyl}amino)carbonyl]amino}-3 -(4-methylphenyl)propansäure,
(3S)-3-{[({1-[(2-Chlorphenyl)methyl]-4-hydroxy-6-methyl-2-oxo-1,2-dihydro-3-pyridinyl}amino)carbonyl]amino}-3-(4-methylphenyl)propansäure,
(3S)-3-{[({1-[(2-Chlorphenyl)methyl]-4-[(1,1-dimethylethyl)amino]-2-oxo-1,2-dihydro-3-pyridinyl}amino)carbonyl]amino}-3-(4-methylphenyl)propansäure,
(3S)-3-{[({1-[(2-Chlorphenyl)methyl]-4-hydroxy-2-oxo-1,2-dihydro-3-pyridinyl}amino)-carbonyl]amino}-3-phenylpropansäure,
(3S)-3-{[({1-[(2-Chlorphenyl)methyl]-4-[4-methyltetrahydro-1(2H)-pyrazinyl]-2-oxo-1,2-dihydro-3-pyridinyl}amino)carbonyl]amino}-3-(4-methylphenyl)propansäure,
(3S)-3-{[({1-[(2-Chlorphenyl)methyl]-4-hydroxy-2-oxo-1,2-dihydro-3-pyridinyl}amino)-carbonyl]amino}-3-[4-(methyloxy)phenyl]propansäure,
(3S)-3-{[({1-[(2-Chlorphenyl)methyl]-4-hydroxy-2-oxo-1,2-dihydro-3-pyridinyl}amino)-carbonyl]amino}-3-(3,5-dimethylphenyl)propansäure,
(3S)-3-{[({1-[(2-Chlorphenyl)methyl]-4-hydroxy-2-oxo-1,2-dihydro-3-pyridinyl}amino)-carbonyl]amino}-3-(3-methylphenyl)propansäure,
(3S)-3-{[({1-[(2-Chlorphenyl)methyl]4-hydroxy-2-oxo-1,2-dihydro-3-pyridinyl}amino)-carbonyl]amino}-3-[3-(methyloxy)phenyl]propansäure,
(3S)-3-[3,5-Bis(methyloxy)phenyl]-3-{ [({1-[(2-chlorphenyl)methyl]-4-hydroxy-2-oxo-1,2-dihydro-3-pyridinyl amino)carbonyl]amino}propansäure,
(3S)-3-{[({1-[(2-Chlorphenyl)methyl]-4-hydroxy-2-oxo-1,2-dihydro-3-chinolinyl}amino)-carbonyl]amino} -3-(4-methylphenyl)propansäure,
(3S)-3-{[({1-[(-Chlorphenyl)methyl]-4-hydroxy-2-oxo-1,2-dihydro-3-pyidinyl }amino)-carbonyl]amino} -3 - [3 -(trifluormethyl)phenyl]propansäure,
(3S)-3-{[({1-[(2-Chlorphenyl)methyl]-4-[({ethyl[(ethylamino)carbonyl]amino}carbonyl)-amino]-2-oxo-1,2-dihydro-3 -pyridinyl}amino)carbonyl]amino}-3-(4-methylphenyl)propansäure,
(3S)-3-{[({4-(1-Azetanyl)-1-[(2-chlorphenyl)methyl]-2-oxo-1,2-dihydro-3-pyridinyl}amino)-carbonyl]amino}-3-(4-methylphenyl)propansäure,
(3S)-3-[({[1-[(2-Chlorphenyl)methyl]-4-((2-[(2-{[2-methyloxy)ethyl]oxy}ethyl)oxy]-ethyl}oxy)-2-oxo-1,2-dihydro-3-pyridinyl]amino}carbonyl)amino]-3-(4-methylphenyl)-propansäure,
(3S)-3-{[({1-[(2-Fluorphenyl)methyl]-4-hydroxy-2-oxo-1,2-dihydro-3-pyridinyl}amino)-carbonyl]amino}-3-(4-methylphenyl)propansäure,
(3S)-3-{[({1-[(2-Chlor-6-fluorphenyl)methyl]-4-hydroxy-2-oxo-1,2-dihydro-3-pyridinyl}amino)carbonyl]amino}-3-(4-methylphenyl)propansäure,
(3S)-3-{[({1-[(2-Chlorphenyl)methyl]-5-methyl-2-oxo-1,2-dihydro-3-pyridinyl}amino)-carbonyl]amino}-3-(4-methylphenyl)propansäure,
(3S)-3-(1,3-Benzodioxol-5-yl)-3-((((2-oxo-1-((4-(trifluormethyl)phenyl)methyl)-1,2-dihydro-3-pyridinyl)amino)carbonyl)amino)propansäure,
(3S)-3-((((1-((2-Chlorphenyl)methyl)-2-oxo-1,2-dihydro-3-pyridinyl)amino)carbonyl)amino)-3-(4-methylphenyl)propansäure,
(3S)-3-((((1-((2-Fluorphenyl)methyl)-2-oxo-1,2-dihydro-3-pyridinyl)amino)carbonyl)amino)-3-(4-methylphenyl)propansäure,
(3S)-3-((((1-((2-Bromphenyl)methyl)-2-oxo-1,2-dihydro-3-pyridinyl)amino)carbonyl)amino)-3-(4-methylphenyl)propansäure,
(3S)-3-((((1-((2,4-Dichlorphenyl)methyl)-2-oxo-1,2-dihydro-3-pyridinyl)amino)carbonyl)-amino)-3-(4-methylphenyl)propansäure,
(3S)-3-((((1-((2-Chlor-6-fluorphenyl)methyl)-2-oxo-1,2-dihydro-3-pyridinyl)amino)-carbonyl)amino)-3-(4-methylphenyl)propansäure,
(3 S)-3-((((1-((2-Chlorphenyl)methyl)-4-hydroxy-2-oxo-1,2-dihydro-3-pyridinyl)amino)-carbonyl)amino)-3-(4-trifluormethyl)oxy)phenyl)propansäure,
und pharmazeutisch akzeptable Salze davon.

6. Pharmazeutische Zusammensetzung, umfassend eine Verbindung nach Anspruch 1 in einem pharmazeutisch akzeptablen Träger.

7. Verwendung einer Verbindung nach Anspruch 1 für die Herstellung eines Arzneimittels zur selektiven Hemmung der α₄β₁-Integrin-Bindung bei einem Säuger, worin dem Säuger eine therapeutische Menge davon zu verabreichen ist.

8. Verbindung nach Anspruch 1 zur Verwendung als Arzneimittel.

## Revendications

1. Composé de structure 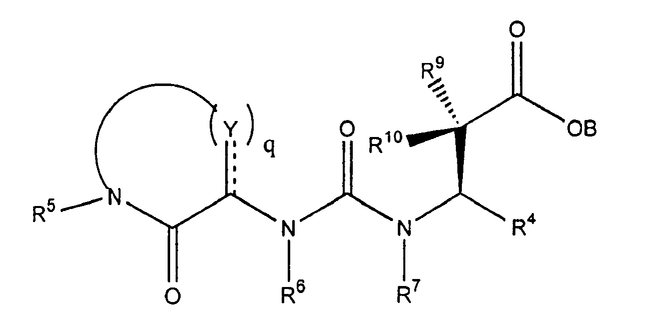 ou sel pharmaceutiquement acceptable de celui-ci,
dans lequel Y, dans chaque cas, est indépendamment choisi dans le groupe constitué de C(O), N, CR¹, C(R²)(R³), et CH ; et le cycle contenant Y peut être éventuellement un cycle bicyclique ;
q est un entier de 2 à 5 ;
B et R⁶ sont indépendamment choisis dans le groupe constitué par un atome d'hydrogène et un groupe alkyle ;
R¹, dans chaque cas, est indépendamment choisi dans le groupe constitué par un atome d'hydrogène, un atome d'halogène, les groupes hydroxyle, alkyle, alcényle, alcynyle, alcoxy, alcénoxy, alcynoxy, thioalcoxy, hydroxyalkyle, alkyl-C(O)-, alcényl-C(O)-, alcynyl-C(O)-, CF₃, nitro, amino, cyano, carboxy, -N(alkyle en C₁₋₃)-C(O)(alkyle en C₁₋₃), NHC(O)N(alkyle en C₁₋₃)C(O)NH(alkyle en C₁₋₃), -NHC(O)NH (alkyle en C₁₋₆), alkylamino, alcénylamino, di(alkyle en C₁₋₃)amino, -C(O)O-alkyle en C₁₋₃, -C(O)NH-alkyle en C₁₋₃, -C(O)N(alkyle en C₁₋₃)₂, -CH=NOH, -PO₃H₂, -OPO₃H₂, halogénoalkyle, alcoxyalcoxy, carboxaldéhyde, carboxamide, cycloalkyle, cycloalcényle, cycloalkylalkyle, aryle, aroyle, aryloxy, arylamino, biaryle, thioaryle, diarylamino, hétérocyclyle, alkylaryle, aralcényle, aralkyle, alkylhétérocyclyle, hétérocyclylalkyle, sulfonyle, -SO₂-(alkyle en C₁₋₃), -SO₃(alkyle en C₁₋₃), sulfonamido, carbamate, aryloxyalkyle, carboxyle et -C(O)NH(benzyle); où chaque R¹ peut être non substitué ou substitué par un ou plusieurs substituants choisis dans le groupe constitué par les groupes aryle, alcoxy en C₁₋₆, alcoxyalcoxy et alkyle ;
et où lorsque au moins un Y est CR¹, R¹ et R⁶ peuvent former ensemble un cycle ;
R² et R³, lorsqu'ils sont présents, sont des atomes d'hydrogène ;
R⁴ est choisi dans le groupe constitué par les groupes alkyle et aryle ;
R⁵ est choisi dans le groupe constitué par les groupes aralkyle, aryloxyalkyle et cycloalkylalkyle ;
R⁷ est un atome d'hydrogène ;
R⁹ et R¹⁰ sont choisis dans le groupe constitué par un atome d'hydrogène et un atome d'halogène ;
et où, sauf si indiqué différemment,
le terme alkyle, seul ou en combinaison, fait référence à un radical en C₁₋₁₂ à chaîne saturée linéaire ou ramifiée dérivé d'hydrocarbures saturés par l'élimination d'un atome d'hydrogène;
le terme alcynyle, seul ou en combinaison, fait référence à un radical alcényle à chaîne linéaire ou ramifiée contenant de 2 à 10 atomes de carbone ;
le terme alcényle, seul ou en combinaison, fait référence à un radical alcynyle à chaîne linéaire ou ramifiée contenant de 2 à 10 atomes de carbone ;
le terme cycloalkyle fait référence à un système de cycle aliphatique ayant de 3 à 10 atomes de carbone et de 1 à 3 cycles qui peuvent être éventuellement substitués par un, deux ou trois substituants indépendamment choisis parmi les groupes alkyle en C₁₋₆, halogénoalkyle, alcoxy, thioalcoxy, amino, alkylamino, dialkylamino, hydroxy, halogéno, mercapto, nitro, carboxaldéhyde, carboxy, alcoxycarbonyle et carboxamide ;
le terme cycloalcényle, seul ou en combinaison, fait référence à un carbocycle cyclique contenant de 4 à 8 atomes de carbone et une ou plusieurs doubles liaisons ;
le terme cycloalkylalkyle fait référence à un groupe cycloalkyle auquel est attaché un radical alkyle en C₁₋₆;
le terme halogénoalkyle fait référence à un radical alkyle en C₁₋₆, auquel est attaché au moins un substituant halogène ;
le terme alcoxy, seul ou en combinaison, fait référence à un radical éther alkylique, où le groupe alkyle est tel que défini ci-dessus ;
le terme alcénoxy fait référence à un radical de formule alcényl-O, à condition que le radical ne soit pas un éther énolique, où le groupe alcényle est tel que défini ci-dessus ;
le terme alcynoxy fait référence à un radical de formule alcynyl-O, à condition que le radical ne soit pas un éther ynolique ;
le terme thioalcoxy fait référence à un radical thioéther de formule alkyl-S-, où le groupe alkyle est tel que défini ci-dessus ;
le groupe alcoxyalcoxy fait référence à R_{g}O-RₕO-, où R_{g} est un groupe alkyle en C₁₋₆ et Rₕ est -(CH₂)_{n'}, où n' est un entier de 1 à 6 ;
le terme alkylamino fait référence à RⱼNH- où Rⱼ est un groupe alkyle en C₁₋₆, à condition que le radical ne soit pas une énamine ;
le terme alcénylamino fait référence à un radical de formule alcényl-NH- ou (alcényl)₂N-, où le groupe alcényle est tel que défini ci-dessus, à condition que le radical ne soit pas une énamine ;
le terme dialkylamino fait référence à RⱼRₖN- où Rⱼ et Rₖ sont indépendamment choisis parmi un groupe alkyle en C₁₋₆;
le terme aryle, seul ou en combinaison, fait référence à un groupe aromatique carbocyclique ayant de 6 à 12 atomes de carbone ou un groupe aromatique hétérocyclique contenant au moins un atome N, O ou S endocyclique, ledit groupe aryle étant éventuellement substitué par au moins un groupe choisi parmi le groupe constitué par les alcools, les éthers, les esters, les amides, les sulfones, les sulfides, l'hydroxyle, le nitro, le cyano, le carboxy, les amines, les hétéroatomes, un alkyle en C₁₋₆, un alcoxy en C₁₋₆, un alcoxy en C₁₋₆-C(O-, un alcoxyalcoxy, un acyloxy, les atomes d'halogène, le trifluorométhoxy, le trifluorométhyle, un alkyle, un aralkyle, un alcényle, un alcynyle, un aryle, un cyano, un carboxy, un carboalcoxy, un carboxyalkyle, un cycloalkyle, un cycloalkylalkyle, un hétérocyclyle, un alkylhétérocyclyle, un hétérocyclylalkyle, un oxo, un arylsulfonyle et un aralkylaminocarbonyle, où lesdits substituants sont éventuellement attachés à l'aide d'un groupe liant choisi parmi une chaîne de 1 à 3 atomes de carbone contenant n'importe quelle combinaison de -C-, -C(O)-, -NH-, -S-, -S(O)-, -O-, - C(O)O- ou S(O)O- ;
le terme thioaryle fait référence à un radical de formule aryl-S-, où le groupe aryle est tel que défini ci-dessus ;
le terme hétérocyclyle, seul ou en combinaison, fait référence à un cycle de 3 à 10 éléments non aromatiques contenant au moins un atome N, O ou S endocyclique, ledit groupe hétérocyclyle étant éventuellement fusionné avec un groupe aryle et éventuellement substitué par au moins un substituant indépendamment choisi dans le groupe constitué par un atome d'hydrogène, un atome d'halogène, les groupes hydroxyle, amino, nitro, trifluorométhyle, trifluorométhoxy, alkyle, aralkyle, alcényle, alcynyle, aryle, cyano, carboxy, carboalcoxy, carboxyalkyle, oxo, arylsulfonyle et aralkylaminocarbonyle.

2. Composé selon la revendication 1, dans lequel 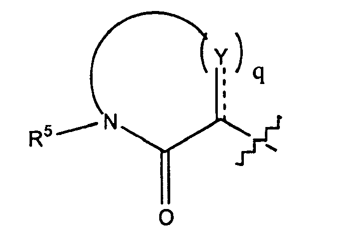 est choisi dans le groupe constitué par 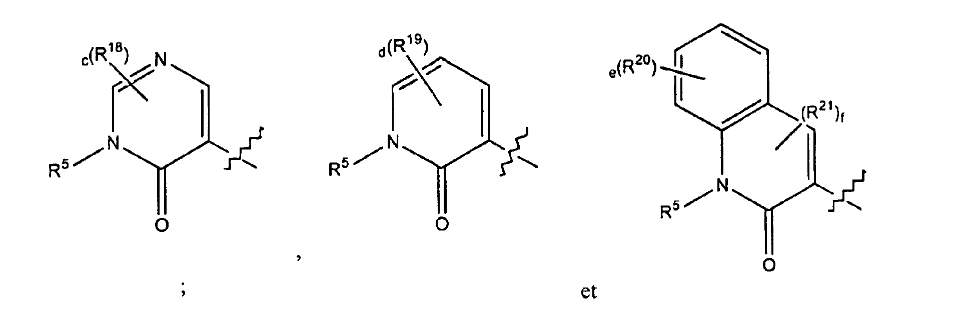 où R¹⁸, R¹⁹ R²⁰ et R²¹, dans chaque cas, sont indépendamment choisis dans le groupe constitué par un atome d'hydrogène, un atome d'halogène, les groupes hydroxyle, alkyle, alcényle, alcynyle, alcoxy, alcénoxy, alcynoxy, thioalcoxy, hydroxyalkyle, acyle aliphatique, -CF₃, nitro, amino, cyano, carboxy, -N(alkyle en C₁₋₃)-C(O)(alkyle en C₁₋₃), -NHC(O)N(alkyle en C₁₋₃)C(O)NH(alkyle en C₁₋₃), -NHC(O)NH(alkyle en C₁₋₆), alkylamino, alcénylamino, di(alkyle en C₁₋₃)amino, -C(O)O-alkyle en C₁₋₃, -C(O)NH-alkyle en C₁₋₃, -C(O)N(alkyle en C₁₋₃)₂, -CH=NOH, -PO₃H₂, -OPO₃H₂, halogénoalkyle, alcoxyalcoxy, carboxaldéhyde, carboxamide, cycloalkyle, cycloalcényle, cycloalcynyle, cycloalkylalkyle, aryle, aroyle, aryloxy, arylamino, biaryle, thioaryle, diarylamino, hétérocyclyle, alkylaryle, aralcényle, aralkyle, alkylhétérocyclyle, hétérocyclylalkyle, sulfonyle, -SO₂-(alkyle en C₁₋₃), -SO₃(alkyle en C₁₋₃), sulfonamido, carbamate, aryloxyalkyle, carboxyle et -C(O)NH(benzyle);
c est un entier de 0 ou 2 ;
d est un entier de 0 à 3 ;
e est en entier de 0 à 4 ; et
f est un entier de 0 ou 1.

3. Composé selon la revendication 1, dans lequel R⁵ est un groupe arylalkyle ; R⁴ est un groupe aryle ; et B et R⁶ sont chacun indépendamment un atome d'hydrogène.

4. Composé selon la revendication 1, dans lequel deux groupes indépendants R¹, R², R³ ou R⁵ pris ensemble peuvent être liés pour former un cycle.

5. Composé selon la revendication 1, choisi dans le groupe constitué par
l'acide (3S)-3-[({[2-méthyl-4-(2-méthylpropyl)-6-oxo-1-(phénylméthyl)-1,6-dihydro-5-pyrimidinyl]amino}carbonyl)amino]-3-(4-méthylphényl)propanoïque,
l'acide (3S)-3-(1,3-benzodioxol-5-yl)-3-[({[2-oxo-1-(phénylméthyl)-4-propyl-1,2-dihydro-3-pyridinyl]amino}carbonyl)amino]propanoïque,
l'acide (3S)-3-{[({1-[(2-chlorophényl)méthyl]-4-éthyl-2-oxo-1,2-dihydro-3-pyridinyl}amino)carbonyl]amino}-3-(4-méthylphényl)propanoïque,
l'acide (3S)-3-{[({1-[(2-chlorophényl)méthyl]-2-oxo-4-propyl-1,2-dihydro-3-pyridinyl}amino)carbonyl]amino}-3-(4-méthylphényl)propanoïque,
l'acide (3S)-3-{[({1-[(2-chlorophényl)méthyl]-4-méthyl-2-oxo-1,2-dihydro-3-pyridinyl}amino)carbonyl]amino}-3-(4-méthylphényl)propanoïque,
l'acide (3S)-3-{[({6-méthyl-2-oxo-1-(phénylméthyl)-4-[(phénylméthyl)oxy]-1,2-dihydro-3-pyridinyl}amino)carbonyl]amino}-3-(4-méthylphényl)propanoïque,
l'acide (3S)-3-{[({1-[(2-chlorophényl)méthyl]-2,4-diméthyl-6-oxo-1,6-dihydro-5-pyrimidinyl}amino)carbonyl]amino}-3-(4-méthylphényl)propanoïque,
l'acide (3S)-3-{[({4-amino-1-[(2-chlorophényl)méthyl]-6-méthyl-2-oxo-1,2-dihydro-3-pyridinyl}amino)carbonyl]amino)-3-(4-méthylphényl)propanoïque,
l'acide (3S)-3-{[({1-[(2-chlorophényl)méthyl]-4-méthyl-2-oxo-1,2-dihydro-3-pyridinyl }amino)carbonyl]amino}-3-[4-(méthyloxy)phényl]propanoïque,
l'acide (3S)-3-{[({1-[(2-chlorophényl)méthyl]-4-méthyl-2-oxo-1,2-dihydro-3-pyridinyl}amino)carbonyl]amino}-3-(3,4-diméthylphényl)propanoïque,
l'acide (3S)-3-{[({4-amino-1-[(2-chlorophényl)méthyl]-2-oxo-1,2-dihydro-3-pyridinyl}amino)carbonyl]amino}-3-(4-méthylphényl)propanoïque,
l'acide (3S)-3-{[({1-[(2-chlorophényl)méthyl]-4-hydroxy-2-oxo-1,2-dihydro-3-pyridinyl}amino)carbonyl]amino}-3-(4-méthylphényl)propanoïque,
l'acide (3S)-3-[({[1-[(2-chlorophényl)méthyl]-4-(1,4-oxazinan-4-yl)-2-oxo-1,2-dihydro-3-pyridinyl]amino]-3-(4-méthylphényl)propanoïque,
l'acide (3S)-3-[({[1-[(2-chlorophényl)méthyl]-2-oxo-4-(propylamino)-1,2-dihydro-3-pyridinyl]amino}carbonyl)amino]-3-(4-méthylphényl)propanoïque,
l'acide (3S)-3-{[({1-[(2-bromophényl)méthyl]-4-méthyl-2-oxo-1,2-dihydro-3-pyridinyl}amino)carbonyl]amino}-3-(4-méthylphényl)propanoïque,
l'acide (3S)- 3-{[({1-[(2-chlorophényl)méthyl]-4-hydroxy-2-oxo-1 ,2-dihydro-3-pyridinyl}amino)carbonyl]amino}-3-[3-méthyl-4-(méthyloxy)phényl]propanoïque,
l'acide (3S)-3-{[({1-[(2-chlorophényl)méthyl]-2-oxo-4-phényl-1,2-dihydro-3-pyridinyl}amino)carbonyl]amino}-3-(4-méthylphényl)propanoïque,
l'acide (3S)-3-{[({1 -[(2-chlorophényl)méthyl]-4-[(2-{[2-(méthyloxy)éthyl]oxy}éthyl)oxy]-2-oxo-1,2-dihydro-3-pyridinyl}amino)carbonyl]amino}-3-(4-méthylphényl)(4-méthylphényl)propanoïque,
l'acide (3S)-3-{[({1-[(2-chlorophényl)méthyl]-4-hydroxy-6-méthyl-2-oxo-1,2-dihydro-3-pyridinyl}amino)carbonyl]amino}-3-(4-méthylphényl)propanoïque,
l'acide (3S)-3-{[({1-[(2-chlorophényl)méthyl]-4-[(1,1-diméthyléthyl)amino]-2-oxo-1,2-dihydro-3-pyridinyl}amino)carbonyl]amino}-3(4-méthylphényl)propanoïque,
l'acide (3S)3-{[({1-[(2-chlorophényl)méthyl]-4-hydroxy-2-oxo-1,2-dihydro-3-pyridinyl}amino)carbonyl]amino}-3-phénylpropanoïque,
l'acide (3S)-3-{[({1-[(2-chlorophényl)méthyl]-4-[4-méthyltétrahydro-1(2H)-pyrazinyl]-2-oxo-1,2-dihydro-3-pyridinyl}amino)carbonyl]amino}-3-(4-méthylphényl)-propanoïque,
l'acide (3S)-3-{[({1-[(2-chlorophényl)méthyl]-4-hydroxy-2-oxo-1,2-dihydro-3-pyridinyl}amino)carbonyl]amino}-3-[4-(méthyloxy)phényl]propanoïque,
l'acide (3S)-3-{[({1-[(2-chlorophényl)méthyl]-4-hydroxy-2-oxo-1,2-dihydro-3-pyridinyl}amino)carbonyl]amino}-3-(3,5-diméthylphényl)propanoïque,
l'acide (3S)-3-{[({1-[(2-chlorophényl)méthyl]-4-hydroxy-2-oxo-1,2-dihydro-3-pyridinyl}amino)carbonyl]amino}-3-(3-méthylphényl)propanoïque,
l'acide (3S)-3-{[({1-[(2-chlorophényl)méthyl]-4-hydroxy-2-oxo-1,2-dihydropyridinyl}amino)carbonyl]amino}-3-[3-(méthyloxy)phényl]propanoïque,
l'acide (3S)-3-[3,5-bis(méthyloxy)phényl]-3-{[({1-[(2-chlorophényl)méthyl]-4-hydroxy-2-oxo-1,2-dihydro-3-pyridinyl}amino)carbonyl]amino}propanoïque,
l'acide (3S)-3-{[({1-[(2-chlorophényl)méthyl]-4-hydroxy-2-oxo-1,2-dihydro-3-quinoléinyl}amino)carbonyl]amino}-3-(4-méthylphényl)propanoïque,
l'acide (3S)-3-{[({1-[(2-chlorophényl)méthyl]-4-hydroxy-2-oxo-1,2-dihydro-3-pyridinyl}amino)carbonyl]amino}-3-[3-(trifluorométhyl)phényl]propanoïque,
l'acide (3S)-3-{[({1-[(2-chlorophényl)méthyl]-4-[({éthyl[(éthylamino)carbonyl]amino}carbonyl)amino]-2-oxo-1,2-dihydro-3-pyridinyl}amino)carbonyl]amino}-3-{4-méthylphényl)propanoïque,
l'acide (3S)-3-{[({4-(1-azétanyl)1-[(2-chlorophényl)méthyl]-2-oxo-1,2dihydro-3-pyridinyl}amino)carbonyl]amino}-3-(4-méthylphényl)propanoïque,
l'acide (3S)-3-[({[1-[(2-chlorophényl)méthyl]-4-({2-[(2-{[2-(méthyloxy)éthyl]oxy} éthyl)oxy]éthyl}oxy-2-oxo-1,2-dihydro-3-pyridinyl]amino]carbonyl)amino]-3-(4-méthylphényl)propanoïque,
l'acide (3S)-3-{[({1-[(2-fluorophényl)méthyl]-4-hydroxy-2-oxo-1,2-dihydro-3-pyridinyl}amino)carbonyl]amino}-3-(4-méthylphényl)propanoïque,
l'acide (3S)-3-{[({1-[(2-chloro-6-fluorophényl)méthyl]-4-hydroxy-2-oxo-1,2-dihydro-3-pyridinyl}amino)carbonyl]amino}-3-(4-méthylphényl)propanoïque,
l'acide (3S)-3-{[({1-[(2-chlorophényl)méthyl]-5-méthyl-2-oxo-1,2-dihydro-3-pyridinyl }amino)carbonyl]amino }-3-(4-méthylphényl)propanoïque,
l'acide (3S)-3-(1,3-benzodioxol-5-yl)-3-((((2-oxo-1-((4-(trifluorométhyl)phényl) méthyl)-1,2-dihydro-3-pyridinyl)amino)carbonyl)amino)propanoïque,
l'acide (3S)-3-((((1-((2-chlorophényl)méthyl)-2-oxo-1,2-dihydro-3-pyridinyl) amino)carbonyl)amino)-3-(4-méthylphényl)propanoïque,
l'acide (3S)-3-((((1-{(2-fluorophényl)méthyl}-2-oxo-1,2-dihydro-3-pyridinyl)amino)carbonyl)amino)-3-(4-méthylphényl)propanoïque,
l'acide (3S)-3-((((1((2-bromophényl)méthyl-2-oxo-1,2-dihydro-3-pyridinyl)amino)carbonyl)amino) -3-(4-méthylphényl)propanoïque,
l'acide (3S)-3-((((1-((2,4-dichlorophényl)méthyl)-2-oxo-1,2-dihydro-3-pyridinyl)amino)carbonyl)amino)-3-(4-méthylphényl)propanoïque,
l'acide (3S)-3-((((1-(2-chloro-6-fluorophényl)méthyl-2-xo-1,2-dihydro-3-pyridinyl)amino)carbonyl)amino)-3-(4-méthylphényl)propanoïque,
l'acide (3S)-3-((((1-(2-chlorophényl)méthyl-4-hydroxy-2-oxo-1,2-dihydro-3-pyridinyl)amino)carbonyl)amino)-3-(4-trifluororméthyl)oxy)phényl)propanoïque, et
les sels pharmaceutiquement acceptables de ceux-ci.

6. Composition pharmaceutique comprenant un composé selon la revendication 1 dans un support pharmaceutiquement acceptable.

7. Utilisation d'un composé selon la revendication 1, pour la préparation d'un médicament destiné à l'inhibition sélective de la liaison de l'intégrine α₄β₁ chez un mammifère, dans laquelle une quantité thérapeutique de celui-ci est administrée audit mammifère.

8. Composé selon la revendication 1, pour une utilisation en tant que médicament.


### SEQUENCE LISTING

(1) GENERAL INFORMATION:
   (i) APPLICANT: Biediger, Ronald J.; Chen, Qi; Holland, George W.; Kassir, Jamal M.; Li, Wen; Market, Robert V.; Scott, Ian L. and Wu, Chengde
   (ii) TITLE OF INVENTION: Carboxylic Acid Derivatives that Inhibit the Binding of Integrins to their Receptors
   (iii) NUMBER OF SEQUENCES: 1
   (iv) CORRESPONDENCE ADDRESS:
      (A) ADDRESSEE: Rockey, Milnamow & Katz, Ltd.
      (B) STREET: 180 N. Stetson Avenue, 2 Prudential Plaza, Suite 47
      (C) CITY: Chicago
      (D) STATE: Illinois
      (E) COUNTRY: U.S.A.
      (F) ZIP: 60601
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Katz, Martin L.
      (B) REGISTRATION NUMBER: 25,011
      (C) REFERENCE/DOCKET NUMBER: TEX4542P0400US
   (ix) TELECOMMUNICATION INFORMATION:
      (A) TELEPHONE: 312-616-5400
      (B) TELEFAX: 312-616-5460
(2) INFORMATION FOR SEQ ID NO:1:
   (i) SEQUENCE CHARACTERISTICS:
      (A) LENGTH: 26 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:1:
   Cys Asp Glu Leu Pro Gln Leu Val Thr Leu Pro His Pro Asn Leu His
   1 5 10 15
   Gly Pro Glu Ile Leu Asp Val Pro Ser Thr
   20 25